# EUROPEAN PATENT SPECIFICATION

(11) **EP 2 346 862 B1**
(45) Date of publication and mention of the grant of the patent: **12.09.2012**
(21) Application number: 09737476.3
(22) Date of filing: 09.10.2009
(51) Int. Cl.: C07D 413/04, C07D 417/04, C07D 417/14, C07D 495/04, A61K 31/422, A61P 31/04

(54) **2-BENZOTHIOPHENYL-AND 2-NAPHTHYL-0XAZ0LIDIN0NES AND THEIR AZAISOSTERE ANALOGUES AS ANTIBACTERIAL AGENTS**
2-BENZOTHIOPHENYL- UND 2-NAPHTHYLOXAZOLIDINONE UND IHRE AZAISOSTERANALOGA ALS ANTIBAKTERIELLE MITTEL
2-BENZOTHIOPHÉNYL- ET 2-NAPHTYL-OXAZOLIDINONES ET LEURS ANALOGUES ISOSTÈRES AZA UTILISÉS COMME AGENTS ANTIBACTÉRIENS

(30) Priority: 10.10.2008 WO PCT/IB2008/054168
(43) Date of publication of application: 27.07.2011
(73) Proprietor: Actelion Pharmaceuticals Ltd., 4123 Allschwil (CH)
(72) Inventor: KAEGI-EGGER, Verena, CH-4123 Allschwil (CH); GUDE, Markus, CH-4123 Allschwil (CH); HUBSCHWERLEN, Christian, CH-4123 Allschwil (CH); RUEEDI, Georg, CH-4123 Allschwil (CH); SURIVET, Jean-Philippe, CH-4123 Allschwil (CH); ZUMBRUNN ACKLIN, Cornelia, CH-4123 Allschwil (CH)
(74) Representative: Ruhlmann, Eric
(86) International application number: PCT/IB2009/054434
(87) International publication number: WO 2010/041219

(56) References cited:
- WO-A-2006/024741
- WO-A-2008/026172
- WO-A-2008/126034
- US-A1- 2002 115 669

## Description

The present invention concerns novel 2-benzothiophenyl and 2-naphthyl oxazolidinones and their azaisostere analogues, a pharmaceutical antibacterial composition containing them and the use of these compounds in the manufacture of a medicament for the treatment of infections (e.g. bacterial infections). These compounds are useful antimicrobial agents effective against a variety of human and veterinary pathogens including among others Gram-positive and Gram-negative aerobic and anaerobic bacteria and mycobacteria.

The intensive use of antibiotics has exerted a selective evolutionary pressure on microorganisms to produce genetically based resistance mechanisms. Modem medicine and socio-economic behaviour exacerbates the problem of resistance development by creating slow growth situations for pathogenic microbes, e.g. in artificial joints, and by supporting long-term host reservoirs, e.g. in immuno-compromised patients.

In hospital settings, an increasing number of strains of *Staphylococcus aureus, Streptococcus pneumoniae, Enterococcus spp.,* and *Pseudomonas aeruginosa,* major sources of infections, are becoming multi-drug resistant and therefore difficult if not impossible to treat:
- *S. aureus* is resistant to ß-lactams, quinolones and now even to vancomycin;
- *S. pneumoniae* is becoming resistant to penicillin or quinolone antibiotics and even to new macrolides;
- *Enteroccocci* are quinolone and vancomycin resistant and ß-lactam antibiotics are inefficacious against these strains;
- *Enterobacteriacea* are cephalosporin and quinolone resistant;
- *P. aeruginosa* are ß-lactam and quinolone resistant.

Furthermore, the incidence of multi-drug-resistant Gram-negative strains such as *Enterobacteriacae* and *Pseudomonas aeruginosa,* is steadily increasing and new emerging organisms like *Acinetobacter spp. or Clostridium difficile,* which have been selected during therapy with the currently used antibiotics, are becoming a real problem in hospital settings. Therefore, there is a high medical need for new antibacterial agents which overcome multidrug-resistant Gram-negative bacilli such as *A. baumannii,* ESBL-producing *E. coli* and *Klebsiella* species and *Pseudomonas aeruginosa* (George H. Talbot et al. Clinical Infectious Diseases (2006), 42, 657-68).

In addition, microorganisms that are causing persistent infections are increasingly being recognized as causative agents or cofactors of severe chronic diseases like peptic ulcers or heart diseases.

Certain antibacterial compounds comprising both a quinoline or naphthyridine moiety and an oxazolidinone group have been described in WO 2008/026172. In these compounds however, unlike the compounds of formula (I) described hereafter, the oxazolidinone is part of a spiro group.

The applicant has now found a new family of 2-benzothiophenyl and 2-naphthyl oxazolidinones antibiotic compounds and their azaisostere analogues, corresponding to the formula (I) described hereafter.

Various embodiments of the invention are presented hereafter:
i) The invention firstly relates to compounds of formula (I) 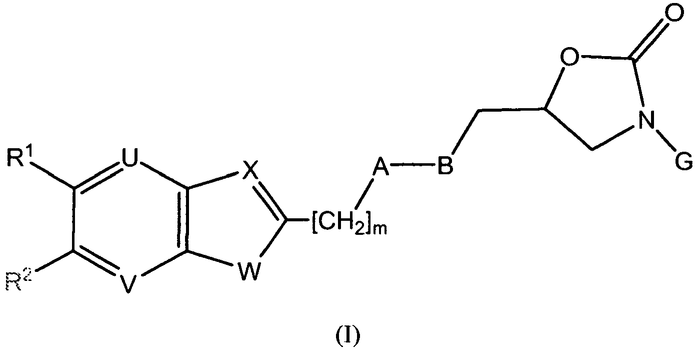 wherein
   R¹ represents hydrogen, (C₁₋₄)alkoxy or halogen;
   R² represents hydrogen or (C₁₋₄)alkoxy (and preferably hydrogen when R¹ represents (C₁₋₄)alkoxy or halogen);
   U represents N or CH;
   V represents N or CR^{b}, wherein R^{b} is hydrogen or halogen;
   W represents *-CH=CR^{a}-, *-N=CH- or S, wherein the asterisks indicate the bond which is linked to the carbon atom connecting V and W and wherein R^{a} represents hydrogen or halogen;
   X represents N or CR^{c}, wherein R^{c} is hydrogen, (C₁₋₄)alkyl or halogen;
   with the proviso that the group of formula (D) 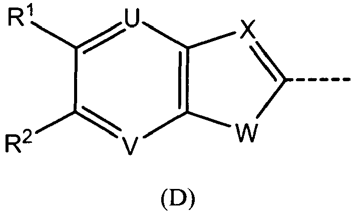 contains between none and three heteroatoms, wherein the heteroatoms are independently selected from nitrogen and, in case of W, sulfur;
   m represents 1, A represents -NHCH₂-^{#}, -CH₂NH-^{#}, -NHCH₂CH₂-^{#}, -CH₂NHCH₂-, -CH₂CH₂NH-^{#}, -NHCH₂CH₂NH-, -CH₂NHCH₂CH₂-^{#} or piperazin-1,4-diyl, wherein the hash indicates the bond which is linked to B, and B represents a bond; or
   m represents 0, A represents -NHCH₂CH₂NHCH₂-^{#} wherein the hash indicates the bond which is linked to B, and B represents a bond; or
   m represents 0, A represents -OCH₂-^{#}, wherein the hash indicates the bond which is linked to B, and B represents a group of the formula (E) 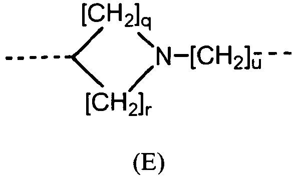 wherein q and r independently from each other represent 1 or 2 and u represents 0 or 1; or
   m represents 0, A represents O and B represents a group of the formula (F) 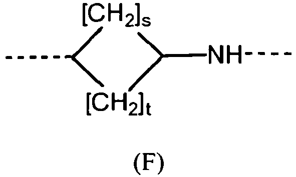 wherein s and t independently from each other represent 1 or 2;
   G represents a group of the formula (G1) 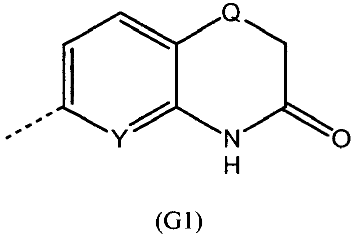 wherein Y represents CH or N, and Q represents O or S; or
   G represents a group of the formula (G2) 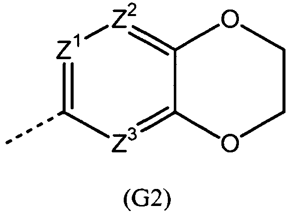 wherein
   Z¹ represents CH, Z² represents CH, and Z³ represents N; or
   Z¹ represents N, Z² represents CH or N, and Z³ represents CH; or
   Z¹ represents CH, Z² represents CH or N, and Z³ represents CH;
and to salts (in particular pharmaceutically acceptable salts) of compounds of formula (I).

The compounds of formula (I) according to embodiment i) may contain one or more stereogenic or asymmetric centers, such as one or more asymmetric carbon atoms. The compounds of formula (I) may thus be present as mixtures of stereoisomers or preferably as pure stereoisomers. Mixtures of stereoisomers may be separated in a manner known to a person skilled in the art.

The following paragraphs provide definitions of the various chemical moieties for the compounds according to the invention and are intended to apply uniformly throughout the specification and claims, unless an otherwise expressly set out definition provides a broader or narrower definition:
- The term "(C₁₋₄)alkyl", used alone or in combination, refers to a saturated straight or branched chain alkyl group containing from one to four carbon atoms. Representative examples of (C₁₋₄)alkyl groups include methyl, ethyl, propyl, *iso*-propyl, *n*-butyl, *iso*butyl, *sec*-butyl and *tert*-butyl. Preferred are methyl and ethyl. Most preferred is methyl.
- The term "(C₁₋₄)alkoxy", used alone or in combination, refers to a saturated straight or branched chain alkoxy group containing from one to four carbon atoms. Representative examples of (C₁₋₄)alkoxy groups include methoxy, ethoxy, propoxy, *iso*-propoxy, *n-*butoxy, *iso*-butoxy, *sec*-butoxy and *tert*-butoxy. The term "(C₁-Cₓ)alkoxy" refers to a straight or branched chain alkoxy group containing 1 to x carbon atoms. Preferred are methoxy and ethoxy. Most preferred is methoxy.
- The term "halogen" refers to fluorine, chlorine, bromine or iodine, preferably to fluorine or chlorine, and most preferably to fluorine.

In this text, a dashed bond shows a point of attachment of the radical drawn to the rest of the molecule. For example, the radical drawn below 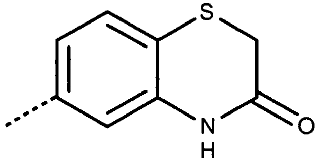 is the 3-oxo-3,4-dihydro-2*H*-benzo[1,4]thiazin-6-yl group.

In this application, the asterisk indicates the bond which is linked to the carbon atom connecting V and W in groups of formula (D). For example, if W represents *-N=CH-, the group of formula (D) is represented by the radical drawn below

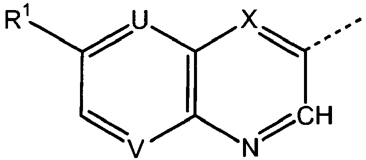

In this application, the hash indicates the bond in radicals A which is linked to B. For example, if A represents -NHCH₂CH₂-^{#}, the group "--[CH₂]ₘ-A-B--" is represented by the radical drawn below

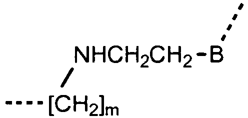

The present invention also includes isotopically labelled, especially ²H (deuterium) labelled compounds of formula (I), which compounds are identical to the compounds of formula (I) except that one or more atoms have each been replaced by an atom having the same atomic number but an atomic mass different from the atomic mass usually found in nature. Isotopically labelled, especially ²H (deuterium) labelled compounds of formula (I) and salts thereof are within the scope of the present invention. Substitution of hydrogen with the heavier isotope ²H (deuterium) may lead to greater metabolic stability, resulting e.g. in increased *in*-*vivo* half-life or reduced dosage requirements, or may lead to reduced inhibition of cytochrome P450 enzymes, resulting e.g. in an improved safety profile. In one variant of the invention, the compounds of formula (I) are not isotopically labelled, or they are labelled only with one or more deuterium atoms. In a sub-variant, the compounds of formula (I) are not isotopically labelled at all. Isotopically labelled compounds of formula (I) may be prepared in analogy to the methods described hereinafter, but using the appropriate isotopic variation of suitable reagents or starting materials.

The term "pharmaceutically acceptable salts" refers to non-toxic, inorganic or organic acid and/or base addition salts. Reference can be made to "Salt selection for basic drugs", Int. J. Pharm. (1986), 33, 201-217.

Where the plural form is used for compounds, salts, pharmaceutical compositions, diseases and the like, this is intended to mean also a single compound, salt, or the like.

Besides, the term "room temperature" as used herein refers to a temperature of 25°C. Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10°C, and preferably to an interval extending from Y minus 5°C to Y plus 5°C.
ii) The invention furthermore relates to compounds of formula (I) as defined in embodiment i) that are also compounds of formula (I_{P}) 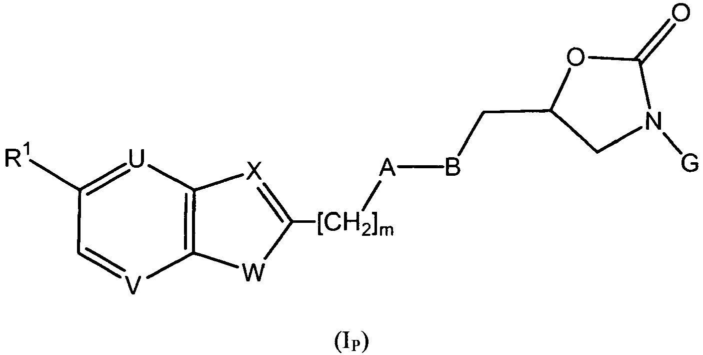 wherein
   R¹ represents hydrogen, (C₁₋₄)alkoxy or halogen;
   U represents N or CH;
   V represents N or CR^{b}, wherein R^{b} is hydrogen or halogen;
   W represents *-CH=CR^{a}-, *-N=CH- or S, wherein the asterisks indicate the bond which is linked to the carbon atom connecting V and W and wherein R^{a} represents hydrogen or halogen;
   X represents N or CR^{c}, wherein R^{c} is hydrogen, (C₁₋₄)alkyl or halogen;
   with the proviso that the group of formula (D) 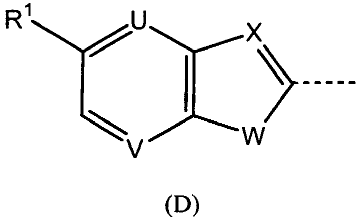 contains between none and three heteroatoms, wherein the heteroatoms are independently selected from nitrogen and, in case of W, sulfur;
   m represents 1, A represents -NHCH₂-^{#}, -NHCH₂CH₂-^{#}, -CH₂NHCH₂- or -CH₂CH₂NH-^{#}, wherein the hash indicates the bond which is linked to B, and B represents a bond; or
   m represents 0, A represents -OCH₂-^{#}, wherein the hash indicates the bond which is linked to B, and B represents a group of the formula (E) 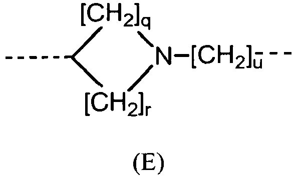 wherein q and r independently from each other represent 1 or 2 and u represents 0 or 1; or
   m represents 0, A represents O and B represents a group of the formula (F) 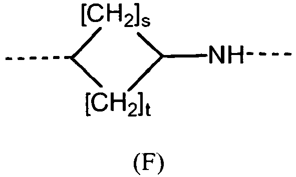 wherein s and t independently from each other represent 1 or 2;
   G represents a group of the formula (G1) 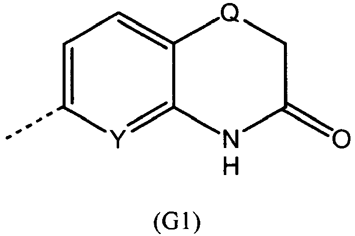 wherein Y represents CH or N, and Q represents O or S; or
   G represents a group of the formula (G2) 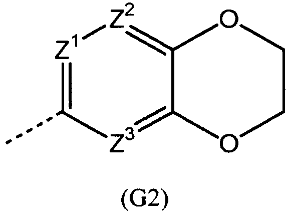 wherein
   Z¹ represents CH, Z² represents CH, and Z³ represents N; or
   Z¹ represents N, Z² represents CH or N, and Z' represents CH; or
   Z¹ represents CH, Z² represents CH or N, and Z³ represents CH;
   and to salts (in particular pharmaceutically acceptable salts) of compounds of formula (I)_{P}.
iii) In particular, the invention relates to compounds of formula (I) as defined in embodiment i) that are also compounds of formula (I_{CE}) 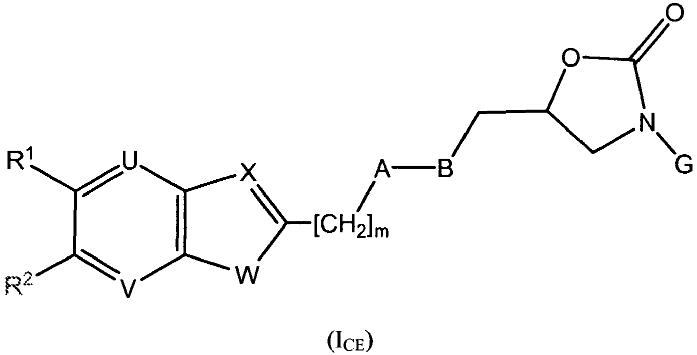 wherein
   R¹ represents hydrogen, (C₁₋₄)alkoxy (notably methoxy) or halogen (notably fluorine);
   R² represents hydrogen or also, if R¹ represents hydrogen, (C₁₋₄)alkoxy (notably methoxy);
   U represents CH, V represents CR^{b}, wherein R^{b} is hydrogen or halogen (notably fluorine),
   W represents -CH=CH- or *-N=CH-, wherein the asterisk indicates the bond which is linked to the carbon atom connecting V and W, and X represents N; or
   U represents CH or N, V represents CH or N, W represents S and X represents CR^{c}, wherein R^{c} is hydrogen or (C₁₋₄)alkyl (notably methyl); or
   U represents CH or N, V represents CH, W represents *-CH=CR^{a}- or *-N=CH-, wherein the asterisks indicate the bond which is linked to the carbon atom connecting V and W and wherein R³ represents hydrogen or halogen (notably fluorine), and X represents CH; or
   U represents CH or N, V represents N, W represents -CH=CH-, and X represents CH; m represents 1, A represents -NHCH₂-^{#}, -CH₂NH-^{#}, -NHCH₂CH₂-^{#}, -CH₂NHCH₂-, -CH₂CH₂NH-^{#}, -NHCH₂CH₂NH-, -CH₂NHCH₂CH₂-^{#} or piperazin-1,4-diyl, wherein the hash indicates the bond which is linked to B, and B represents a bond; or
   m represents 0, A represents -NHCH₂CH₂NHCH₂-^{#} wherein the hash indicates the bond which is linked to B, and B represents a bond; or
   m represents 0, A represents -OCH₂-^{#}, wherein the hash indicates the bond which is linked to B, and B represents a group of the formula (E) 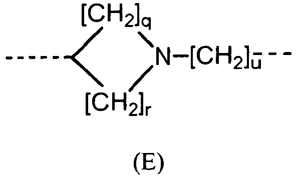 wherein q and r independently from each other represent 1 or 2 and u represents 0 or 1; or
   m represents 0, A represents O and B represents a group of the formula (F) 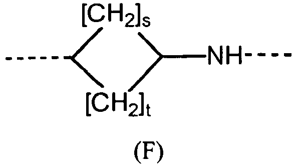 wherein s and t both represent 1;
   G represents a group of the formula (G1-a) 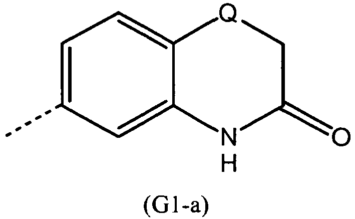 wherein Q represents O or S; or
   G represents a 2,3-dihydro-benzo[1,4]dioxin-6-yl group.
iv) The invention furthermore relates to compounds of formula (I)_{P} as defined in embodiment ii) that are also compounds of formula (I)_{CEP} 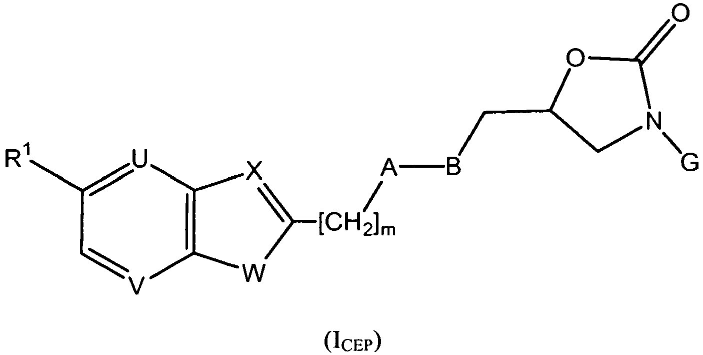 wherein
   R¹ represents hydrogen, (C₁₋₄)alkoxy (notably methoxy) or halogen (notably fluorine);
   U represents CH, V represents CR^{b}, wherein R^{b} is hydrogen or halogen (notably fluorine),
   W represents -CH=CH- or *-N=CH-, wherein the asterisk indicates the bond which is linked to the carbon atom connecting V and W, and X represents N; or
   U represents CH or N, V represents CH or N, W represents S, and X represents CR^{c}, wherein R^{c} is hydrogen or (C₁₋₄)alkyl (notably methyl); or
   U represents CH or N, V represents CH, W represents *-CH=CR^{a}- or *-N=CH-, wherein the asterisks indicate the bond which is linked to the carbon atom connecting V and W and wherein R^{a} represents hydrogen or halogen (notably fluorine), and X represents CH; or
   U represents CH or N, V represents N, W represents -CH=CH-, and X represents CH;
   m represents 1, A represents -NHCH₂-^{#}, -NHCH₂CH₂-^{#}, -CH₂NHCH₂- or -CH₂CH₂NH-^{#}, wherein the hash indicates the bond which is linked to B, and B represents a bond; or
   m represents 0, A represents -OCH₂-^{#}, wherein the hash indicates the bond which is linked to B, and B represents a group of the formula (E) 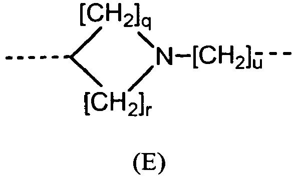 wherein q and r independently from each other represent 1 or 2 and u represents 0 or 1; or
   m represents 0, A represents O and B represents a group of the formula (F) 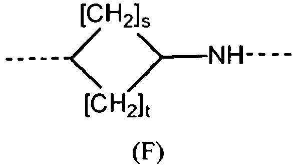 wherein s and t both represent 1;
   G represents a group of the formula (G1-a) 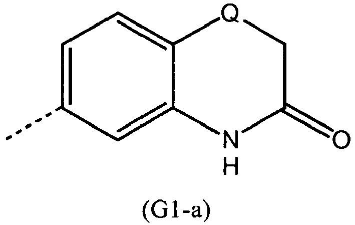 wherein Q represents O or S; or
   G represents a 2,3-dihydro-benzo[1,4]dioxin-6-yl group.
v) A further embodiment of the invention relates to compounds of formula (I) according to embodiment i) or iii), which are also compounds of formula (I_{E1}) drawn below wherein the stereocenter at position 5 of the oxazolidin-2-one moiety is in absolute (*S*)-configuration 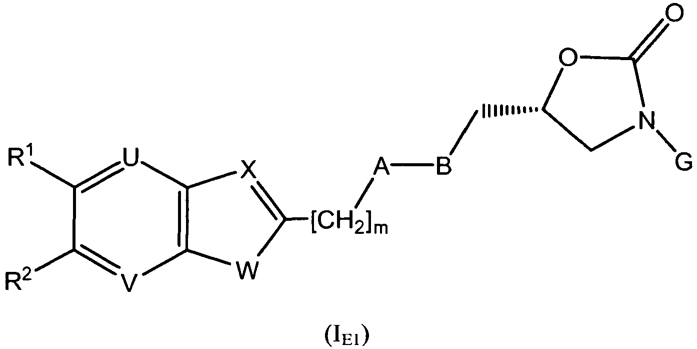 vi) The invention also relates to compounds of formula (I) according to embodiment ii) or iv), which are also compounds of formula (I_{E1P}) drawn below wherein the stereocenter at position 5 of the oxazolidin-2-one moiety is in absolute (*S*)-configuration 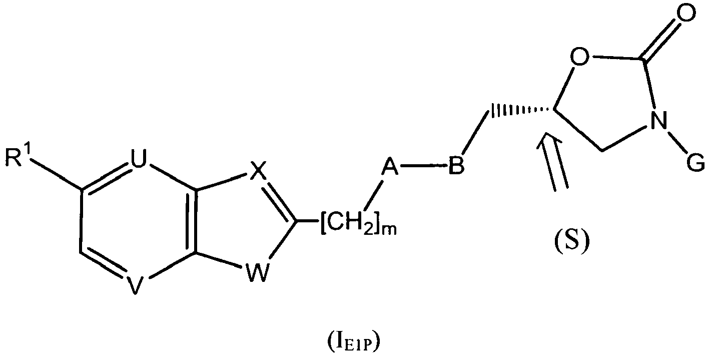
vii) A further embodiment of the invention relates to compounds of formula (I) according to embodiment i) or iii), which are also compounds of formula (I_{E2}) wherein the stereocenter at position 5 of the oxazolidin-2-one moiety is in absolute (R)-configuration: 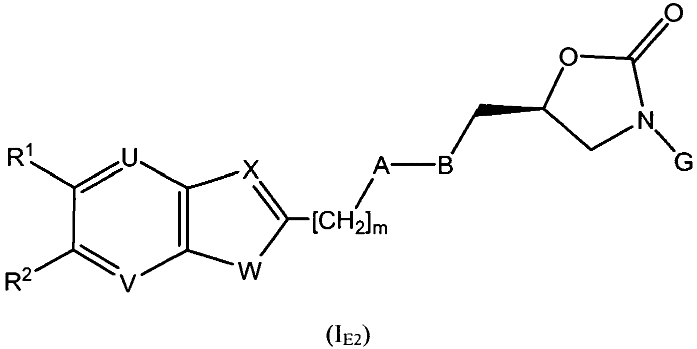
viii) A further embodiment of the invention relates to compounds of formula (I) according to embodiment ii) or iv), which are also compounds of formula (I_{E2P}) wherein the stereocenter at position 5 of the oxazolidin-2-one moiety is in absolute (*R*)-configuration: 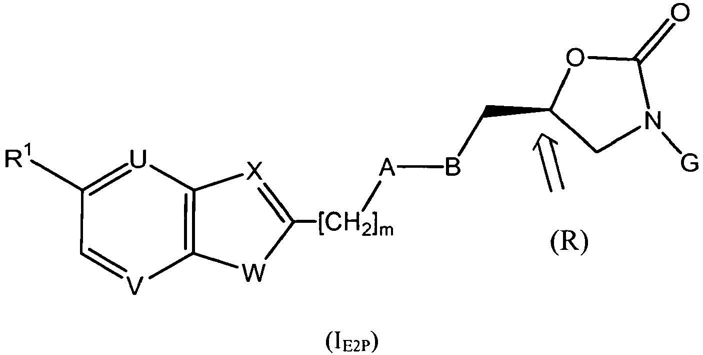
ix) A further embodiment of the invention relates to compounds of formula (I) according to any one of embodiments i) to viii), wherein R¹ represents hydrogen, (C₁-C₂)alkoxy or halogen.
x) A further embodiment of the invention relates to compounds of formula (I) according to any one of embodiments i) to ix), wherein R¹ represents hydrogen, methoxy or fluorine.
xi) Yet a further embodiment of the invention relates to compounds of formula (I) as defined in embodiment i), iii), v) or vii), or to compounds of formula (I) as defined in embodiment i), iii), v) or vii) taken together with embodiment ix) or x), wherein R¹ represents hydrogen and R² represents hydrogen, (C₁-C₂)alkoxy or halogen (and notably hydrogen, methoxy or fluorine).
xii) A further embodiment of the invention relates to compounds of formula (I) according to any one of embodiments i) to xi), wherein U represents CH, V represents CR^{b}, wherein R^{b} is hydrogen or halogen (and preferably hydrogen or fluorine), W represents -CH=CH-or *-N=CH-, wherein the asterisk indicates the bond which is linked to the carbon atom connecting V and W, and X represents N.
xiii) According to a sub-embodiment of embodiment xii), the compounds of formula (I) will be such that U represents CH, V represents CR^{b}, wherein R^{b} is hydrogen or fluorine, W represents -CH=CH-, and X represents N.
xiv) According to a sub-embodiment of embodiment xii), the compounds of formula (I) will be such that U represents CH, V represents CH, W represents *-N=CH- and X represents N.
xv) A further embodiment of the invention relates to compounds of formula (I) according to any one of embodiments i) to xi), wherein U represents CH or N, V represents CH or N, W represents S, and X represents CR^{c}, wherein R^{c} is hydrogen or (C₁₋₄)alkyl (and preferably hydrogen or methyl).
xvi) A further embodiment of the invention relates to compounds of formula (I) according to any one of embodiments i) to xi), wherein U represents CH or N, V represents CH, W represents *-CH=CR^{a}- or *-N=CH-, wherein the asterisks indicate the bond which is linked to the carbon atom connecting V and W and wherein R^{a} represents hydrogen or halogen (and preferably hydrogen or fluorine), and X represents CH.
xvii) A further embodiment of the invention relates to compounds of formula (I) according to any one of embodiments i) to xi), wherein U represents CH or N, V represents N, W represents -CH=CH-, and X represents CH.
xviii) A further embodiment of the invention relates to compounds of formula (I) according to any one of embodiments i) to xvii), wherein m represents 1, A represents -NHCH₂-^{#}, -CH₂NH-^{#}, -NHCH₂CH₂-^{#}, -CH₂NHCH₂-, -CH₂CH₂NH-^{#}, -NHCH₂CH₂NH-, -CH₂NHCH₂CH₂-^{#} or piperazin-1,4-diyl, wherein the hash indicates the bond which is linked to B, and B represents a bond.
xix) According to a sub-embodiment of embodiment xviii), the compounds of formula (I) will be such that A represents -NHCH₂-^{#} or -NHCH₂CH₂-^{#}.
xx) According to another sub-embodiment of embodiment xviii), the compounds of formula (I) will be such that A represents -CH₂NH-^{#}, -CH₂NHCH₂- or -CH₂NHCH₂CH₂-^{#}.
xxi) According to yet another sub-embodiment of embodiment xviii), the compounds of formula (I) will be such that A represents -CH₂CH₂NH-^{#}.
xxii) According to a further sub-embodiment of embodiment xviii), the compounds of formula (I) will be such that A represents -NHCH₂CH₂NH-.
xxii) According to a further sub-embodiment of embodiment xviii), the compounds of formula (1) will be such that A represents piperazin-1,4-diyl.
xxiii) A further embodiment of the invention relates to compounds of formula (I) as defined in embodiment ii) or iv), or to compounds of formula (I) as defined in embodiment ii) or iv) taken together with any of embodiments v) to x) or xii) to xvii), wherein m represents 1, A represents -NHCH₂-^{#}, -NHCH₂CH₂-^{#}, -CH₂NHCH₂- or -CH₂CH₂NH-#, wherein the hash indicates the bond which is linked to B, and B represents a bond.
xxiv) According to a sub-embodiment of embodiment xxiii), the compounds of formula (I) will be such that A represents -NHCH₂-^{#} or -NHCH₂CH₂-^{#}.
xxv) According to a sub-embodiment of embodiment xxiii), the compounds of formula (I) will be such that A represents -CH₂NHCH₂-.
xxvi) According to a sub-embodiment of embodiment xxiii), the compounds of formula (I) will be such that A represents -CH₂CH₂NH-^{#}.
xxvii) Yet a further embodiment of the invention relates to compounds of formula (I) as defined in embodiment i) or iii), or to compounds of formula (I) as defined in embodiment i) or iii) taken together with any of embodiments v) to xvii), wherein m represents 0, A represents -NHCH₂CH₂NHCH₂-^{#} wherein the hash indicates the bond which is linked to B, and B represents a bond.
xxviii) A further embodiment of the invention relates to compounds of formula (I) according to any one of embodiments i) to xvii), wherein m represents 0, A represents -OCH₂-^{#}, wherein the hash indicates the bond which is linked to B, and B represents a group of the formula (E), wherein q and r independently from each other represent 1 or 2 (and preferably q and r both represent 1) and u represents 0 or 1.
xxix) According to a sub-embodiment of embodiment xxvii), the compounds of formula (I) will be such that B represents a group of the formula (E), wherein q and r independently from each other represent 1 or 2 (and preferably q and r both represent 1) and u represents 0.
xxx) According to another sub-embodiment of embodiment xxix), the compounds of formula (I) will be such that B represents a group of the formula (E), wherein q, r and u all represent 1.
xxxi) A further embodiment of the invention relates to compounds of formula (I) according to any one of embodiments i), ii) or v) to xvii), wherein m represents 0, A represents O and B represents a group of the formula (F), wherein s and t independently from each other represent I or 2.
xxxii) According to a sub-embodiment of embodiment xxxi), the compounds of formula (I) will be such that B represents a group of the formula (F), wherein s and t both represent 1.
xxxiii) A further embodiment of the invention relates to compounds of formula (I) according to any one of embodiments i), ii) or v) to xxxi), wherein G represents a group of the formula (G1) as defined in embodiment i) or ii).
xxxiv) A further embodiment of the invention relates to compounds of formula (I) according to any one of embodiments i) to xxxiii), wherein G represents a group of the formula (G1-a) as defined in embodiment iii) or iv).
xxxv) A further embodiment of the invention relates to compounds of formula (I) according to any one of embodiments i), ii) or v) to xxxi), wherein G represents a group of the formula (G2) as defined in embodiment i) or ii).
xxxvi) A further embodiment of the invention relates to compounds of formula (I) according to embodiment xxxv), wherein G represents a 2,3-dihydro-benzo[1,4]dioxin-6-yl group.
xxxvii) Particularly preferred are the following compounds of formula (I) as defined in one of embodiments i) to iv):
   - 6-((*R*)-5-{3-[(7-fluoro-quinolin-2-ylmethyl)-amino]-propyl]-2-oxo-oxazolidin-3-yl)-4*H*-benzo[1,4]oxazin-3-one;
   - 6-((*R*)-2-oxo-5-{3-[(quinolin-2-ylmethyl)-amino]-propyl]-oxazolidin-3-yl)-4*H*-benzo[1,4]oxazin-3-one;
   - 6-{(*S*)-2-oxo-5-[(3-quinolin-2-yl-propylamino)-methyl]-oxazolidin-3-yl}-4*H*-benzo[1,4]thiazin-3-one;
   - 6-((*R*)-2-oxo-5-{2-[(quinolin-2-ylmethyl)-amino]-ethyl}-oxazolidin-3-yl)-4*H*-benzo[1,4]thiazin-3-one;
   - 6-{(*R*)-2-oxo-5-[2-(2-quinolin-2-yl-ethylamino)-ethyl]-oxazolidin-3-yl}-4*H*-benzo[1,4]thiazin-3-one;
   - 6-{(*R*)-5-[(*R*)-3-(7-methoxy-quinolin-2-yloxymethyl)-pyrrolidin-1-ylmethyl]-2-oxo-oxazolidin-3-yl}-4*H*-benzo[1,4]thiazin-3-one;
   - 6-{(*R*)-5-[4-(7-methoxy-quinolin-2-yloxymethyl)-piperidin-1-ylmethyl]-2-oxo-oxazolidin-3-yl}-4*H*-benzo[1,4]thiazin-3-one;
   - (*R*)-3-(2,3-dihydro-benzo[1,4]dioxin-6-yl)-5-[3-(7-methoxy-quinoxalin-2-yloxymethyl)-azetidin-1-ylmethyl]-oxazolidin-2-one;
   - 6-((*R*)-5-{2-[3-(7-methoxy-quinoxalin-2-yloxymethyl)-azetidin-1-yl]-ethyl}-2-oxo-oxazolidin-3-yl)-4*H*-benzo[1,4]thiazin-3-one;
   - 6- {(*R*)-5-[3-(7-methoxy-quinoxalin-2-yloxymethyl)-azetidin-1-ylmethyl]-2-oxo-oxazolidin-3-yl}-4*H*-benzo[1,4]thiazin-3-one;
   - 6- {(*R*)-5-[3-(7-methoxy-quinoxalin-2-yloxymethyl)-azetidin-1-ylmethyl]-2-oxo-oxazolidin-3-yl}-4*H*-benzo[1,4]oxazin-3-one; and
   - 6-((*R*)-5-{[3-(7-methoxy-quinolin-2-yloxy)-cyclobutylamino]-methyl}-2-oxo-oxazolidin-3-yl)-4*H*-benzo[1,4]thiazin-3-one;
   wherein it is well understood that in any above listed compound a cyclobutylamino moiety which is substituted in 3-position may be in relative *cis-* or *trans*-configuration.
xxxviii) In addition to the compounds of embodiment xxxvii), further preferred compounds of formula (I) according to one of embodiments i) to iv) are selected from the group consisting of:
   - 6-(2-oxo-5-{3-[(quinolin-2-ylmethyl)-amino]-propyl}-oxazolidin-3-yl)-4*H*-benzo[1,4]thiazin-3-one;
   - 6-(5-{3-[(7-fluoro-quinolin-2-ylmethyl)-amino]-propyl}-2-oxo-oxazolidin-3-yl)-4*H*-benzo[1,4]thiazin-3-one;
   - 6-(5-{3-[(6-fluoro-quinolin-7-ylmethyl)-amino]-propyl}-2-oxo-oxazolidin-3-yl)-4*H*-benzo[1,4]thiazin-3-one;
   - 6-(5-{3-[(5-fluoro-quinolin-2-ylmethyl)-amino]-propyl}-2-oxo-oxazolidin-3-yl)-4*H*-benzo[1,4]thiazin-3-one;
   - 6-(5-{3-[(3-methyl-benzo[*b*]thiophen-2-ylmethyl)-amino]-propyl}-2-oxo-oxazolidin-3-yl)-4*H*-benzo[1,4]thiazin-3-one;
   - 6-(2-oxo-5-{3-[(thieno[2,3-*b*]pyridin-2-ylmethyl)-amino]-propyl}-oxazolidin-3-yl)-4*H*-benzo[1,4]thiazin-3-one;
   - 6-(2-oxo-5-{3-[(thieno[2,3-*b*]pyrazin-6-ylmethyl)-amino]-propyl}-oxazolidin-3-yl)-4*H*-benzo[1,4]thiazin-3-one;
   - 6-(2-oxo-5-{3-[(quinolin-3-ylmethyl)-amino]-propyl}-oxazolidin-3-yl)-4*H*-benzo[1,4]thiazin-3-one;
   - 6-(5-{3-[(naphthalen-2-ylmethyl)-amino]-propyl]-2-oxo-oxazolidin-3-yl)-4*H*-benzo[1,4]thiazin-3-one;
   - 6-(2-oxo-5-{3-[(quinolin-7-ylmethyl)-amino]-propyl}-oxazolidin-3-yl)-4*H*-benzo[1,4]thiazin-3-one; and
   - 6-(2-oxo-5-{3-[(quinolin-6-ylmethyl)-amino]-propyl}-oxazolidin-3-yl)-4*H*-benzo[1,4]thiazin-3-one;
   wherein it is well understood that the oxazolidinyl moiety of the above listed compounds may be at 5-position in absolute (*R*)- or (*S*')-configuration.
xxxviii) A further embodiment of this invention thus relates to the compounds of formula (I) as defined in one of embodiments i) to iv), which compounds are selected from the group consisting of the compounds listed in embodiment xxxvi) and the compounds listed in embodiment xxxvii), and to the salts (in particular the pharmaceutically acceptable salts) of such compounds.
xxxix) Yet a further embodiment of this invention relates the compounds of formula (I) as defined in embodiment i) or iii), which compounds are selected from the group consisting of:
   - 6-{(*R*)-2-oxo-5-[(2-quinolin-2-yl-ethylamino)-methyl]-oxazolidin-3-yl}-4*H*-benzo[1,4]thiazin-3-one;
   - 6-{(*R*)-2-oxo-5-[3-(2-quinolin-2-yl-ethylamino)-propyl]-oxazolidin-3-yl}-4*H*-benzo[1,4]thiazin-3-one;
   - 6-((*R*)-2-oxo-5-{2-[2-(quinolin-2-ylamino)-ethylamino]-ethyl}-oxazolidin-3-yl)-4*H*-benzo[1,4]oxazin-3-one;
   - 6-[(*R*)-2-oxo-5-({2-[(quinolin-2-ylmethyl)-amino]-ethylamino}-methyl)-oxazolidin-3-yl]-4*H*-benzo[1,4]thiazin-3-one;
   - 6-[(*R*)-2-oxo-5-(4-quinolin-2-ylmethyl-piperazin-1-ylmethyl)-oxazolidin-3-yl]-4*H*-benzo[1,4]thiazin-3-one;
   - 6-((*S*)-5-{3-[(7-fluoro-2-methoxy-quinoxalin-6-ylmethyl)-amino]-propyl}-2-oxo-oxazolidin-3-yl)-4*H*-benzo[1,4]thiazin-3-one;
   and to the salts (in particular the pharmaceutically acceptable salts) of such compounds.
xl) Another embodiment of this invention furthermore relates to the compounds of formula (I) as defined in embodiment i) or iii), which compounds are selected from the group consisting of the compounds listed in embodiment xxxvi), the compounds listed in embodiment xxxvii) and the compounds listed in embodiment xxxix), and to the salts (in particular the pharmaceutically acceptable salts) of such compounds.

The compounds of formula (I) according to the invention, i.e. according to one of embodiments i) to xl), are suitable for the use as chemotherapeutic active compounds in human and veterinary medicine and as substances for preserving inorganic and organic materials in particular all types of organic materials for example polymers, lubricants, paints, fibres, leather, paper and wood.

The compounds of formula (I) according to the invention are particularly active against bacteria and bacteria-like organisms. They are therefore particularly suitable in human and veterinary medicine for the prophylaxis and chemotherapy of local and systemic infections caused by these pathogens as well as disorders related to bacterial infections comprising pneumonia, otitis media, sinusitis, bronchitis, tonsillitis, and mastoiditis related to infection by *Streptococcus pneumoniae, Haemophilus influenzae, Moraxella catarrhalis, Staphylococcus aureus, Enterococcus faecalis, E. faecium, E. casseliflavus, S. epidermidis, S. haemolyticus,* or *Peptostreptococcus spp.;* pharyngitis, rheumatic fever, and glomerulonephritis related to infection by *Streptococcus pyogenes,* Groups C and G streptococci, *Corynebacterium diphtheriae,* or *Actinobacillus haemolyticum;* respiratory tract infections related to infection by *Mycoplasma pneumoniae, Legionella pneumophila, Streptococcus pneumoniae, Haemophilus influenzae,* or *Chlamydia pneumoniae;* blood and tissue infections, including endocarditis and osteomyelitis, caused by S. *aureus, S. haemolyticus, E. faecalis, E. faecium, E. durans,* including strains resistant to known antibacterials such as, but not limited to, beta-lactams, vancomycin, aminoglycosides, quinolones, chloramphenicol, tetracyclines and macrolides; uncomplicated skin and soft tissue infections and abscesses, and puerperal fever related to infection by *Staphylococcus aureus,* coagulase-negative staphylococci (i.e., *S. epidermidis, S. haemolyticus,* etc.), *Streptococcus pyogenes, Streptococcus agalactiae,* Streptococcal groups C-F (minute colony streptococci), viridans streptococci, *Corynebacterium minutissimum, Clostridium spp.,* or *Bartonella henselae;* uncomplicated acute urinary tract infections related to infection by *Staphylococcus aureus,* coagulase-negative staphylococcal species, or *Enterococcus spp*.; urethritis and cervicitis; sexually transmitted diseases related to infection by *Chlamydia trachomatis, Haemophilus ducreyi, Treponema pallidum, Ureaplasma urealyticum,* or *Neiserria gonorrheae;* toxin diseases related to infection by S. *aureus* (food poisoning and toxic shock syndrome), or Groups A, B, and C streptococci; ulcers related to infection by *Helicobacter pylori;* systemic febrile syndromes related to infection by *Borredia recurrentis;* Lyme disease related to infection by *Borrelia burgdorferi*; conjunctivitis, keratitis, and dacrocystitis related to infection by *Chlamydia trachomatis, Neisseria gonorrhoeae, S. aureus, S. pneumoniae, S. pyogenes, H. influenzae,* or *Listeria* spp.; disseminated *Mycobacterium avium* complex (MAC) disease related to infection by *Mycobacterium avium,* or *Mycobacterium intracellulare;* infections caused by *Mycobacterium tuberculosis, M. leprae, M. paratuberculosis, M. kansasii,* or *M*. *chelonei;* gastroenteritis related to infection by *Campylobacter jejuni;* intestinal protozoa related to infection by *Cryptosporidium* spp.; odontogenic infection related to infection by viridans streptococci; persistent cough related to infection by *Bordetella pertussis;* gas gangrene related to infection by *Clostridium perfringens* or *Bacteroides* spp.; and atherosclerosis or cardiovascular disease related to infection by *Helicobacter pylori* or *Chlamydia pneumoniae.*

The compounds of formula (I) according to the present invention are further useful for the preparation of a medicament for the treatment of infections that are mediated by bacteria such as *E. coli, Klebsiella pneumoniae* and other Enterobacteriaceae, *Acinetobacter spp.* including *Acinetobacter baumanii, Stenothrophomonas maltophilia, Neisseria meningitidis, Bacillus cereus, Bacillus anthracis, Clostridium difficile, Corynebacterium spp., Propionibacterium acnes* and bacteroide *spp.*

The compounds of formula (I) according to the present invention are further useful to treat protozoal infections caused by *Plasmodium malaria, Plasmodium falciparum, Toxoplasma gondii, Pneumocystis carinii, Trypanosoma brucei* and *Leishmania spp.*

The present list of pathogens is to be interpreted merely as examples and in no way as limiting.

The compounds of fomula (I) according to this invention, or the pharmaceutically acceptable salt thereof, may be used for the preparation of a medicament, and are suitable, for the prevention or treatment (and notably the treatment) of a bacterial infection.

As well as in humans, bacterial infections can also be treated using compounds of formula (I) (or pharmaceutically acceptable salts thereof) in other species like pigs, ruminants, horses, dogs, cats and poultry.

The present invention also relates to pharmacologically acceptable salts and to compositions and formulations of compounds of formula (I).

Any reference to a compound of formula (I), (I_{P}), (I_{CE}), (I_{CEP}), (I_{E1}), (I_{E1P}), (I_{E2}) or (I_{E1P}) is to be understood as referring also to the salts (and especially the pharmaceutically acceptable salts) of such compounds, as appropriate and expedient.

A pharmaceutical composition according to the present invention contains at least one compound of formula (I) (or a pharmaceutically acceptable salt thereof) as the active agent and optionally carriers and/or diluents and/or adjuvants, and may also contain additional known antibiotics.

The compounds of formula (I) and their pharmaceutically acceptable salts can be used as medicaments, e.g. in the form of pharmaceutical compositions for enteral or parenteral administration.

The production of the pharmaceutical compositions can be effected in a manner which will be familiar to any person skilled in the art (see for example Remington, The Science and Practice of Pharmacy, 21 st Edition (2005), Part 5, "Pharmaceutical Manufacturing" [published by Lippincott Williams & Wilkins]) by bringing the described compounds of formula (I) or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

Another aspect of the invention concerns a compound of formula (I) for use in a method for the prevention or the treatment (and notably the treatment) of a bacterial infection in a patient comprising the administration to said patient of a pharmaceutically active amount of a compound of formula (I) or a pharmaceutically acceptable salt thereof.

Besides, any preferences and (sub-)embodiments indicated for the compounds of formula (I) (whether for the compounds themselves, salts thereof, compositions containing the compounds or salts thereof, uses of the compounds or salts thereof, etc.) apply *mutatis mutandis* to compounds of formula (I_{P}), compounds of formula (ICE) and compounds of formula (I_{CEP}).

Moreover, the compounds of formula (I) may also be used for cleaning purposes, e.g. to remove pathogenic microbes and bacteria from surgical instruments or to make a room or an area aseptic. For such purposes, the compounds of formula (I) could be contained in a solution or in a spray formulation.

The compounds of formula (I) can be manufactured in accordance with the present invention using the procedures described hereafter.

### PREPARATION OF COMPOUNDS OF FORMULA (I)

### Abbreviations:

The following abbreviations are used throughout the specification and the examples:
- Ac: acetyl
- AcOH: acetic acid
- app.: apparent
- aq.: aqueous
- AD-mix α: (DHQ)₂PHAL, K₃Fe(CN)₆, K₂CO₃ and K₂OₛO₄.2H₂O
- AD-mix β: (DHQD)₂PHAL, K₃Fe(CN)₆, K₂CO₃ and K₂OsO₄.2H₂O
- Alloc: allyloxycarbonyl
- Boc: tert-butoxycarbonyl
- Bn: benzyl
- br.: broad
- Cbz: benzyloxycarbonyl
- CC: column chromatography over silica gel
- CDI: 1,1'-carbonyldiimidazole
- DBU: 1,8-diazabicyclo(5.4.0)undec-7-ene
- DCC: dicyclohexylcarbodiimide
- DCM: dichloromethane
- DCE: 1,2-dichloroethane
- DEAD: diethylazodicarboxylate
- (DHQ)₂PHAL: 1,4-*bis*(dihydroquinine)phthalazine
- (DHQD)₂PHAL: 1,4-*bis*(dihydroquinidine)phthalazine
- DIAD: diisobutylazodicarboxylate
- DIBAH: diisobutylaluminium hydride
- DIPA: *N,N*-diisopropylamine
- DIPEA: *N,N*-diisopropylethylamine
- DMA: *N,N*-dimethylacetamide
- DMAP: 4-dimethylaminopyridine
- DME: 1,2-dimethoxyethane
- DMF: *N,N*-dimethylformamide
- DMSO: dimethylsulfoxide
- DPEphos: bis(2-diphenylphosphinophenyl)ether
- DPPA: diphenyl phosphoryl azide
- EA: ethyl acetate
- EDCI: 1-(3-dimethylaminopropyl)-3-ethylcarbodiimide hydrochloride
- eq.: equivalent
- ESI: Electron Spray Ionisation
- Et: ethyl
- ether: diethyl ether
- Fmoc: 9-fluorenylmethoxycarbonyl
- Ft: phthaloyl
- HATU: (*O*-(7-azabenzotriazol-1-yl)-1,1,3,3-tetramethyl-uronium hexafluorophoshate
- Hept: heptane
- Hex: hexane
- HOBT: hydroxybenzotriazole
- HV: high vacuum
- KHMDS: potassium hexamethyldisilazide
- LDA: lithium diisopropylamide
- LG: leaving group
- LiHMDS: lithium hexamethyldisilazide
- mCPBA: *m*-chloroperbenzoic acid
- Me: methyl
- MeCN: acetonitrile
- min: minutes
- Ms: methanesulfonyl
- nBu: *n*-butyl
- NMO: *N*-methylmorpholine-*N*-oxide
- NMP: *N*-methylpyrrolidin-2-one
- org: organic
- Pd/C: palladium on charcoal
- PG: protecting group
- Ph: phenyl
- PTT: phenyltrimethylammonium tribromide
- Pyr: pyridine
- quant.: quantitative
- rac: racemic
- RaNi: Raney-Nickel
- rt: room temperature
- sat.: saturated
- TBAF: tetrabutyl ammonium fluoride
- TBDMS: *tert*-butyldimethylsilyl
- TBDPS: *tert*-butyldiphenylsilyl
- tBu: *tert-*butyl
- TBDMSOTf: *tert*-butyldimethylsilyltrifluoromethanesulphonate
- TEA: triethylamine
- TEMPO: 2,2,4,4-tetramethylpiperidine-1-oxyl
- Tf: trifluoromethanesulfonyl (triflyl)
- TFA: trifluoroacetic acid
- THF: tetrahydrofuran
- TMS: trimethylsilyl
- Ts: *p*-toluenesulfonyl
- wt%: percent in weight
- Z (in amino acids): benzyloxycarbonyl (in amino acids)

### General synthetic methods:

### General synthetic method 1 (reductive amination):

A solution of amine (1 mmol) and aldehyde or ketone (1 mmol) in DCE/MeOH 1:1 (10 mL) is stirred at rt overnight possibly in presence of a dessicant such as MgSO₄ or 3Å molecular sieves. NaBH₄ (2-5 eq.) is added and the reaction allowed to proceed for one hour. The reaction is diluted with DCM and aq. NH₄OH. The org. phase is washed with water, dried over MgSO₄ and concentrated. Alternatively, a solution of amine (1 mmol) and aldehyde or ketone (1 mmol) in DCE/MeOH 1:1 (10 mL) is treated with NaBH(OAc)₃ (2 eq.). The mixture is stirred at rt until complete conversion. The reaction is diluted with DCM and aq. NH₄OH. The org. phase is washed with water, dried over MgSO₄ and concentrated.

### General synthetic method 2 (alkylation of an amine):

The appropriate amine derivative is reacted with an alkylating agent bearing a leaving group such as OMs, OTf, OTs, Cl, Br or I in presence of an inorganic base such as K₂CO₃ or an org. base such as TEA in a solvent such as THF, DMF or DMSO between 0°C and +80°C. Further details can be found in Comprehensive Organic Transformations. A guide to Functional Group Preparations; 2nd Edition, R. C. Larock, Wiley-VC; New York, Chichester, Weinheim, Brisbane, Singapore, Toronto, (1999). Section Amines p.779.

### General synthetic method 3 (Wittig reaction):

The required phosphonium salt is treated in a solvent such as water with an inorganic base such as NaOH. The corresponding phosphorane is collected by filtration and dried in vacuo. It is reacted with the required aldehyde in an aprotic solvent such as THF, DCM or toluene between 0°C and 90°C. Alternatively the Wittig-Homer variant of the reaction can be used wherein the phosphono ester (generated from the corresponding bromide and triethylphosphite) is reacted with the adehyde in presence of a base such as NaH or NaOMe in a solvent such as ether or THF between 0°C and 50°C.

### General synthetic method 4 (reduction of a double bond):

The alkene derivatives dissolved in a solvent such as MeOH, EA or THF are hydrogenated over a noble metal catalyst such as Pd/C or PtO₂, or over RaNi. At the end of the reaction the catalyst is filtered off and the filtrate is evaporated under reduced pressure. Alternatively the reduction can be performed by catalytic transfer hydrogenation using Pd/C and ammonium formate as hydrogen source.

### General synthetic method 5 (removal of amino protecting groups):

The benzyl carbamates are deprotected by hydrogenolysis over a noble metal catalyst (e.g. Pd/C or Pd(OH)₂/C). The Boc group is removed under acidic conditions such as HCl in an organic solvent such as MeOH or dioxane, or TFA neat or diluted in a solvent such as DCM. The Fmoc group is removed by reaction in presence of an organic base such as morpholine or piperidine in a solvent such as MeCN. Further general methods to remove amine protecting groups have been described in T.W. Greene, P.G.M. Wuts, Protecting Groups in Organic Synthesis, 3rd Ed (1999), 494-653 (Publisher: John Wiley and Sons, Inc., New York, N.Y.).

### General synthetic method 6 (removal of hydroxy protecting groups):

The silyl ether groups are removed either using fluoride anion sources such as TBAF in THF between 0°C and +40°C or HF in MeCN or using acidic conditions such as AcOH in THF/MeOH or HCl in MeOH. Further methods to remove the TBDMS and TBDPS groups are given in T.W. Greene, P.G.M. Wuts, Protecting Groups in Organic Synthesis, 3rd Ed (1999), 133-139 and 142-143 respectively (Publisher: John Wiley and Sons, Inc., New York, N.Y.). Further general methods to remove alcohol protecting groups are described in T.W. Greene, P.G.M. Wuts, Protecting Groups in Organic Synthesis, 3rd Ed (1999), 23-147 (Publisher: John Wiley and Sons, Inc., New York, N.Y.). In the particular case of alkylcarboxy protecting group, the free alcohol can be obtained by the action of an inorganic base such as K₂CO₃ in a solvent such as MeOH.

### General synthetic method 7 (activation of an alcohol and substitution with an amine or an azide):

The alcohol is reacted with MsCl, TfCl or TsCl in presence of a base such as TEA in a dry aprotic solvent such as Pyr, THF or DCM between -30°C and 50°C. In the case of the triflate or mesylate, Tf₂O or Ms₂O can also be used. These sulfonates can be reacted with sodium iodide in a ketone such as acetone or 2-butanone, in MeCN or in DMF between 40°C and 120°C delivering the corresponding iodide derivatives. Once activated (either as a sulphonate or a iodide derivative), the alcohol reacts with an amine or sodium azide in presence of an organic base such as DIPEA or TEA or an inorganic base such as sodium carbonate in a solvent such as DMSO or DMF between 20°C and 100°C. Alternatively, the azide can also be obtained by activation of the alcohol under Mitsunobu conditions in presence of PPh₃ and DEAD or DIAD in a solvent such as THF, DMF, DCM or DME between -20°C and 60°C as reviewed by O. Mitsunobu, in Synthesis (1981), 1 and reaction with DPPA.

### General synthetic method 8 (reduction of azides into amines):

The azides are hydrogenated over a noble metal catalyst such as Pd/C in a solvent such as MeOH or EA. In case the molecule is containing a double or triple bond, the reduction can be performed using PPh₃ in presence of water as described in J. Med. Chem. (1993), 36, 2558-68.

### General synthetic method 9 (oxidation of alcohols/aldehydes into acids):

Alcohols can be directly oxydized into their corresponding acids by a variety of methods as described in Comprehensive Organic Transformations. A guide to Functionnal Group Preparations; 2nd Edition, R. C. Larock, Wiley-VC; New York, Chichester, Weinheim, Brisbane, Singapore, Toronto, 1999. Section nitriles, carboxylic acids and derivatives p.1646-1648. Among them, [bis(acetoxy)iodo]benzene in presence of TEMPO, the Jones reagent (CrO₃/H₂SO₄), NaIO₄ in presence of RuCl₃, KMnO₄ or Pyr.H₂Cr₂O₇ are preferably used.

Aldehydes can be oxidized into their corresponding acids by a variety of methods as described in Comprehensive Organic Transformations. A guide to Functionnal Group Preparations; 2nd Edition, R. C. Larock, Wiley-VC; New York, Chichester, Weinheim, Brisbane, Singapore, Toronto, 1999. Section nitriles, carboxylic acids and derivatives, p. 1653-1655. Among them, KMnO₄ in an acetone-water mixture (see Synthesis (1987), 85) or sodium chlorite in 2-methyl-2-propanol in presence of 2-methyl-2-butene (see Tetrahedron (1981), 37, 2091-2096) are preferably used.

### General synthetic method 10 (cis-dihydroxylation):

Diols are obtained by dihydroxylation of the corresponding olefine using a catalytic amount of osmium tetroxide in the presence of a co-oxidant such as NMO in an aq. solvent such as an acetone-water or DCM-water mixture (see Cha, J.K. Chem. Rev. (1995), 95, 1761-1795). Enantiomerically pure or enriched chiral cis-diols are obtained by using AD-mix α or AD-mix β in presence of methanesulfonamide in a water/tBuOH mixture as described in Chem. Rev. (1994), 94, 2483. The sense of induction relies on the chiral ligand contained in the AD mixture, either a dihydroquinine-based ligand in AD-mix α or a dihydroquinidine-based ligand in AD-mix β.

### General synthetic method 11 (aldehyde reduction);

Aldehydes are reduced with a boron or aluminium hydride reducing agent such as NaBH₄, LiBH₄ or LiAlH₄ in a solvent such as THF or ether between -20°C and 40°C.

### General synthetic method 12 (amine protection):

Amines are usually protected as carbamates such as Alloc, Cbz, Boc or Fmoc. They are obtained by reacting the amine with allyl or benzyl chloroformate, di *tert*-butyl dicarbonate or FmocCl in presence of a base such as NaOH, TEA, DMAP or imidazole. They can also be protected as N-benzyl derivatives by reaction with benzyl bromide or chloride in presence of a base such as Na₂CO₃ or TEA. Alternatively, N-benzyl derivatives can be obtained through reductive amination in presence of benzaldehyde and a borohydride reagent such as NaBH₄, NaBH₃CN or NaBH(OAc)₃ in a solvent such as MeOH, DCE or THF. Further strategies to introduce other amine protecting groups have been described in T.W. Greene, P.G.M. Wuts, Protecting Groups in Organic Synthesis, 3rd Ed (1999), 494-653 (Publisher: John Wiley and Sons, Inc., New York, N.Y.).

### General preparation method:

### Preparation of the compounds of formula (I):

The compounds of formula (I) can be manufactured by the methods given below, by the methods given in the examples or by analogous methods. Optimum reaction conditions may vary with the particular reactants or solvents used, but such conditions can be determined by a person skilled in the art by routine optimisation procedures.

Sections a) to f) hereafter describe general methods for preparing the compounds of formula (I). If not indicated otherwise, the generic groups or integers m, q, r, s, t, u, A, B, G, Q, R¹, R², U, V, W, X and Y are as defined for formula (I). In some instances generic groups as used below might be incompatible with the assembly illustrated in the following schemes and so will require the use of protecting groups (PG). The use of protecting groups is well known in the art (see for example "Protective Groups in Organic Synthesis", T.W. Greene, P.G.M. Wuts, Wiley-Interscience, 1999). General synthetic methods used repeatedly throughout the text below are referenced to and described in the above section entitled "General synthetic methods".
a) The compounds of formula (I) wherein m is 1, A is -NHCH₂-^{#}, -CH₂NH-^{#}, - NHCH₂CH₂-^{#}, -CH2NHCH2-, -CH₂CH₂NH-^{#} or -CH₂NHCH₂CH₂-^{#}, wherein the hash indicates the bond which is linked to B, and B is a bond can be manufactured in accordance with the present invention by reacting the compounds of formula (II) 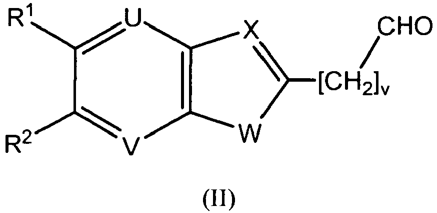 wherein v represents 0, 1 or 2 with the appropriate compounds of formula (III) 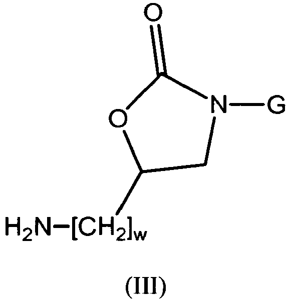 wherein w represents 1, 2 or 3, following general synthetic method 1.
b) The compounds of formula (I) wherein m is 1, W represents *-CH=CR^{a}- or *-N=CH-, wherein the asterisks indicate the bond which is linked to the carbon atom connecting V and W and wherein R^{a} represents hydrogen or halogen, A represents -CH₂NHCH₂-and B represents a bond can be manufactured in accordance with the present invention by reacting the compounds of formula (IV) 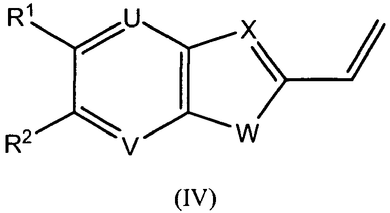 wherein X represents N and W represents -CH=CH- with the appropriate compounds of formula (III) wherein w represents 2, in the presence of an acid such as AcOH between 60°C and 120°C.
c) The compounds of formula (I) wherein m is 0, A is -OCH₂-^{#}, wherein the hash indicates the bond which is linked to B, and B is a group of formula (E) can be manufactured in accordance with the present invention by reacting the compounds of formula (V) 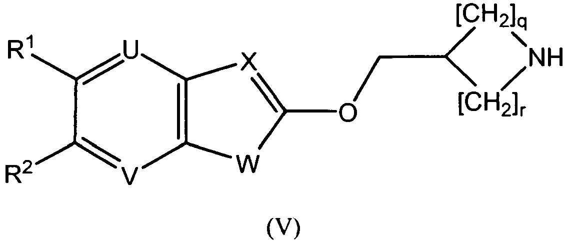 with the appropriate compounds of formula (VI) 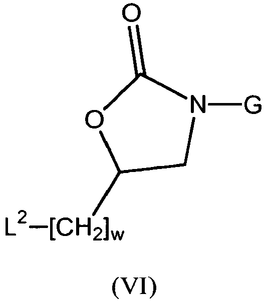 wherein w is 1 or 2 and L² is a halogen such as iodide, or a group of formula -OSO₂R^{d} wherein R^{d} is methyl, CF₃ or p-tolyl, according to general synthetic method 2.
d) The compounds of formula (I) wherein m is 0, A is O and B is a group of formula (F) can be manufactured in accordance with the present invention by reacting the compounds of formula (VII) 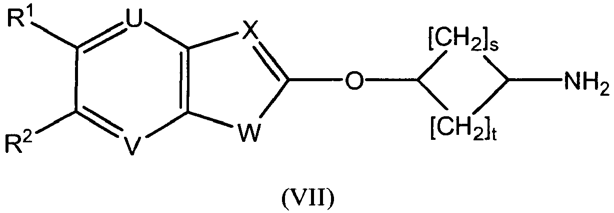 with the appropriate compounds of formula (VI) wherein w is 1.
e) The compounds of formula (I) can also be obtained by a nucleophilic aromatic substitution reaction or a Pd- or Cu- catalyzed coupling reaction (as described for example in Org. Lett. (2006), 8, 5609-5612) from the compounds of formula (VIII) 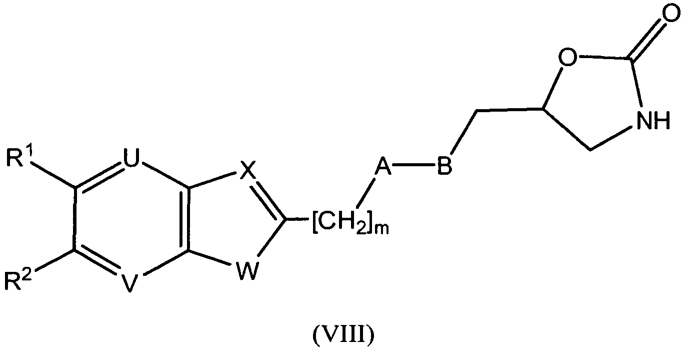 and the appropriate compounds of formula (IX)

   G-LG² (IX)

   wherein LG² represents halogen or OTf.
f) The compounds of formula (I) wherein either m is 1, A is -NHCH₂CH₂NH- or piperazin-1,4-diyl and B represents a bond or m is 0, A represents -NHCH₂CH₂NHCH₂-^{#} wherein the hash indicates the bond which is linked to B, and B represents a bond can be manufactured in accordance with the present invention by reacting the compounds of formula (X) 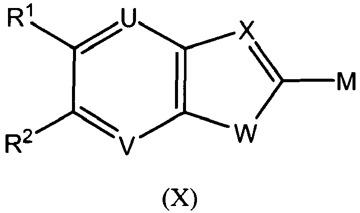 wherein M represents -CH₂NHCH₂CH₂NH₂, -NHCH₂CH₂NH₂ or piperazin-1-ylmethyl with the appropriate compounds of formula (VI) according to general synthetic method 2.

### Preparation of the compounds of formulae II to IX:

The compounds of formula (II) can be obtained as described in Scheme 1 hereafter.

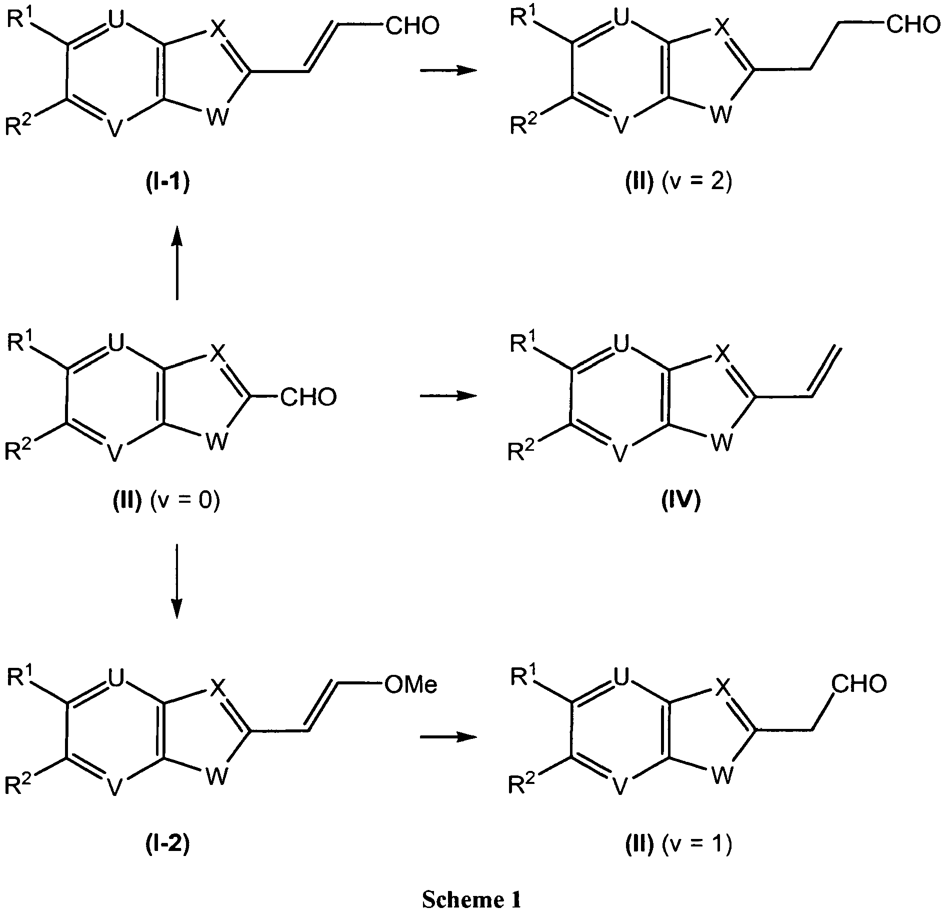

The aldehydes of formula (II), wherein v is 0, are reacted with formyl methylene triphenylphosphorane following general synthetic method 3 and the resulting unsaturated aldehydes of formula (I-1) are reduced to the corresponding aldehydes of formula (II), wherein v is 2, following general synthetic method 4. The aldehydes of formula (II), wherein v is 0, are also reacted with methoxy methylenetriphenylphosphorane following general synthetic method 3 and the resulting enolethers (I-2) are transformed into the corresponding aldehydes of formula (II), wherein v is 1, by treatment with an acid such as aq. hydrochloric acid.

The compounds of formula (IV) can be obtained by reaction of aldehydes of formula (II), wherein v is 0, with methylenetriphenylphosphorane following general synthetic method 3. The compounds of formulae (V) and (VII) can be obtained as described in Scheme 2 hereafter.

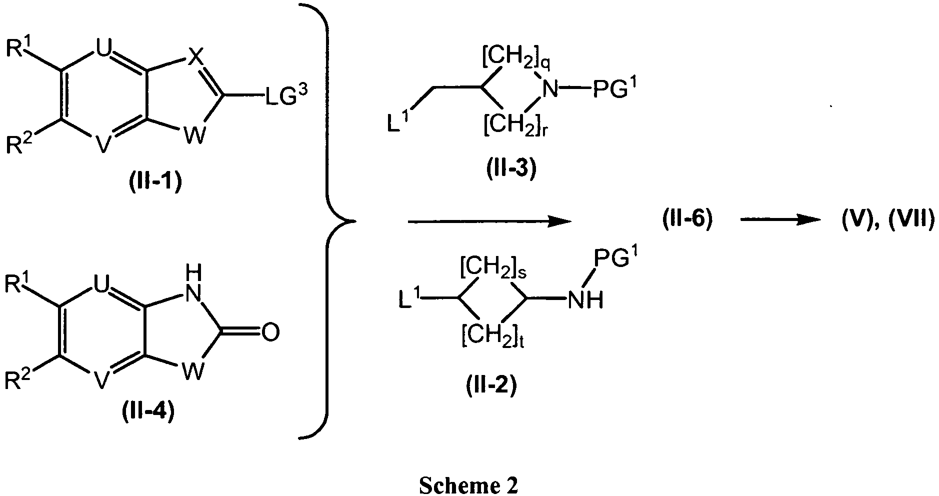

In Scheme 2, LG³ represents a halogen, L¹ represents hydroxy, halogen or OMs and PG¹ represents an amino protecting group such as Cbz or Boc.

The derivatives of formula (II-1) can be reacted with the corresponding derivatives of formulae (II-2) or (II-3) wherein L¹ is OH in the presence of a base such as NaH or tBuOK to give the *N*-protected intermediates (II-6). Alternatively, the derivatives of formula (II-4) can be reacted with the corresponding derivatives of formulae (II-2) or (II-3) wherein L¹ is OH under Mitsunobu conditions (Synthesis (1981), 1) or wherein L¹ is halogen or mesylate in the presence of a base such as NaH, Cs₂CO₃ or K₂CO₃. The resulting compounds of formula (II-6) can then be *N*-deprotected following general synthetic method 5, affording the compounds of formula (V) or (VII).

The compounds of formula (VIII) are prepared as described in Scheme 3 hereafter.

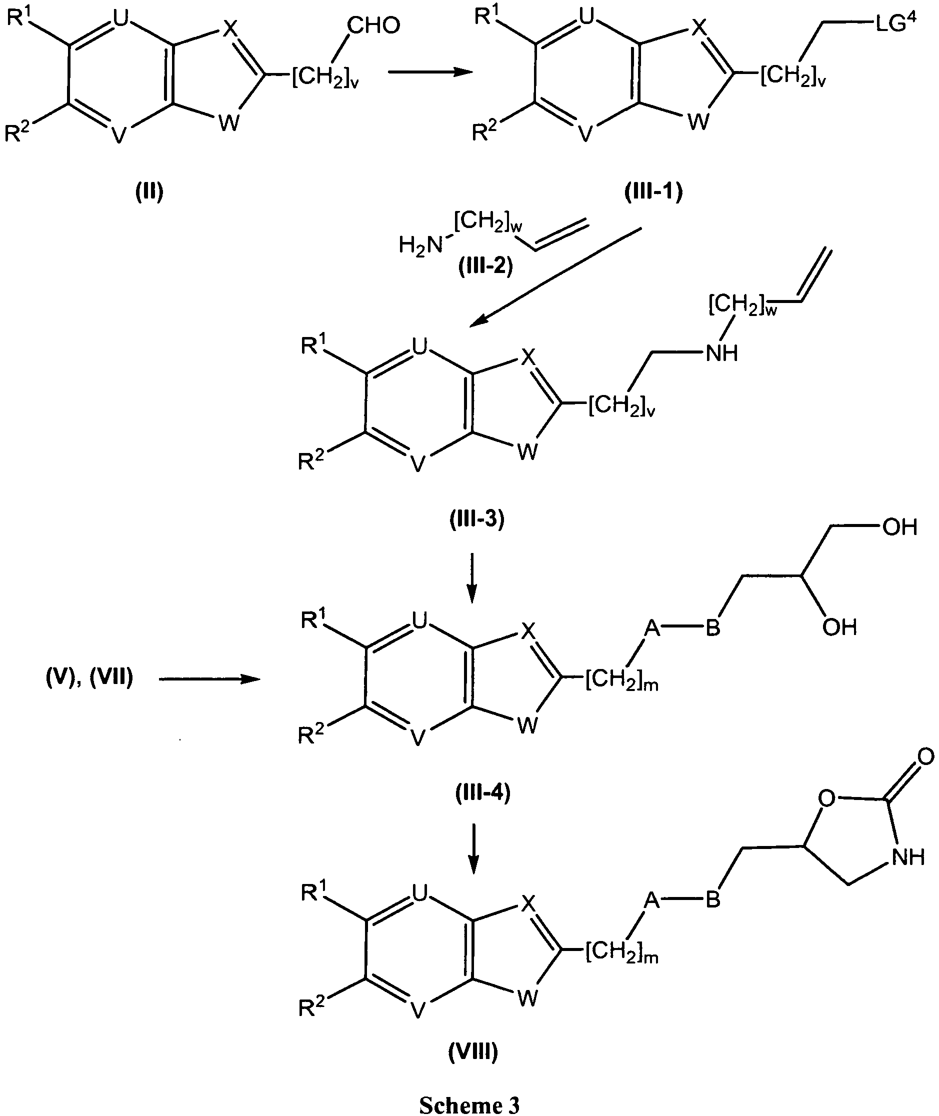

In Scheme 3, LG⁴ represents halogen such as iodine or bromine, or OSO₂R^{d} wherein R^{d} is methyl, CF₃ or p-tolyl; v represents 0, 1 or 2 and w represents 1, 2, 3 and v plus w is 3.

The oxazolidinones of formula (VIII) can be formed from diols of formula (III-4) by sequential activation of the primary alcohol, following the activation step of general synthetic method 7, epoxide formation by treatment with a base such as K₂CO₃, reaction with sodium azide followed by hydrogenation over a noble metal catalyst such as Pd/C or reduction in presence of PPh₃/H₂O, subsequent transformation into their corresponding carbamates with CbzCl or Boc₂O and subsequent ring closure with a base like NaH. The intermediates of formula (III-4) can be obtained either by reaction of the intermediates of formulae (V) and (VII) with an allyl- or homoallyl-halogenide, followed by cis-dihydroxylation using general synthetic method 10, or by cis-dihydroxylation of the derivatives of formula (III-3); secondary amines need to be protected following general synthetic method 12 prior to *cis*-dihydroxylation. The intermediates of formula (III-3) can be obtained by sequential reduction of aldehydes of formula (II) following general synthetic method 11, activation of the resulting alcohol and reaction with an amine of formula (III-2) following general synthetic method 7. Alternatively they can be obtained through reductive amination between the aldehydes of formula (II) and the amines of formula (III-2) following general synthetic method 1. If required, the amino protecting group can be removed group following general synthetic method 5.

The compounds of formula (III) and (VI) can be prepared as described in Scheme 4 hereafter.

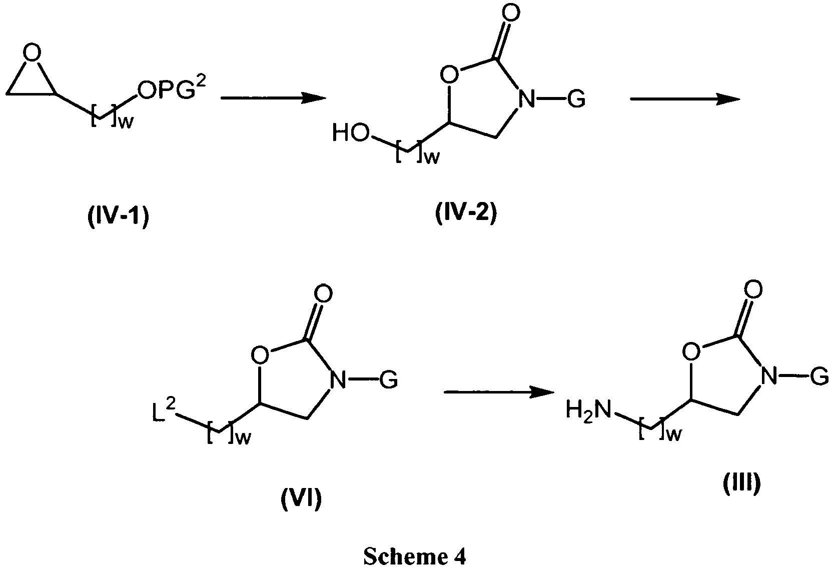

In Scheme 4, PG² represents an alcohol protecting group such as TBDMS, TBDPS or -C(O)R^{e}, wherein R^{e} represents (C₁₋₄)alkyl, L² represents N₃, halogen or OSO₂R^{d} wherein R^{d} is methyl, CF₃ or *p*-tolyl.

The compounds of formula (III) can be obtained from the corresponding derivatives of formula (VI), wherein L² represents azide, following general synthetic method 8. Compounds of formula (VI) can be obtained from the compounds of formula (IV-2) following general synthetic method 7. The alcohols of formula (IV-2) can be obtained by reaction of the epoxides of formula (IV-1) with the anions of the carbamates of formula GNHCOOR wherein R represents (C₁₋₄)alkyl (preferably methyl or ethyl) or benzyl in presence of a base such as KHMDS or lithium tert-butylate, followed by alcohol deprotection as described in general synthetic method 6. Alternatively the epoxides of formula (IV-1) can be reacted with the amines of formula GNH₂ in presence of LiClO₄ and the resulting aminoalcohol derivatives can be reacted with CDI and the alcohol protecting group can be removed following general synthetic method 6, affording the intermediates of formula (IV-2).

The compounds of formula GNH₂ are either commercially available or prepared from the known benzylic alcohols of formula (V-1) as described in Scheme 5 hereafter.

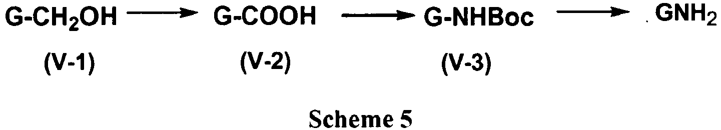

The known benzylic alcohols of formula (V-1) can be oxidised into the corresponding carboxylic acids following general synthetic method 9. The resulting carboxylic acids of formula (V-2) can then be reacted with diphenylphosphoryl azide in the presence of tBuOH between 40° and 100°C affording the carbamates of formula (V-3). The compounds of formula GNH₂ can then be obtained following general synthetic method 5.

The compounds of formula (IX) are commercially available (e.g. IX-a = G1-LG² wherein LG² is halogen, Q=O; Y=N: CAS 337463-99-7; Q=S; Y=CH CAS 6376-70-1; Q=O; Y=CH CAS 7652-29-1). The compound of formula (IX-a) = G1-LG² wherein Y is N and Q is S can be obtained as described in Scheme 6 hereafter.

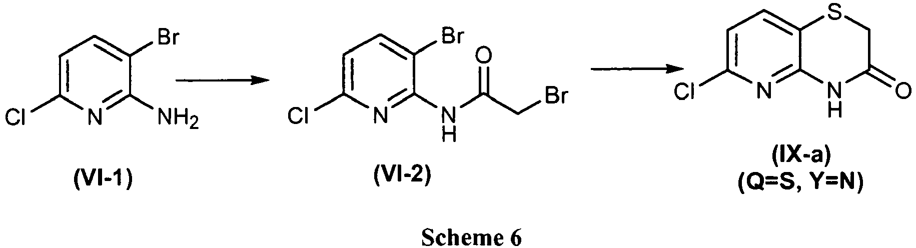

The bromo derivative of formula (VI-1), prepared according to WO 2008/065198, can be reacted with bromoacetyl bromide and the resulting derivative of formula (VI-2) can be reacted with sodium thioacetate in presence of sodium methylate, affording the compound of formula (IX-a) wherein Q = S and Y = N.

The compounds of formula (IX-a) = GI-LG² wherein Y is CH, Q is O or S and LG² is OTf and the compounds of formula (IX-b) = G2-LG² wherein Z¹, Z² and Z³ are CH and LG² is OTf can be obtained by reaction of the corresponding compounds of formula (IX-a) or (IX-b) wherein LG² is OH with Tf₂O. The compound of formula (IX-b) = G2-LG² wherein Z¹, Z² and Z³ are CH and LG² is iodine is commercial (CAS 57744-67-9). The respective intermediates of formula (IX-a) or (IX-b) wherein LG² is OH are either commercially available (CAS 53412-38-7; CAS 10288-72-9) or prepared according to EP 106816.

The compounds of formula (X) wherein M represents -NHCH₂CH₂NH₂ can be obtained as described in Scheme 7 hereafter.

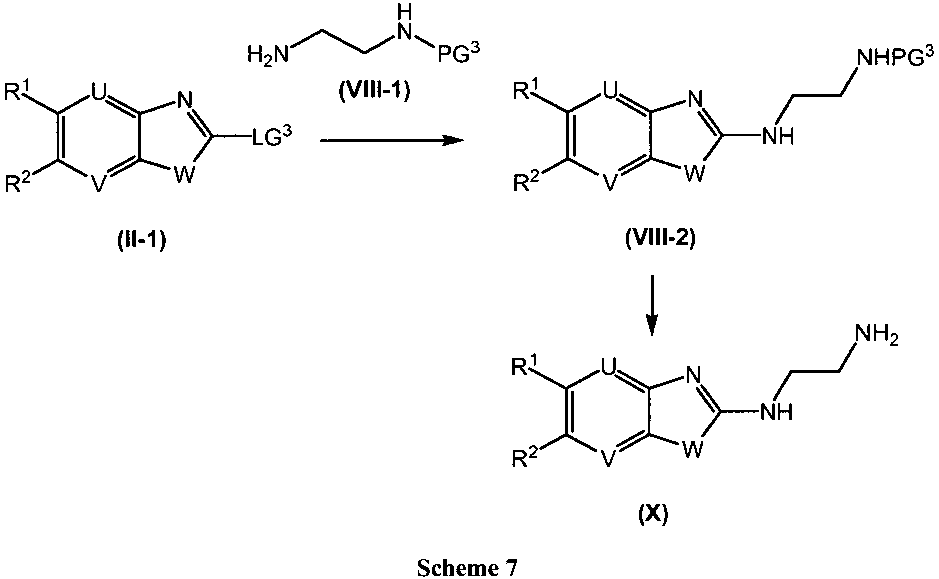

The compounds of formula (II-1) can be reacted (Scheme 8) with the compounds of formula (VIII-1) (wherein PG³ represents an amino protecting group such as Cbz or Boc). The protecting group PG³ can then be removed according to general synthetic method 5.

The compounds of formula (X) wherein M represents -CH₂NHCH₂CH₂NH₂ or piperazin-1-ylmethyl can be obtained by reacting the aldehydes of formula (II) with either (2-amino-ethyl)-carbamic acid *tert*-butyl ester or piperazine according to general synthetic method 1, followed, in the first case, by removal of the amino protecting group using general synthetic method 5.

### Preparation of the elaborated intermediates of formulae (II) and (IV-1):

The compounds of formula (II) wherein v is 0, W is S, X is N, U is CH, V is CH and R¹ is hydrogen, fluorine or methoxy are commercially available or can be prepared according to e.g. US 5,322,847.

The compounds of formula (II) wherein v is 0, W is S, X is CH, U is CH or N, V is CH or N and R¹ is hydrogen, fluorine or methoxy are commercially available or can be prepared according to e.g. WO 2004/22551 or Bioorg. Med. Chem. (2004), 12, 2251-2273.

The compounds of formula (II) wherein v is 0, W is CH=CR^{a}, X is CH or N, U is CH or N, V is CH or N and R¹ is hydrogen, fluorine or methoxy are commercially available or can be prepared according to e.g. Org. Biomol. Chem. (2005), 3(14), 2543-57, Sciences of Synthesis (2005), 15, 389-549 or WO 2006/132739.

The compounds of formula (II) wherein v is 0, W is *-N=CH-, wherein the asterisk indicates the bond which is linked to the carbon atom connecting V and W, X is CH or N, U is CH or N, V is CH or N, with the proviso that at least one of X, U and V are CH, and R¹ is hydrogen, fluorine or methoxy are commercially available or can be prepared according to WO 2006/009734.

The compounds of formula (II) wherein v is 0 can also be obtained as described in Scheme 8 hereafter.

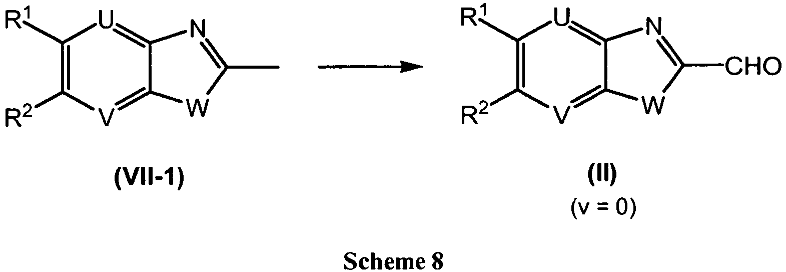

The compounds of formula (II) wherein v is 0 can thus be obtained by SeO₂ oxidation of the corresponding methyl derivative of formula (VII-1). The compounds of formula VII-1 are either commercially available (e.g. CAS 399-75-7, 30489-80-6, 857970-22-0, 1831-88-5, 1128-74-1 or 19490-87-0) or can be prepared in analogy to known methods (e.g. reaction of a commercially available aniline precursor with crotonaldehyde (Tetrahedron Lett. (2006), 47(11), 1783-1785) or reaction of an o-phenylenediamine derivative with hydroxyacetone (Synlett (2005), 6, 1003-1005).

The compounds of formula (II-2) and (II-3), wherein L¹ is halogen or mesylate, can be derived from the respective, commercially available alcohol (II-2) or (II-3), wherein L¹ is hydroxy, following the first step of general synthetic method 7.

The compounds of formula (II-1) are commercially available (U = V = CH and W = S) or can be prepared according to J. Med. Chem. (2008), 51(5), 1492-1495; J. Med. Chem. (1981), 24(1), 93-101; WO 98/43975 or Gazz. Chim. Ital. (1966), 96(11), 1456-69.

The compounds of formula (II-4) wherein W is S, R¹ is MeO or Br and U = V = CH or wherein W is S, R¹ is Cl, R² is H, U = CH and V = N are commercially available (CAS 15193-51-8, 199475-45-1 and 112523-34-9) or can be prepared according to WO 2008/009700 or WO 2003/011868. The other compounds of formula (II-4) wherein W is -CH=CH- are commercially available (e.g. U = V = CH) or can be prepared according to WO 2008/071961; J.Heterocycl. Chem. (1986), 23(2), 501-4; WO2006/1343 or WO 2008/009700.

The compounds of formula (IV-1) wherein w is 1 and PG² is -C(O)R^{e}, wherein R^{e} represents (C₁₋₄)alkyl, are commercially available. The compounds of formula (III-1) wherein w is 2 or 3 and PG² is TBDMS can be prepared according to WO 2007/144423 or EP 518672.

The compounds of formula GNHCOOR can be prepared from the corresponding aniline derivatives GNH₂ and the corresponding chloroformate.

### EXAMPLES

All temperatures are stated in °C. Compounds are characterized by ¹H-NMR (300 MHz) (Varian Oxford); or by ¹H-NMR (400 MHz) (Bruker Avance 400). Chemical shifts δ are given in ppm relative to the solvent used; multiplicities: s = singlet, d = doublet, t = triplet, q = quadruplet, p = pentuplet, hex = hexet, hep = heptet, m = multiplet, br = broad, coupling constants are given in Hz. Alternatively compounds are characterized by LC-MS (Sciex API 2000 with Agilent 1100 Binary Pump with DAD and ELSD or an Agilent quadrupole MS 6140 with Agilent 1200 Binary Pump, DAD and ELSD); by TLC (TLC-plates from Merck, Silica gel 60 F₂₅₄); or by melting point. Compounds are purified by chromatography on Silica gel 60A. NH₄OH as used for CC is 25% aq.

The HPLC are done over a stationary phase such as a rapid resolution Zorbax SB C 18 (1.8µm) column, or a rapid resolution Zorbax Eclipse Plus C18 (1.8µm) column. Typical conditions of HPLC are a gradient of eluent A (water: acetonitrile 95:5 with 0.1% of formic acid, in presence or not of 5 mmol/L ammonium formate) and eluent B (acetonitrile: water 95:5 with 0.1% of formic acid, in presence or not of 5 mmol/L ammonium formate), at a flow rate of 0.8 to 5 mL/min.

Racemates can be separated into their enantiomers as described before. Preferred conditions of chiral HPLC are: ChiralPak AD (4.6x250mm, 5µm) column, using an isocratic mixture (eg. at a ratio of 10/90) of eluent A (EtOH, in presence of the appropriate amount of diethylamine; eg. 0.1%) and eluent B (Hex), at rt, at a flow rate of e.g. 0.8 mL/min.

### General procedures:

### Procedure A: reductive amination:

A solution of amine (1 mmol) and aldehyde or ketone (1 mmol) in DCE/MeOH 1:1 (10 mL) is stirred at rt overnight possibly in presence of a dessicant such as MgSO₄ or 3Å molecular sieves. NaBH₄ (2-5 eq) is added and the reaction allowed to proceed for one hour. The reaction is diluted with DCM and aq. NH₄OH. The org. phase is washed with water, dried over MgSO₄ and concentrated. Alternatively, a solution of amine (1 mmol) and aldehyde or ketone (1 mmol) in DCE/MeOH 1:1 (10 mL) is treated with NaBH(OAc)₃ (2 eq). The mixture is stirred at rt until complete conversion. The reaction is diluted with DCM and aq. NH₄OH. The org. phase is washed with water, dried over MgSO₄ and concentrated. The products are purified by CC (DCM/MeOH + NH₄OH) or prep. HPLC (Waters XBridge C18, 5uM, ODB, 19x50mm, MeCN/0.5%NH4OH, 10:90 to 95:5, flow: 40 mL/min)

### Procedure B: alkylation of amines with mesylates,

A solution of amine (1.0-2.3 mmol), mesylate (1 mmol) and DIPEA (1-1.1 mmol) in dry DMSO is heated to 60-80°C until completion of the reaction (1-5 days). After cooling, water and EA are added and the phases are separated. The aq. layer is extracted two more times with EA and the combined org. layers are washed with water (3x) and brine, dried over MgSO₄ and concentrated under reduced pressure. The residue is then purified by CC.

### Procedure C; alkylation of amines with iodides;

A solution of amine (1 mmol), iodide (1 mmol) and DIPEA (1.1 mmol) in dry DMSO is heated to 70°C until completion of the reaction (1-3 days). After cooling, water and EA are added and the phases are separated. The aq. layer is extracted two more times with EA and the combined org. layers are washed with water (3 x) and brine, dried over MgSO₄ and concentrated under reduced pressure. The residue is then purified by CC.

### Procedure D: Boc deprotection:

The Boc protected amine (1 mmol) is dissolved in DCM (5 mL) and treated with Et₃SiH (optional; 0.2 mL, 1.1 eq.) and TFA (2 mL). The mixture is stirred at rt for 1 h, concentrated *in vacuo* and taken up in DCM/aq. NH₄OH. The org. layer is washed with water, dried over MgSO₄ and concentrated under reduced pressure.

### Preparation of intermediates:

### Intermediate 1: methanesulfonic acid (S)-2-oxo-3-(3-oxo-3,4-dihydro-2H-benzo[1,4]oxazin-6-yl)-oxazolidin-5-ylmethyl ester:

### 1.i. 6-[(S)-3-(tert-butyl-dimethyl-silanyloxy)-2-hydroxy-propylamin]-4H-benzo[1,4]oxazin-3-one:

To a solution of *tert*-butyl-dimethyl-((S)-1-oxiranylmethoxy)-silane (commercial; 4.25 g, 22.6 mmol) in MeCN (70 mL) was added LiClO₄ (7.20 g, 3 eq). 6-amino-4*H*-benzo[1,4]oxazin-3-one (commercial; 3.70 g, 1 eq) was then added and the mixture was stirred at 50°C for 6 h. The solvent was removed under reduced pressure and the residue was purified by CC (DCM/MeOH/NH₄OH 1000:25:2) to afford the title compound as a pale brown foam (5.25 g, 66% yield).
MS (ESI, m/z): 353.3 [M+H]⁺.

### 1.ii. 6-[(S)-5-(tert-butyl-dimethyl-silanyloxymethyl)-2-oxo-oxazolidin-3-yl]-4H-benzo[1,4]oxazin-3-one:

A solution of intermediate 1.i (10.24 g, 29 mmol) and CDI (9.71 g, 2 eq.) in THF (140 mL) was heated at 50°C for 2 h; the mixture was concentrated under reduced pressure and partitioned between EA and water. The organic layer was washed with water and brine, dried over MgSO₄, concentrated and triturated with Et₂O to afford the title compound as a pale yellow solid (6.30 g, 57% yield).
MS (ESI, m/z): 379.2 [M+H]⁺.

### 1.iii. 6-((S)-5-hydroxyntethyl-2-oxo-oxazolidin-3-yd)-4H-benzo[1,4]oxazin-3-one:

A suspension of intermediate 1.ii (6.30 g, 16.6 mmol) in THF (15 mL) was treated with TBAF (1*M* in THF, 16.6 mL) at 0°C. The solution was stirred at 0°C for 3 h and then partitioned between water and EA. The aq. phase was extracted with EA (3x). The combined org. layers were washed with brine, dried over MgSO₄, and concentrated. The residue was triturated with EA to afford the title compound as a colourless solid (3.49 g, 79% yield).
MS (ESI, m/z): 265.5 [M+H]⁺.

### 1.iv. Methanesulfonic acid (S)-2-oxo-3-(3-oxo-3,4-dihydro-2H-benzo[1,4]oxazin-6-yl)-oxazolidin-5-ylmethyl ester:

A suspension of intermediate 1.iii (4.93 g, 18.7 mmol) in anhydrous DCM (110 mL) was treated with DIPEA (12.0 mL, 3.75 eq.) and the mixture was cooled to 0°C. Ms₂O (4.88 g, 1.5 eq.) was added portionwise. The resulting mixture was stirred at 0°C for 15 min. Water was added and stirring was continued for 15 min at rt. The precipitated product was filtered, washed with water and DCM. The thus obtained solid was triturated with DCM/MeOH/NH₄OH (1000/25/2) to give the title compound as a colourless solid (3.785 g, 60% yield).
1H NMR (DMSO-d6) δ: 10.72 (s, 1H); 7.29 (dd, J = 2.1, 0.6 Hz, 1H); 6.94 (m, 2H); 4.95 (m, 1H); 4.52 (s, 2H); 4.49 (m, 2H); 4.11 (t, J = 9.1 Hz, 1H); 3.73 (m, 2H); 3.23 (s, 3H).
MS (ESI, m/z): 343.3 [M+H]⁺.

### Intermediate 2: 6-((S)-5-iodomethyl-2-oxo-oxazolidin-3-yl)-4H-benzo[1,4]thiazin-3-one:

### 2.i. 6-((S)-3-chloro-2-hydroxy-piopylamino)-4H-benzo[1,4]thiazin-3-one:

A suspension of 6-amino-4H-benzo[1,4]thiazin-3-one (18.0 g, 100 mmol; commercial) and Ca(OTf)₂ (0.5 eq.) in MeCN (800 mL) was heated at 50° for 1 h. (*S*)-epichlorohydrin (18.5 g, 200 mmol) was added and the mixture was stirred at rt for 72 h and at 45°C for 24 h. The volatiles were removed under reduced pressure. After aq. workup and extraction with EA, the title compound crystallized from EA as a beige solid (17.38 g, 64% yield). MS (ESI, m/z): 273.2 [M+H]⁺.

### 2.ii. 6-((S)-5-chlononaethyl-2-oxo-oxazolidin-3-yl)-4H-benzo[1,4]thiazin-3-one:

A solution of intermediate 2.i (39.3 g, 144 mmol) and CDI (28.0 g, 1.2 eq.) in THF (1 L) was heated at 50°C overnight. The mixture was concentrated under reduced pressure and partitioned between EA and water. The aq. layer was extracted once more with EA and the combined org. layers were dried over MgSO₄ and concentrated. The residue was purified by CC (EA/Hept 2:1, EA) to afford the title compound as a beige solid (34.2 g, 79% yield).
MS (ESI, m/z): 299.1 [M+H]⁺.

### 2.iii. 6-((S)-5-iodomethyl-2-oxo-oxazolidin-3-yl)-4H-benzo[1,4]thiazin-3-one:

A mixture of intermediate 2.ii (14.0 g, 46.9 mmol) and NaI (3 eq.) in 2-butanone (150 mL) was heated at 85°C for 2 days. After cooling to rt, the mixture was diluted with 10% aq. (300 mL) and ether/EA (150 mL). The mixture was vigorously stirred for 10 min and filtered. The solids were thoroughly washed with water and ether and dried under HV to afford a pale beige solid. The phases of the combined filtrates were separated and the org. phase washed with brine, dried over MgSO₄ and concentrated to afford a pale beige solid. The solids of both processes were combined to afford the title compound as a pale beige solid (15.0 g, 82% yield).
¹H NMR (DMSO-d6) δ: 10.56 (s, 1H); 7.31 (m, 2H); 7.12 (dd, J = 8.5, 2.3 Hz, 1H); 4.71 (m, 1H); 4.14 (t, J = 9.1 Hz, 1H); 3.59 (m, 3H); 3.31 (s, 2H).
MS (ESI, m/z): 391.4 [M+H]⁺.

### Intermediate 3: (S)-3-(2,3-dihydro-benzo[1,4]dioxin-6-yl)-5-iodomethyl-oxazolidin-2-one:

### 3.i. (S)-3-(2,3-dihydro-benzo[1,4]dioxin-6-yl)-5-hydroxymethyl-oxazolidin-2-one:

A solution of (2,3-dihydro-benzo[1,4]dioxin-6-yl)-carbamic acid benzyl ester (13.0 g, 45.6 mmol) in THF (220 mL) was cooled to -78°C before the drop wise addition of nBuLi (29.5 mL of a 2.36M solution in hexanes, 1.1 eq). The mixture was stirred at -78°C for 1 h and then warmed to -15°C. At this temperature (S)-glycidyl butyrate (7.37 g, 1.1 eq.) was added dropwise. The mixture was stirred at rt overnight. Cs₂CO₂ (tip of a spatula) was added and the mixture heated at 40°C until complete conversion. The mixture was diluted with EA and washed with a sat. aq. NH₄Cl and water. The org. layer was dried over MgSO₄ and concentrated. The residue was purified by CC (Hex/EA 2:1, 1:1) to afford the title compound as a grey solid (7.04 g, 62% yield).
¹H NMR (DMSO-d6) δ: 7.13 (d, J = 2.5 Hz, 1H), 6.96 (dd, J = 2.5, 8.9 Hz, 1H), 6.86 (d, J = 8.9 Hz, 1H), 5.16 (t, J = 5.8 Hz, 1H), 4.70-4.50 (m, 1H), 4.30-4.10 (m, 4H), 4.10-3.90 (m, 1H), 4.80-4.70 (m, 1H), 4.70-4.60 (m, 1H), 4.60-4.50 (m, 1H).

### 3.ii. Methanesulfonic acid (S)-3-(2,3-dihydro-benzo[1,4]dioxin-6-yl)-2-oxo-oxazodidin-5-ylmethyl ester:

A solution of intermediate 3.i (7.0 g, 27.9 mmol) in DCM (140 mL) was cooled to 0°C. DIPEA (5.70 mL, 1.2 eq) and MsCl (2.40 mL, 1.1 eq.) were added and the mixture was stirred for 1 h at 0°C. The mixture was diluted with DCM and washed with water. The org. phase was dried over MgSO₄ and concentrated to give the title compound as a colourless solid (9.0 g, 98% yield).
MS (ESI, m/z): 330.3 [M+H]⁺.

### 3.iii. (S)-3-(2,3-dihydro-benzo[1,4]dioxin-6-yl)-5-iodomethyl-oxazolidin-2-one:

A mixture of intermediate 3.ii (9.0 g, 27.3 mmol) and NaI (16.4 g, 4 eq.) in acetone (150 mL) was heated at reflux for 20 h. The solvent was evaporated and the residue extracted with water/DCM. The org. layer was washed with brine, dried over MgSO₄ and concentrated under reduced pressure. The residue was triturated with ether/EA to afford the title compound as an off-white solid (6.91 g, 70% yield).
¹H NMR (CDCl₃) δ: 7.07 (d, J = 2.6 Hz, 1 H), 6.98 (dd, J = 9.1, 2.6 Hz, 1 H), 6.85 (d, J = 8.9 Hz, 1 H), 4.68 (m, 1 H), 4.24 (s, 4 H), 4.10 (t, J = 9.1 Hz, 1 H), 3.72 (dd, J = 9.1, 5.9 Hz, 1 H), 3.46 (m, 1 H), 3.33 (m, 1 H).
MS (ESI, m/z): 362.2 [M+H]⁺.

### Intermediate 4: 6-[(R)-5-(2-iodo-ethyl)-2-oxo-oxazolidin-3-yl]-4H-benzo[1,4]thiazin-3-one:

### 4.i. Tert-butyl-dimethyl-((R)-2-oxiranyl-ethoxy)-silane and (2S)-4-(tett-butyl-dimethyl-silanyloxy)-butane-1,2-diol:

The title intermediates were prepared in analogy to Kishi et al., Org. Lett. (2005), 7, 3997, (intermediate S2-3) via hydrolytic kinetic resolution of (*RS*)-*tert*-butyl-dimethyl-(2-oxiranyl-ethoxy)-silane (prepared according to J. Org. Chew. (2008), 73, 1093). Two compounds were isolated after CC (Hept/EA 2:1).
First eluting compound: tert-butyl-dimethyl-((R)-2-oxiranyl-ethoxy)-silane (colourless oil, 25.3 g, 48% yield).
¹H NMR (CDCl₃) δ: 3.77 (t, J = 6.4 Hz, 2H), 3.04 (m, 1H), 2.78 (m, 1H), 2.51 (dd, J = 5.0, 2.9 Hz, 1H), 1.74 (m, 2H), 0.90 (d, J = 0.6 Hz, 9H), 0.06 (s, 6H).
Second eluting compound: (2S)-4-(*tert*-butyl-dimethyl-silanyloxy)-butane-1,2-diol (colourless oil, 24.9 g, 43% yield).
¹H NMR (CDCl₃) δ: 3.89 (m, 3 H), 3.62 (s, 1H), 3.53 (m, 1H), 3.42 (br. s, 1H), 2.29 (m, 1H), 1.70 (m, 2H), 0.90 (s, 9H), 0.09 (s, 6H).

### 4.ii. 6-[(R)-4-(tert-butyl-dimethyl-silanyloxy)-2-hydroxy-butylamino]-4H-benzo[1,4]thiazin-3-one:

A solution of 6-amino-4*H*-benzo[1,4]thiazin-3-one (10.68 g, 59.3 mmol; commercial) and tert-butyl-dimethyl-((R)-2-oxiranyl-ethoxy)-silane (first eluting compound in step 4.i., 12.0 g, 59.3 mmol) in 9-1 EtOH/H₂O (320 mL) was heated at 80°C for 2 days. The mixture was concentrated under reduced pressure. Residual starting aniline could be removed by addition of Et₂O/MeOH followed by filtration. The filtrate containing the product was concentrated under reduced pressure to afford the title compound as a brown oil (18.8 g, 83% yield) which was used as such in the next step.
MS (ESI, m/z): 383.2 [M+H]⁺.

### 4.iii. 6-{(R)-5-[2-(tert-butyl-dimethyl-silanyloxy)-ethyl]-2-oxo-oxazolidin-3-yl}-4H-benzo[1,4]thiazin-3-one:

A solution of intermediate 4.ii (23.5 g, 49.1 mmol) and CDI (9.57 g, 1.2 eq.) in THF (250 mL) was heated at 50°C overnight. The mixture was concentrated under reduced pressure and partitioned between EA and water. The aq. layer was extracted once more with EA and the combined org. layers were dried over MgSO₄ and concentrated. The residue was purified by CC (DCM/MeOH/NH₄O 1000:50:4) to afford the title compound as a colourless solid (8.4 g, 42% yield).
MS (ESI, m/z): 409.3 [M+H]⁺.

### 4.iv. 6-[(R)-5-(2-hydroxy-ethyl)-2-oxo-oxazodidin-3-yl]-4H-benzo[1,4]thiazin-3-one:

A solution of intermediate 4.iii (8.4 g, 20.6 mmol) in THF (50 mL) was treated with TBAF (1M solution in THF, 24.7 mL, 1.2 eq.) at 0°C. The solution was stirred at 0°C for 6 h. The mixture was partitioned between water and EA and the aq. phase was extracted with EA (3x). The combined org. layers were washed with water and brine, dried over MgSO₄ and concentrated. The residue was triturated with Et₂O/E to afford the title compound as an off-white solid (4.79 g, 79% yield).
MS (ESI, m/z): 295.5 [M+H]⁺.

### 4.v. Methanesulfonic acid 2-[(R)-2-oxo-3-(3-oxo-3,4-dihydro-2H-benzo[1,4]thiazin-6-yl)-oxazolidin-5-yl-ethyl ester:

A solution of intermediate 4.iv (4.7 g, 16.0 mmol) and DIPEA (7.54 mL, 2.9 eq.) in anhydrous DCM (80 mL) was cooled to 0°C and treated dropwise with MsCl (1.50 mL, 1.2 eq.). The resulting mixture was stirred at 0°C for 1 h. Water and DCM were added and the phases separated. The org. layer was dried over MgSO₄ and concentrated under reduced pressure. The residue was purified by CC (DCM/MeOH/NH₄OH 1000:50:4) to afford the title compound as an off-white solid (5.80 g, 98% yield).
MS (ESI, m/z): 373.4 [M+H]⁺.

### 4.vi. 6-[(R)-5-(2-iodo-ethyl)-2-oxo-oxazolidin-3-yl]-4H-benzo[1,4]thiazin-3-one:

A suspension of intermediate 4.v (3.5 g, 9.4 mmol) and Nal (4.23 g, 3 eq.) in 2-butanone (35 mL) was heated at 85°C overnight. After cooling, the mixture was diluted with ether/EA (20 mL) and treated with 10% aq. Na₂S₂O₃ (60 mL). After stirring for 10 min the phases were separated and the aq. layer was washed with EA. The combined org. layers were washed with water (2x), dried over MgSO₄ and concentrated under reduced pressure. The residue was triturated with Et₂O/EA to afford the title compound as an off-white solid (3.52 g, 93% yield).
¹H NMR (DMSO-d6) δ: 10.55 (s, 1H), 7.30 (m, 2H), 7.04 (dd, J = 8.5, 2.3 Hz, 1H), 4.68 (m, 1H), 4.10 (t, J = 8.8 Hz, 1H), 3.70 (dd, J = 8.8, 6.7 Hz, 1H), 3.41 (s, 2H), 3.29 (m, 2H), 2.23 (m, 2H).
MS (ESI, m/z): 405.0 [M+H]⁺.

### Intermediate 5: 6-[(rac)-(3-amino-propyl)-2-oxo-oxazolidin-3-yl]-4H-benzo[1,4]thiazin-3-one:

### 5.i. [(rac)-4-hydroxy-5-(3-oxo-3,4-dihydro-2H-benzo[1,4]thiazin-6-ylamino)-pentyl]-carbamic acid tert-butyl ester:

A solution of ((*rac*)-3-oxiranyl-propyl)-carbamic acid *tert*-butyl ester (1.08 g, 5.34 mmol, commercial) and 6-amino-4*H*-benzo[1,4]thiazin-3-one (1.01 g, 5.61 mmol) in 9:1 EtOH/H₂O (20 mL) was heated at 70°C overnight. After cooling the mixture was concentrated *in vacuo* and purified by CC (DCM-MeOH-NH₄O 1000:50:4) to afford the title compound as a beige foam (767 mg, 38% yield).
MS (ESI, m/z): 382.1 [M+H]⁺.

### 5.ii. {3-[(rac)-2-oxo-3-(3-oxo-3,4-dihydro-2H-benzo[1,4]thiazin-6-yl)-oxazolidin-5-yl]-propyl}-carbamic acid tert-butyl ester:

A solution of intermediate 5.i (519 mg, 1.36 mmol) and CDI (455 mg, 2 eq.) in anhydrous THF (5.5 mL) was heated at 50°C for 3 h. After cooling the mixture was concentrated *in vacuo* and partitioned between EA and water. The org. layer was washed with brine, dried over MgS0₄ and concentrated *in vacuo.* The residue was purified by CC (DCM-MeOH-NH₄0H: 1000:50:4) to afford the title compound as an off-white foam (412 mg, 74% yield).
MS (ESI, m/z): 408.5 [M+H]⁺.

### 5.iii). 6-[(rac)-5-(3-amino-propyl)-2-oxo-oxazolidin-3-yl]-4H-benzo[1,4]thiazin-3-one:

Starting from intermediate *5.ii* (402 mg, 0.987 mmol) and using procedure D, the title compound was obtained as a yellow solid (290 mg, 96% yield).
MS (ESI, m/z): 308.2 [M+H]⁺.

### Intermediate 6: 6-[(R)-5-(3-amino-propyl)-2-oxo-oxazolidin-3-yl]-4H-benzo[1,4]oxazin-3-one:

### 6.i. 6-[(R)--(tert-butyl-dimethyl-silanyloxy)-2-hydroxy-penylamino]-4H-benzo[1,4]oxazin-3-one:

A mixture of (*R*)-*tert*-butyl-dimethyl-(3-oxiranyl-propoxy)-silane (13 g, 60 mmol, prepared according to Organic Letters (2005), 7, 3997) and 6-amino-4*H*-benzo[1,4]oxazin-3-one (9.9 g) in EtOH/H₂O (9:1, 325 mL) was heated at reflux overnight. The volatiles were removed under reduced pressure and the residue purified by CC (Hept/EA 1:1) to give the desired compound as a brown oil (8.9 g , 39% yield).
MS (ESI, m/z): 381.2 [M+H]⁺.

### 6.ii. 6-{(R)-5-[3-(tert-butyl-dimethyll-silanyloxy)-propyl]-2-oxo-oxazolidin-3-yl}-4H-benzo[1,4]oxazin-3-one:

A solution of intermediate 6.i (8.8 g, 23 mmol) in THF (500 mL) was treated with CDI (5.6 g, 1.5 eq.) and heated at 50°C overnight. The mixture was cooled to rt, diluted with EA and washed with water and brine, dried over MgSO₄ and concentrated. The product was crystallised from Hept/EA to give the desired oxazolidinone as an orange solid (9.8 g, quant.).
MS (ESI, m/z): 407.6 [M+H]⁺.

### 6.iii. 6-[(R)-5-(3-hydroxy-propyl)-2-oxo-oxazolidin-3-yl]-4H-benzo[1,4]thiazin-3-one:

A suspension of intermediate 6.ii (9.8 g, 24 mmol) in THF (95 mL) was treated with a 1*M* TBAF solution in THF (1 eq.). The brown solution was stirred at rt for 2 h, diluted with EA, washed with water and brine, dried over MgSO₄ and concentrated. The residue was purified by CC (EA, EA/MeOH 9:1) followed by crystallisation with ether/EA to give the desired alcohol as a yellowish solid (5.0 g, 71 % yield).
MS (ESI, m/z): 293.3 [M+H]⁺.

### 6.iv. Methanesulfonic acid 3-[(R)-2-oxo-3-(3-oxo-3,4-dihydro-2H-benzo[1,4]oxazin-6-yl)-oxazolidin-5-yl]-propyl ester:

A suspension of intermediate 6.iii (2.0 g, 6.8 mmol) in DCM (40 mL) was treated with TEA (1.9 mL, 2 eq.) and drop wise with MsCl (0.940 g, 1.2 eq.). The mixture was stirred at rt for 2.5 h, diluted with DCM, washed with water, dried over MgSO₄ and concentrated to give the desired mesylate as a beige solid (2.5 g, 98% yield).
MS (ESI, m/z): 371.3 [M+H]⁺.

### 6.v. 6-[(R)-5-(3-azido-propyl)-2-oxo-oxazolidin-3-yl]-4H-benzo[1,4]oxazin-3-one:

A solution of intermediate 6.iv (2.3 g, 5.9 mmol) in DMF (12 mL) was treated with NaN₃ (464 mg, 1.2 eq.) and the mixture was stirred at 80°C overnight. The mixture was poured on water and extracted with EA. The org. layer was dried over MgSO₄ and concentrated. The residue was triturated with Hept to give the desired azide as a beige solid (1.7 g, 90 % yield).
MS (ESI, m/z): 318.0 [M+H]⁺.

### 6.vi. 6-[(R)-5-(3-amino-propyl)-2-oxo-oxazolidin-3-yl]-4H-benzo[1,4]oxazin-3-one:

A solution of intermediate 6.v (1.6 g, 5 mmol) in THF/MeOH (1:1, 15 mL) was hydrogenated over 10% Pd/C (535 mg) at 1 bar of H₂ for 2 h. The catalyst was filtered off and the filtrate concentrated *in vacuo* to give the desired compound as a colourless foam (1.4 mg, 95 % yield).
MS (ESI, m/z): 292.3 [M+H]⁺.

### Intermediate 7: 6-((S)-5-aminomethyl-2-oxo-oxazolidin-3-yl)-4H-benzo[1,4]thiazin-3-one:

### 7.i. (S)-3-chloro-2-hydroxy-propyl)-carbamic acid tert-butyl ester:

This compound (25.6 g, 45% yield) was prepared according to the literature (Org. Process Research and Development (2003), 7, 533-546) starting from *(S)*-epichlorohydrin (25 g, 270 mmol).
¹H NMR (CDCl₃) δ: 4.95 (br, 1H), 4.00-3.80 (m, 1H), 3.60-3.50 (m, 2H), 3.50-3.35 (m, 2H), 3.30-3.20 (m, 1H), 1.42 (s, 9H).

### 7.ii. [(S)-2-oxo-3-(3-oxo-3,4-dihydro-2H-benzo[1,4]thiazin-6-yl)-oxazolidin-5-ylmethyl]-carbamic acid tert-butyl ester:

A solution of intermediate 7.i (1.3 g, 6.3 mmol) and (3-oxo-3,4-dihydro-2*H*-benzo[1,4]thiazin-6-yl)-carbamic acid benzyl ester (2.0 g, 1 eq.) in DMF (20 mL) at 0°C was treated dropwise with LiOtBu (2.2*M* in THF, 8.7 mL, 3 eq.). The mixture was stirred at rt overnight. 1*M* HCl (12.6 mL) was added and the mixture partitioned between EA and water. The org. phase was washed with water and brine, dried over MgSO₄ and concentrated. The residue was purified by CC (Hept/EA 1:2, EA) to give the desired intermediate as a beige solid (1 g, 41% yield).
MS (ESI, m/z): 380.1 [M+H]⁺.

### 7.iii. 6-((S)-5-aminomethyl-2-oxo-oxazolidin-3-yl)-4H-benzo[1,4]thiazin-3-one:

Starting from intermediate 7.ii (1 g) and using procedure D, the title intermediate was obtained after purification by CC (DCM/MeOH 9:1 containing 1% NH₄OH) as a beige solid (280 mg, 76% yield).
MS (ESI, m/z): 280.2 [M+H]⁺.

### Intermediate 8: 6-[(R)-5-(2-amino-ethyl)-2-oxo-oxazolidin-3-yl]-4H-benzo[1,4]thiazin-3-one:

### 8.i. 6-[(R)-5-(2-azido-ethyl)-2-oxo-oxazodidin-3-yl]-4H benzo[1,4]thiazin-3-one:

A solution of intermediate 4.v (2.5 g, 6.7 mmol) in DMF (12 mL) was treated with NaN₃ (523 mg, 1.2 eq.) and the mixture was stirred at 80°C overnight. The mixture was poured on water and extracted with EA. The org. layer was dried over MgSO₄ and concentrated. The residue was triturated with ether/MeOH to give the desired azide as a beige solid (1.9 g, 89 % yield).
MS (ESI, m/z): 320.2 [M+H]⁺.

### 8.ii. 6-[(R)-5-(2-amino-ethyl)-2-oxo-oxazolidin-3-yl]-4H-benzo[1,4]thiazin-3-one:

A solution of intermediate 8.i (1.8 g, 5.6 mmol) in THF/MeOH (2:1, 70 mL) was hydrogenated over 10% Pd/C (597 mg) at 1 bar of H₂ for 2 h. The catalyst was filtered off and the filtrate concentrated *in vacuo* and triturated with ether to give the desired intermediate as a white solid (1.4 g, 85 % yield).
MS (ESI, m/z): 294.4 [M+H]⁺.

### Intermediate 9: 7-methoxy-2-(piperidin-4-ylmethoxy)-quinoline: 9.i. 4-(7-methoxy-quinolin-2-yloxymethyl)-piperidine-1-carboxylic acid tert-butyl ester:

A suspension of 7-methoxy-2(1*H*)-quinolinone (350 mg; commercial) in DMF (20 mL) was treated at rt with NaH (50% dispersion in oil; 96 mg). After stirring for 30 min, the suspension was treated with (1-(tert-butoxycarbonyl)-piperidin-4-yl)methyl methanesulfonate (645 mg; commercial) and heated at 100°C overnight. The reaction mixture was partitioned between EA and water. The org. phase was dried over MgSO₄, concentrated under reduced pressure and purified by CC (Hept/EA 2:1, to EA), affording 4-(7-methoxy-quinolin-2-yloxymethyl)-piperidine-1-carboxylic acid *tert*-butyl ester (O-alkylated compound, less polar product) as a colourless oil (448 mg, 60% yield).
¹H NMR (CDCl₃) δ: 7.89 (d, J = 8.8 Hz, 1H), 7.58 (d, J = 8.8 Hz, 1H), 7.20 (d, J = 2.3 Hz, 1H), 7.01 (dd, J = 8.8, 2.6 Hz, 1H), 6.74 (d, J = 8.5 Hz, 1H), 4.32 (d, J = 6.4 Hz, 2H), 4.15 (m, 2H), 3.93 (s, 3H), 2.75 (m, 2H), 2.05 (m, 1H), 1.83 (m, 2H), 1.47 (m, 9H).

### 9.ii. 7-methoxy-2-(piperidin-4-ylmethoxy)-quinoline:

Starting from intermediate 9.i and using procedure D, the title compound (455 mg; 100% yield) was obtained.
¹H NMR (CDCl₃) δ: 7.89 (d, J = 8.5 Hz, 1H), 7.58 (d, J = 8.8 Hz, 1H), 7.20 (d, J = 2.3 Hz, 1H), 7.01 (dd, J = 8.8, 2.3 Hz, 1H), 6.74 (d, J = 8.8 Hz, 1H), 4.30 (d, J = 6.4 Hz, 2H), 3.93 (s, 3H), 3.15 (m, 2H), 2.68 (m, 2H), 1.88 (m, 2H), 1.32 (m, 2H).

### Intermediate 10: 2-(azetidin-3-ylmethoxy)-7-methoxy-quinoxaline:

### 10.i. 3-(7-methoxy-quinoxalin-2-yloxymethyl)-azetidine-1-carboxylic acid tert-butyl ester:

The title compound was prepared in analogy to the protocol of intermediate 9, step 9.i, starting from 7-methoxy-quinoxalin-2(1*H*)-one (1.00 g; prepared according to WO 2008/009700) and 3-methanesulfonyloxymethyl-azetidine-1-carboxylic acid *tert*-butyl ester (1.65 g; prepared according to WO 2008/033764). 3-(7-methoxy-quinoxalin-2-yloxymethyl)-azetidine-1-carboxylic acid *tert*-butyl ester (*O*-alkylation, less polar product) was obtained as a yellow oil (1.07 g; 55% yield).
¹H NMR (CDCl₃;) δ: 8.31 (s, 1H), 7.89 (d, J = 9.1 Hz, 1H), 7.21 (m, 2H), 4.61 (d, J = 6.7 Hz, 2H), 4.12 (t, J = 8.8 Hz, 2H), 3.84 (dd, J = 8.8, 5.3 Hz, 2H), 3.06 (m, 1H), 1.45 (m, 9H).
MS (ESI, m/z): 228.3 [M+H]⁺.

### 10.ii. 2-(azetidin-3-ylmethoxy)-7-methoxy-quinoxaline:

Starting from intermediate 10.i (1.00 g) and using procedure D, the title compound was obtained as a beige solid (506 mg; 71 % yield).
1H NMR (CDCl₃) δ: 8.32 (s, 1H), 7.88 (d, J = 9.1 Hz, 1H), 7.18 (m, 2H), 4.63 (d, J = 6.4 Hz, 2H), 3.94 (s, 3H), 3.82 (t, J = 8.2 Hz, 2H), 3.63 (m, 2H), 3.26 (m, 1H).
MS (ESI, m/z): 246.4 [M+H]⁺.

### Intermediate 11: cis- and trans-3-(7-methoxy-quinolin-2-yloxy)-cyclobutylamine:

### 1.i. cis- and trans-[3-(7-methoxy-quinolin-2-yloxy)-cyclobutyl]-carbamic acid tert-butyl ester:

A mixture of 7-methoxy-1*H*-quinolin-2-one (495 mg, 2.83 mmol, commercial), methanesulfonic acid 3-*tert*-butoxycarbonylamino-cyclobutyl ester (900 mg, 1.2 eq., 1:1 mixture of *cis-* and *trans*-isomers, prepared according to WO 2005/116009) and Cs₂CO₃ (1.38 g, 1.5 eq.) in DMF (6 mL) was heated at 80°C for 5 days. The mixture was partitioned between EA and water and the phases were separated. The aq. layer was extracted with EA. The combined org. layers were washed with water (3x) and brine, dried over MgSO₄ and concentrated under reduced pressure. The residue was purified by CC (Hept-EA 1-1) to afford the title intermediate as a yellow oil (321 mg, 33% yield).
MS (ESI, m/z): 345.2 [M+H]⁺.

### 11.ii. cis- and trans-3-(7-methoxy-quinolin-yloxy)-cyclobutylamine:

Starting from intermediate 11.i and using procedure D, the title intermediate was obtained as a colourless solid (160 mg, 70% yield).
MS (ESI, m/z): 245.4 [M+H]⁺.

### Intermediate 12: 7-methoxy-2-((R)-1-pyrrolidin-3-ylmethoxy)-quinoline:

### 12.i. (R)-3-(7-methoxy-quinolin-2-yloxymethyl)-pyrrolidine-1-carboxylic acid tert-butyl easter:

The title compound was prepared in analogy to the protocol of intermediate 9, step 9.i, starting from 7-methoxy-2(1*H*)-quinolinone (500 mg; commercial) and (*R*)-3-methanesulfonyloxymethyl-pyrrolidine-1-carboxylic acid *tert*-butyl ester (797 mg; prepared according to WO 2005/116009).
(*R*)-3-(7-methoxy-quinolin-2-yloxymethyl)-pyrrolidine-1-carboxylic acid *tert*-butyl ester (O-alkylation, less polar product): 550 mg of a colourless oil (54% yield).
¹H NMR (CDCl₃) δ: 7.89 (d, J = 8.5 Hz, 1H), 7.58 (d, J = 8.8 Hz, 1H), 7.19 (d, J = 2.3 Hz, 1H), 7.01 (dd, J = 8.8, 2.6 Hz, 1H), 6.74 (d, J = 8.8 Hz, 1H), 4.39 (m, 2H), 3.93 (s, 3H), 3.50 (m, 5 H), 2.95 (m, 3 H), 2.66 (m, 1 H), 2.03 (m, 2 H), 11.40 (s, 9H).

### 12.ii. 7-methoxy-2-((R)-1-pyrrodidin-3-ylmethoxy)-quinoline:

Starting from intermediate 12.i (40 mg) and using procedure D, the title compound, was obtained as a colourless oil (26 mg; 91 % yield).
¹H NMR (CDCl₃) δ: 7.89 (d, J = 8.5 Hz, 1H), 7.58 (d, J = 8.8 Hz, 1H), 7.19 (d, J = 2.3 Hz, 1H), 7.01 (dd, J = 8.8, 2.6 Hz, 1H), 6.74 (d, J = 8.8 Hz, 1H), 4.39 (m, 2H), 3.93 (s, 3H), 3.62 (m, 1 H), 3.17 (m, 1H), 2.95 (m, 3H), 2.66 (m, 1H), 2.03 (m, 2H).

### Intermediate 13: 7-fluoro-quinoline-2-carbaldehyde:

A solution of 7-fluoro-2-methyl-quinoline (1.10 g; commercial) in dioxane (8 mL) was treated with SeO₂ (0.79 g) and stirred at 80°C for 4 h. The reaction mixture was filtered and concentrated *in vacuo.* The crude product was purified by CC (Hex/EA 4:1, 2:1) affording, after stirring of the crystals in MeOH, a yellow solid (610 mg; 51 % yield).
¹H NMR (CDCl₃) δ: 10.15 (s, 1H), 8.25 (d, J = 8.5 Hz, 1H), 7.94 (d, J = 8.4 Hz, 1H), 7.83 (m, 2H), 7.42 (m, 1H).

### Intermediate 14: 5-fluoro-quinoline-2-carbaldehyde:

The title aldehyde was prepared in analogy to intermediate 13, starting from 5-fluoro-2-methyl-quinoline (519 mg). The product was purified by CC (Hex/EA 4:1, 2:1), affording a yellow solid (146 mg; 26% yield).
¹H NMR (CDCl₃) δ: 10.15 (s, 1H), 8.53 (d, J = 8.5 Hz, 1H), 8.01 (m, 2H), 7.71 (m, 1H), 7.30 (m, 1H).

### Intermediate 15: 6-fluoro-quinoline-5-carbaldehyde and 6-fluoro-quinoline-7-carbaldehyde:

To a solution of DIPA (1.1 mL) in THF (20 mL) cooled to -78°C, was added *n*-BuLi (2.5*N* in Hex, 3 mL). The mixture was stirred 5 min at this temperature and was warmed in an ice-bath. After 10 min, the mixture was cooled down to -78°C and treated with a solution of 6-fluoroquinoline (0.95 g; commercial) in THF (10 mL). After 4 h, DMF (0.75 mL) was added and the mixture was further stirred for 30 min. The mixture was warmed to rt, further stirred for 30 min and treated with water (20 mL). The two layers were decanted. The aq. layer was extracted with EA (2 x 50 mL). The combined org. layers were washed with brine, dried over Na₂SO₄, filtered and concentrated to dryness. The residue was purified by CC (Hept-EA 1-1), affording the expected aldehydes in a 2:1 mixture favouring the isomer with the aldehyde at C-5 as a yellowish solid (0.17 g; 15% yield).
¹H NMR (CDCl₃) δ: 10.76 (s, 2/3 H, aldehyde at C-5); 10.47 (s, 1/3 H, aldehyde at C-6); 9.59 (m); 8.97 (m); 8.65 (d, J = 6.7 Hz); 8.37 (dd, J = 9.4, 5.6 Hz); 8.15 (m); 7.52 (m).

### Intermediate 16: 3-quinolin-2-yl-propionaldehyde:

A solution of 2-(3-hydroxypropyl)quinoline (850 mg; commercial) and DIPEA (2.33 mL) in DCM (15 mL) was treated dropwise at rt with a solution of pyridine.S0₃ complex (1.44 g) in DMSO (5 mL). The mixture was further stirred at rt for 1.5 h, then quenched with water. The org. layer was extracted with water (3 times), dried over MgSO₄ and purified by CC (Hex/EA 1: 1 to EA), affording a brown oil (470 mg, 56% yield).
MS (ESI, m/z): 345.2 [M+H]⁺.

### Intermediate 17: 6-[(R)-5-(3-amino-propyl)-2-oxo-oxazolidin-3-yl]-4H-benzo[1,4]thiazin-3-one:

### 17.i. 6-[(R)-5-(tert-butyl-dimethyl-silanyloxy)-2-hydroxy-pentylamino]-4H-benzo[1,4]thiazin-3-one:

A mixture of (R)-tert-butyl-dimethyl-(3-oxiranyl-propoxy)-silane (13 g, 60 mmol, prepared according to Organic Letters (2005), 7, 3997) and 6-amino-4*H*-benzo[1,4]thiazin-3-one (10.8 g) in EtOH/H₂O (9:1, 325 mL) was heated at reflux overnight. The volatiles were removed under reduced pressure and the residue purified by CC (Hept/EA 1:1) to give the desired intermediate as a brown oil (6.8 g, 28% yield).
MS (ESI, m/z): 397.1 [M+H⁺].

### 17.ii. 6-{(R)-5-[3-(tert-butyl-dimethyl-silanyloxy)-propyl]-2-oxo-oxazolidin-3-yl}-4H-benzo[1,4]thiazin-3-one:

A solution of intermediate 17.i (6.7 g, 17 mmol) in THF (370 mL) was treated with CDI (4.1 g, 1.5 eq) and heated at 50°C for 7 h. The mixture was cooled to rt, diluted with EA and washed with water and brine, dried over MgSO₄ and concentrated. The product was crystallized from Hept/EA to give the desired oxazolidinone as an orange solid (7.8 g, quant).
MS (ESI, m/z): 423.4 [M+H⁺].

### 17.iii. 6-[(R)-5-(3-hydnoxy-propyl)-2-oxo-oxazolidin-3-yl]-4H-benzo[1,4]thiazin-3-one:

A suspension of intermediate 17.ii (7.1 g, 16.8 mmol) in THF (100 mL) was treated with a 1M TBAF solution in THF (1 ed). The brown solution was stirred at rt for 4 h, diluted with EA and washed with water and brine, dried over MgSO₄ and concentrated. The residue was purified by CC (EA, EA/MeOH 9:1) followed by crystallization to give the desired alcohol as a yellowish solid (3.1 g, 60% yield).
MS (ESI, m/z): 309.1 [M+H⁺].

### 17.iv. Methanesulfonic acid 3-[(R)-2-oxo-3-(3-oxo-3,4-dihydro-2H-benzo[1,4]thiazin-6-yl)-oxazolidin-5-yl]-propyl ester:

A suspension of intermediate 17.iii) (0.42 g, 1.36 mmol) in DCM was treated with NEt₃ (0.265 mL, 1.4 eq.) and dropwise with MsCl (0.127 g, 1.2 eq.). The mixture was stirred at rt for 2.5 h, diluted with DCM, washed with water, dried over MgSO₄ and concentrated. The residue was crystallized from ether/EA to give the desired mesylate as a beige solid (0.4 g, 76% yield).
MS (ESI, m/z): 387.2 [M+H⁺].

### 17. v. 6-[(R)-5-(3-azido-propyl)-2-oxo-oxazolidin-3-yl]-4H-benzo[1,4]thiazin-3-one:

A solution of intermediate 17.iv) (1.26 g, 3.26 mmol) in DMF (10 mL) was treated with NaN₃ (254 mg, 1.2 eq.) and the mixture was stirred at 80°C for 3 h. The mixture was poured on water and extracted with EA. The org. layer was dried over MgSO₄ and concentrated. The residue was triturated with ether to give the desired azide as a rose solid (691 mg, 64% yield).
MS (ESI, m/z): 334.3 [M+H⁺].

### l7.vi. 6-[(R)-5-(3-amino-propyl)-2-oxo-oxazolidin-3-yl]-4H-benzo[1,4]thiazin-3-one:

A solution of intermediate 17.v (690 mg, 2 mmol) in THF/MeOH (1:1, 15 mL) was hydrogenated over 10% Pd/C (220 mg) at 1 bar of H₂ for 2 h. The catalyst was filtered off and the filtrated concentrated *in vacuo* to give the desired intermediate as an off-white solid (590 mg, 93% yield).
MS (ESI, m/z): 308.2 [M+H⁺].

### Intermediate 18: 6-[(R)-5-(2-iodo-ethyl)-2-oxo-oxazolidin-3-yl]-4H-benzo[1,4]oxazin-3-one:

### 18.i. (2R)-tert-butyl-dimethyl-(2-oxiranyl-ethoxy)-silane and (-S)-4-(tert-butyl-dimethyl-silanyloxy)-butane-12-diol:

The title intermediates were prepared in analogy to Kishi et al, Org. Lett. (2005), 7, 3997, (intermediate S2-3) *via* hydrolytic kinetic resolution of (*RS*)-*tert*-butyl-dimethyl-(2-oxiranyl-ethoxy)-silane (prepared according to J. Oi-g. Chem. (2008), 73, 1093). Two compounds were isolated after CC (Hept/EA 2:1).
First eluting compound: (2R)-tert-butyl-dimethyl-(2-oxiranyl-ethoxy)-silane (colourless oil, 25.3 g, 48% yield).
¹H NMR (CDl13) δ: 3.77 (t, J = 6.4 Hz, 2H), 3.04 (m, 1H), 2.78 (m, 1H), 2.51 (dd, J = 5.0, 2.9 Hz, 1H), 1.74 (m, 2H), 0.90 (d, J = 0.6 Hz, 9H), 0.06 (s, 6H).
Second eluting compound: (*2S*)-4-(*tert*-butyl-dimethyl-silanyloxy)-butane-1,2-diol (colourless oil, 24.9 g, 43% yield).
¹H NMR (CDl₃) δ: 3.89 (m, 3H), 3.62 (s, 1H), 3.53 (m, 1H), 3.42 (br. s, 1H), 2.29 (m, 1H), 1.70 (m, 2H), 0.90 (s, 9H), 0.09 (s, 6H).

### 18.ii. 6-[(R)-4-(tert-butyl-dimethyl-silanyloxy)-2-hydroxy-butylamio]-4H-benzo[1,4]oxzin-3-one:

A solution of 6-amino-4*H*-benzo[14]oxazin-3-one (commercial; 6.49 g, 39.5 mmol) and (2*R*)-*tert*-butyl-dimethyl-(2-oxiranyl-ethoxy)-silane (first eluting compound in step 18.i, 8.0 g, 39.5 mmol) in 9-1 EtOH/H₂O (240 mL) was heated at 80°C for 2 days. The mixture was concentrated under reduced pressure. Residual starting aniline could be removed by addition of Et₂O/MeOH followed by filtration. The filtrate containing the product was concentrated under reduced pressure and the residue was purified by CC (DCM/MeOH/NH₄OH 1000:50:4) to afford the title intermediate as a brown oil (5.82 g, 40% yield).
MS (ESI, m/z): 367.3 [M+H⁺].

### 18. iii. 6-{(R)-5-[2-(tert-butyl-dimethyl-silanyloxy)-ethyl]-2-oxo-oxazolidin-3-lyl}-4H-benzo[1,4]oxazin-3-one:

A solution of intermediate 18.ii (5.8 g, 15.8 mmol) and CDI (3.07 g, 1.2 eq.) in THF (50 mL) was heated at 50°C overnight. The mixture was concentrated under reduced pressure and partitioned between EA and water. The aq. layer was extracted once more with EA and the combined org. layers were dried over MgSO₄ and concentrated. The residue was triturated with Et₂O/EA/Me to afford the title intermediate as a beige solid (2.7 g, 43% yield).
MS (ESI, m/z): 393.5 [M+H⁺].

### 18.iv. 6-[(R)-5-(-2hydroxy-ethyl)-2-oxo-oxazolidin-3-yl]-4H-benzo[1,4]oxazin-3-one:

A solution of intermediate 18.iii (2.70 g, 6.88 mmol) in THF (15 mL) was treated with TBAF (1M solution in THF, 8.3 mL, 1.2 eq.) at 0°C. The solution was stirred at 0°C for 2 h. The mixture was partitioned between water and EA and the aq. phase was extracted with EA (3x). The combined org. layers were washed with water and brine, dried over MgSO₄ and concentrated. The residue was triturated with Et₂O/MeOH to afford the title intermediate as an off-white solid (1.25 g, 65% yield).
MS (ESI, m/z): 279.5 [M+H⁺].

### 18.v. Methanesulfonic acid 2-[(R)-2-oxo-3-(3-oxo-3,4-dihydro-2H-benzo[1,4]oxazin-6-yl)-oxazodidin-S-ylJ-ethy_{J} ester:

A solution of intermediate 18.iv (2.1 g, 7.55 mmol) and DIPEA (3.57 mL, 2.9 eq.) in anhydrous DCM (40 mL) was cooled to 0°C and treated dropwise with MsCl (0.71 mL, 1.2 eq.). The resulting mixture was stirred at 0°C for 1 h. Water and DCM were added and the phases separated. The org. layer was dried over MgSO₄ and concentrated under reduced pressure. The residue was triturated with MeOH to afford the title intermediate as an off-white solid (1.16 g, 43% yield).
¹H NMR (DMSO-d6) δ: 10.72 (s, 1 H), 7.30 (d, J = 2.1 Hz, 1H), 6.93 (m, 2H), 4.76 (m, 1H), 4.52 (s, 2H), 4.34 (m, 2H), 4.11 (t, J = 8.8 Hz, 1H), 3.72 (m, 1H), 3.20 (s, 3H), 2.17 (m, 2H).
MS (ESI, m/z): 357.2 [M+H⁺].

### 18.vi. 6-[(R)-5-(2-iodo-ethyl)-2-oxo-oxazolidin-3-yl]-4H-benzo[1,4]oxazin-3-one:

A suspension of intermediate 18.v (1.16 g, 3.26 mmol) and NaI (1.46 g, 3 eq.) in 2-butanone (10 mL) was heated at 85°C overnight. After cooling, the mixture was diluted with ether/EA (10 mL) and treated with 10% aq. Na₂S₂O₃ (30 mL). After stirring for 10 min, the phases were separated and the aq. layer was washed with EA. The combined org. layers were washed with water (2x), dried over MgSO₄ and concentrated under reduced pressure to afford the title intermediate as an off-white solid (0.91 g, 72% yield).
¹H NMR (CDCl₃) δ: 8.24 (s, 1H), 7.42 (d, J = 2.3 Hz, 1H), 6.95 (d, J = 8.8 Hz, 1H), 6.79 (dd, J = 8.8, 2.6 Hz, 1H), 4.80 (m, 1H), 4.59 (s, 2H), 4.14 (t, J = 8.8 Hz, 1 H), 3.65 (dd, J = 8.8, 6.7 Hz, 1H), 3.33 (m, 2H), 2.30 (m, 2H).
MS (ESI, m/z): 389.0 [M+H⁺].

### Intermediate 19: 7-fluoro-2-methoxy-quinoxaline-6-carbaldehyde:

### 19.i. 2-chloro-6-fluoro-3-methoxy-quinoxaline-5-carbaldehyde and 3-chloro-7-fluoro-2-methoxy-quinoxaline-6-carbaldehyde:

A solution of 2,2,6,6-tetramethylpiperidine (5.2 mL) in THF (dry; 90 mL) was treated at -78°C with a solution of nBuLi (2.5*M* in Hex; 12.2 mL). After stirring at this temperature for 30 min, the reaction mixture was added via a cannula to a pre-cooled (at -78°C) solution of 2-chloro-6-fluoro-3-methoxy-quinoxaline (5.0 g; prepared according to EP 107455) in THF (120 mL). After stirring at this temperature for a further 30 min the reaction mixture was treated with DMF (3.62 mL). After stirring at this temperature for a further 20 min the reaction mixture was poured into a mixture of ice and NH₄Cl and extracted with EA. The org. layer was washed with brine, dried over MgSO₄, evaporated under reduced pressure and the residue was crystallized from Hex/EA affording a yellow solid (3.7 g; 65% yield) of a 7:1 mixture of the two isomers which was used in the next step without further characterisation.

### 19.ii. 7-fluoro-2-methoxy-quinoxaline-6-carbaldehyde:

A solution of intermediate 19.i. (3.70 g) in MeOH/THF (1:4; 150 mL) was hydrogenated in presence of TEA (4.28 mL) over 10% Pd/C (815 mg). The catalyst was filtered off and the filtrate was evaporated under reduced pressure. The residue was taken up in water and extracted with EA. The org. layer was dried over MgSO₄, evaporated under reduced pressure and the residue was purified by CC (EE/Hex 1.1) affording the title compound (70 mg; 2% yield) as well as its 6-fluoro-3-methoxy-quinoxaline-5-carbaldehyderegioisomer (1.74 g; 55% yield). 7-fluoro-2-methoxy-quinoxaline-6-carbaldehyde:
¹H NMR (CDCl₃) δ: 10.43 (s, 1H), 8.54 (d, J = 7.3 Hz, 1H), 7.55 (d, J = 11.4 Hz, 1H), 4.13 (s, 3H).
6-fluoro-3-methoxy-quinoxaline-5-carbaldehyde:
¹H NMR (CDCl₃) δ: 11.16 (dd, J = 1.5, 0.6 Hz, 1H), 8.52 (s, 1H), 8.24 (dd, J = 9.1, 5.6 Hz, 1H), 7.39 (m, 1H), 4.15 (s, 3H)

### Preparation of the compounds of Examples 1 to 29:

### Example 1: rac-6-(2-oxo-5-{3-[(quinolin-2-ylmethyl)-amino]-propyl}-oxazolidin-3-yl)-4H-benzo[1,4]thiazin-3-one:

Starting from 2-quinolinecarboxaldehyde (commercial) and intermediate 5 and using procedure A, the title compound was isolated as a yellowish foam (56 mg, 64% yield).
MS (ESI, m/z): 449.3 [M+H]⁺.

### Example 2: rac-6-(5-{3-[(7-fluoro-quinolin-2-ylmethyl)-amino]-propyl}-2-oxo-oxazolidin-3-yl)-4H-benzo[1,4]thiazin-3-one:

Starting from intermediate 13 and intermediate 5 and using procedure A, the title compound was isolated as a yellow oil (4 mg, 7% yield).
MS (ESI, m/z): 467.04 [M+H]⁺.

### Example 3: 6-((R)-5-{3-[(7-fluoro-quinolin-2-ylmethyl)-amino]-propyl}-2-oxo-oxazolidin-3-yl)-4H-benzo[1,4]oxazin-3-one:

Starting from intermediate 13 and intermediate 6 and using procedure A, the title compound was isolated as a colourless foam (70 mg, 45% yield).
¹H NMR (CDCl₃) δ: 8.10 (m, 1H), 7.78 (dd, J = 8.8, 6.2 Hz, 1H), 7.78 (dd, J = 8.8, 6.2 Hz, 1H), 7.78 (dd, J = 8.8, 6.2 Hz, 1H), 7.40 (m, 2H), 7.30 (m, 2H), 6.92 (m, 1H), 6.77 (dd, J = 8.8, 2.3 Hz, 1H), 4.68 (m, 1H), 4.57 (s, 2H), 4.11 (s, 2H), 4.03 (t, J=8.5Hz, 1H), 3.61 (dd, J = 8.5, 7.0 Hz, 1H), 2.82 (t, J = 7.0 Hz, 2H), 1.88 (m, 5H).
MS (ESI, m/z): 451.3 [M+H]⁺.

### Example 4: 6-((R)-2-oxo-5-{3-[(quinolin-2-ylmethyl)-amino]-propyl}-oxazolidin-3-yl)-4H-benzo[1,4]oxazin-3-one:

Starting from 2-quinolinecarboxaldehyde (commercial) and intermediate 6 and using procedure A, the title compound was isolated as a beige foam (60 mg, 40% yield).
¹H NMR (CDCl₃) δ: 8.12 (d, J = 8.5 Hz, 1H), 8.05 (m, 1H), 7.80 (dd, J = 7.9, 0.9 Hz, 1H), 7.70 (m, 1H), 7.51 (m, 1H), 7.42 (m, 2H), 6.93 (m, 1H), 6.78 (dd, J = 8.8, 2.6 Hz, 1 ), 4.67 (m, 1H), 4.58 (s, 2H), 4.12 (s, 2H), 4.02 (t, J = 8.5 Hz, 1H), 3.61 (dd, J = 8.5, 7.0 Hz, 1H), 2.83 (m, 2H), 1.88 (m, 5H).
MS (ESI, m/z): 433.3 [M+H]⁺.

### Example 5: 6-{(S)-2-oxo-5-[(3-quinolin-2-yl-propylamino)-methyl)-oxazolidin-3-yl}-4H-benzo[1,4]thiazin-3-one:

Starting from intermediate 16 and intermediate 7 and using procedure A, the title compound was isolated as a beige foam (40 mg, 21 % yield).
MS (ESI, m/z): 449.3 [M+H]⁺.

### Example 6: rac-6-(5-{3-[(6-fluoro-quinolin-7-ylmethyl)-amino]-propyl]-2-oxo oxazolidin-3-yl)-4H-benzo[1,4]thiazin-3-one:

Starting from intermediate 15 and intermediate 5 and using procedure A, the title compound was isolated, after purification by CC (DCM/MeOH/NH₄OH 100-50-4), as a colourless oil (8 mg, 21 % yield).
Retention time: 2.96 min (HPLC, Phenomenex Gemini 5 µ C18, 50 x 4.60 mm; 1.5 mL/min, 10% MeCN/water to 90% MeCN/water in 6.5 min).
¹H NMR (CDCl₃) δ: 8.91 (dd, J = 4.1, 1.5 Hz, 1H), 8.69 (s, 1H), 8.09 (m, 2H), 7.39 (m, 3H), 7.26 (m, 1H), 7.00 (dd, J = 8.5, 2.3 Hz, 1H), 4.66 (m, 1H), 4.05 (m, 3H), 3.64 (dd, J = 8.5, 6.7 Hz, 1H), 3.41 (s, 2H), 2.77 (t, J = 6.7 Hz, 2H), 1.82 (m, 5H).
MS (ESI, m/z): 467.2 [M+H]⁺.

### Example 7: rac-6-(5-{3-[(5-fluoro-quinolin-2-ylmethyl)-amino]-propyl}-2-oxo-oxazolidin-3-yl)-4H-benzo[1,4]thiazin-3-one:

Starting from intermediate 14 and intermediate 5 and using procedure A, the title compound was isolated as a yellow oil (17 mg, 33% yield).
MS (ESI, m/z): 467.0 [M+H]⁺.

### Example 8: rac-6-(5-{3-[(3-methyl-benzo[b]thiophen-2-ylmethyl)-amino]-propyl}-2-oxo-oxazolidin-3-yl)-4H-benzo[1,4]thiazin-3-one:

Starting from 3-methylbenzo[*b*]thiophene-2-carbaldehyde (commercial) and intermediate 5 and using procedure A, the title compound was isolated as a yellow oil (19 mg, 35% yield).
MS (ESI, m/z): 468.1 [M+H]⁺.

### Example 9: rac-6-(2-oxo-5-{3-[(thieno[2,3-b]pyridin-2-ylmethyl)-amino]-propyl}-oxazolidin-3-yl)-4H-benzo[1,4] thiazin-3-one:

Starting from thieno[2,3-b]pyridine-2-carboxaldehyde (commercial) and intermediate 5 and using procedure A, the title compound was isolated as a yellowish oil (12 mg, 23% yield).
MS (ESI, m/z): 455.1 [M+H]⁺.

### Example 10: rac-6-(2-oxo-5-{3-[(thieno[2,3-b]pyrazin-6-ylmethyl)-amino]-propyl}-oxazolidin-3-yl)-4H-benzo[1,4]thiazin-3-one:

Starting from thieno[2,3-*b*]pyrazine-6-carbaldehyde (commercial) and intermediate 5 and using procedure A, the title compound was isolated as a yellowish oil (3 mg, 7% yield).
MS (ESI, m/z): 456.1 [M+H]⁺.

### Example 11: rac-6-(2-oxo-5-{3-[(quinolin-3-ylmethyl)-amino]-propyl}-oxazolidin-3-yl)-4H-benzo[1,4]thiazin-3-one:

Starting from 3-quinolinecarboxaldehyde (commercial) and intermediate 5 and using procedure A, the title compound was isolated as a colourless foam (49 mg, 56% yield).
MS (ESI, m/z): 449.9 [M+H]⁺.

### Example 12: rac-6-(5-{3-[(naphthalen-2-ylmethyl)-amino]-propyl}-2-oxo-oxazolidin-3-yl)-4H-benzo[1,4]thiazin-3-one:

Starting from 2-naphthalenecarboxaldehyde (commercial) and intermediate 5 and using procedure A, the title compound was isolated as a colourless foam (44 mg, 50% yield).
MS (ESI, m/z): 448.3 [M+H]⁺.

### Example 13: rac-6-(2-oxo-5-{3-[(quinolin-7-ylmethyl)-amino]-propyl}-oxazolidin-3-yl)-4H-benzo[1,4]thiazin-3-one:

Starting from 7-quinolinecarboxaldehyde (commercial) and intermediate 5 and using procedure A, the title compound was isolated as a colourless foam (54 mg, 62% yield).
MS (ESI, m/z): 449.4 [M+H]⁺.

### Example 14: rac-6-(2-oxo-5-{3-[(quinolin-6-ylmethyl)-amino]-propyl}-oxazolidin-3-yl)-4H-benzo[1,4]thiazin-3-one:

Starting from 6-quinolinecarboxaldehyde (commercial) and intermediate 5 and using procedure A, the title compound was isolated as a colourless foam (44 mg, 50% yield).
MS (ESI, m/z): 449.4 [M+H]⁺.

### Example 15: 6-((R)-2-oxo-5-{2-[(quinolin-2-ylmethyl)-amino]-ethyl}-oxazolidin-3-yl)-4H-benzo[1,4] thiazin-3-one:

Starting from 2-quinolinecarboxaldehyde (commercial) and intermediate 8 and using procedure A, the title compound was isolated as a beige foam (20 mg, 13% yield).
MS (ESI, m/z): 435.2 [M+H]⁺.

### Example 16: 6-{(R)-2-oxo-5-[2-(2-quinolin-2-yl-ethylamino)-ethyl]-oxazolidin-3-yl}-4H-benzo[1,4]thiazin-3-one:

A solution of 2-vinylquinoline (100 mg; commercial) and intermediate 8 (189 mg) in MeOH (2 mL) was treated with AcOH (0.037 mL) and stirred at 90°C overnight in a sealed flask. The reaction mixture was concentrated under reduced pressure and the residue was taken up in aq. NH₄OH and extracted with EA. The org. layer was dried over Na₂SO₄ and purified by CC (EA/MeOH 9:1 to 4:1, +1% NH₄OH), affording a beige foam (120 mg 41% yield).
¹H NMR (DMSO-d6) δ: 10.54 (m, 1H), 8.24 (d, J = 8.2 Hz, 1H), 7.91 (m, 2H), 7.69 (m, 1H), 7.52 (m, 1H), 7.45 (d, J = 8.5 Hz, 1H), 7.34 (d, J = 2.3 Hz, 1H), 7.28 (d, J = 8.8 Hz, 1H), 7.02 (dd, J = 8.5, 2.3 Hz, 1H), 4.72 (m, 1H), 4.03 (m, 1H), 3.67 (dd, J = 8.8, 7.3 Hz, 1H), 3.42 (s, 2H), 3.01 (m, 5H), 2.69 (m, 2H), 1.85 (m, 2H).
MS (ESI, m/z): 449.3[M+H]⁺;

### Example 17: 6-{(R)-5-[(R)-3-(7-methoxy-quinolin-2-yloxymethyl)-pyrrolidin-1-ylmethyl]-2-oxo-oxazolidin-3-yl}-4H-benzo[1,4]thiazin-3-one:

Starting from intermediate 12 and intermediate 2 and using procedure C, the title compound was isolated as a beige foam (70 mg, 35% yield).
MS (ESI, m/z): 521.2 [M+H]⁺.

### Example 18: 6-{(R)-5-[4-(7-methoxy-quinolin-2-yloxymethyl)-piperidin-1-ylmethyl]-2-oxo-oxazolidin-3-yl}-4H-benzo[1,4]thiazin-3-one:

Starting from intermediate 9 (111 mg, 0.4 mmol) and intermediate 2 (176 mg, 0.45 mmol) and using procedure C, the title compound was isolated as a beige solid (67 mg, 31 % yield) after CC (EA/MeOH 9:1 containing 1% NH₄OH).
¹H NMR (DMSO d6) δ: 10.54 (s, 1H), 8.11 (d, J = 8.8 Hz, 1H), 7.74 (d, J = 9.1 Hz, 1H), 7.31 (m, 2H), 7.13 (m, 2H), 7.04 (dd, J = 8.8, 2.6 Hz, 1H), 6.81 (d, J = 8.8 Hz, 1H), 4.83 (m, 1H), 4.23 (d, J = 5.9 Hz, 2H), 4.07 (s, 1H), 3.87 (s, 3H), 3.70 (m, 1H), 3.43 (s, 2H), 2.95 (m, 2H), 2.65 (d, J = 5.9 Hz, 2H), 2.11 (m, 2H), 1.78 (s, 3H).
MS (ESI, m/z): 535.5 [M+H]⁺.

### Example 19: (R)-3-(2,3-dihydro-benzo[1,4]dioxin-6-yl)-5-[3-(7-methoxy-quinoxalin-2-yloxymethyl)-azetidin-1-ylmethyl]-oxazolidin-2-one:

Starting from intermediate 10 and intermediate 3 and using procedure C, the title compound was isolated as a beige foam (56 mg, 29% yield).
MS (ESI, m/z): 479.3 [M+H]⁺.

### Example 20: 6-((R)-5-{2-[3-(7-methoxy-quinoxalin-2-yloxymethyl)-azetidin-1-yl]-ethyl}-2-oxo-oxazolidin-3-yl)-4H-benzo[1,4]thiazin-3-one:

Starting from intermediate 10 and intermediate 4 and using procedure C, the title compound was isolated as a beige foam (110 mg, 52% yield).
MS (ESI, m/z): 522.3[M+H]⁺.

### Example 21: 6-{(R)-5-[3-(7-methoxy-quinoxalin-2-yloxymethyl)-azetidin-1-ylmethyl]-2-oxo-oxazolidin-3-yl}-4H-benzo[1,4]thiazin-3-one:

Starting from intermediate 10 and intermediate 2 and using procedure C, the title compound was isolated as a beige solid (70 mg, 34% yield).
MS (ESI, m/z): 508.3 [M+H]⁺.

### Example 22: 6-{(R)-5-[3-(7-methoxy-quinoxalin-2-yloxymethyl)-azetidin-1-ylmethyl]-2-oxo-oxazolidin-3-yl}-4H-benzo[1,4]oxazin-3-one:

Starting from intermediate 10 and intermediate 1 and using procedure B, the title compound was isolated as a beige solid (40 mg, 20% yield) after CC (EA/MeOH 9:1 containing 1% NH₄OH).
¹H NMR (DMSO d6) δ: 10.69 (s, 1H), 8.38 (s, 1H), 7.87 (d, J = 8.8 Hz, 1H), 7.26 (m, 3H), 6.93 (d, J = 1.5 Hz, 2H), 4.53 (m, 4H), 4.00 (m, 1H), 3.91 (s, 3H), 3.72 (m, 1H), 3.45 (m, 2H), 3.13 (m, 2H), 2.74 (dd, J = 6.7, 4.4 Hz, 2H), 2.4 (m, 2H).
MS (ESI, m/z): 492.3 [M+H]⁺.

### Example 23: 6-((R)-5-{[3-(7-methoxy-quinolin-2-yloxy)-cyclobutylamino]-methyl}-2-oxo-oxazolidin-3-yl)-4H-benzo[1,4]thiazin-3-one (mixture of cis- and trans-isomers):

Starting from intermediate 11 and intermediate 2 and using procedure C, the title compound was isolated as a yellow solid (64 mg, 19% yield).
MS (ESI, m/z): 507.0 [M+H]⁺.

### Example 24: 6-{(R)-2-oxo-5-[(2-quinolin-2-yl-ethylamino)-methyl]-oxazolidin-3-yl}-4H-benzo[1,4]thiazin-3-one:

A solution of 6-[(5*R*)-5-(aminomethyl)-2-oxo-oxazolidin-3-yl]-4*H*-benzo[1,4]thiazin-3-one (180 mg, prepared according to WO 2008/126024) and 2-vinyl-quinoline (100 mg; commercial) in MeOH (2 mL) was treated with AcOH (0.037 mL) and refluxed at 90°C overnight. The solvent was removed under reduced pressure and the residue was taken up in EA/aq. NH₄OH. The org. phase was separated, dried over Na₂SO₄, filtered, evaporated and purified by CC (DCM/MeOH/19:1 to 9:1 (+ 1% NH₄OH)), affording a beige foam (30 mg; 11 % yield).
¹H NMR (DMSO-d6) δ: 10.53 (s, 1H), 8.20 (d, J = 8.2 Hz, 1H), 7.89 (m, 2H), 7.68 (m, 1H), 7.51 (m, 1H), 7.43 (d, J = 8.5 Hz, 1H), 7.29 (m, 2H), 7.04 (dd, J = 8.5, 2.3 Hz, 1H), 4.71 (m, 1H), 3.99 (t, J = 8.8 Hz, 1H), 3.72 (dd, J = 8.8, 6.7 Hz, 1H), 3.42 (s, 2H), 3.04 (m, 4H), 2.88 (m, 2H).
MS (ESI, m/z): 435.1 [M+H]⁺.

### Example 25: 6-{(R)-2-oxo-5-[3-(2-quinolin-2-yl-ethylamino)-propyl]-oxazolidin-3-yl}-4H-benzo[1,4]thiazin-3-one:

Starting from intermediate 17 (198 mg) and 2-vinyl-quinoline (100 mg; commercial) and proceeding in analogy to the protocol of Example 24, the title compound was obtained as a pale yellow foam (50 mg; 17% yield).
MS (ESI, m/z): 463.0 [M+H]⁺.

### Example 26: 6-((R)-2-oxo-5-{2-[2-(quinolin-2-ylaminopethylamino]-ethyl}-oxazolidin-3-yl)-4H-benzo[1,4]oxazin-3-one:

Starting from N¹-quinolin-2-yl-ethane-1,2-diamine (30 mg; commercial) and intermediate 18 (64 mg) and using procedure C, the title compound was isolated as a beige solid (4 mg; 5% yield).
MS (ESI, m/z): 448.3 [M+H]⁺.

### Example 27: 6-[(R)-2-oxo-5-({2-[(quinolin-2-ylmethyl)-amino]-ethylamino}-methyl)-oxazolidin-3-yl]-4H-benzo[1,4] thiazin-3-one:

Starting from N¹-(2-quinolinylmethyl)-1,2-ethanediamine (90 mg; prepared according to WO 00/50086) and intermediate 2 (175 mg) and using procedure C, the title compound was isolated as a beige solid (5 mg; 5% yield).
MS (ESI, m/z): 464.2 [M+H]⁺.

### Example 28: 6-[(R)-2-oxo-5-(4-quinolin-2-ylmethyl-piperazin-1-ylmethyl)-oxazolidin-3-yl]-4H-benzo[1,4]thiazin-3-one:

Starting from 2-(1-piperazinylmethyl)-quinoline (40 mg; commercial) and intermediate 2 (69 mg) and using procedure C, the title compound was isolated as a beige solid (20 mg; 23% yield).
¹H NMR (DMSO-d6) δ: 10.52 (s, 1H), 8.30 (d, J = 8.2 Hz, 1H), 7.94 (m, 2H), 7.71 (m, 1H), 7.61 (d, J = 8.5 Hz, 1H), 7.55 (m, 1H), 7.29 (m, 2H), 7.11 (dd, J = 8.5, 2.3 Hz, 1H), 4.81 (d, J = 1.5 Hz, 1H), 4.05 (t, J = 8.8 Hz, 1H), 3.74 (s, 2H), 3.68 (dd, J = 8.8, 7.0 Hz, 1H), 3.42 (s, 2H), 2.65 (d, J = 5.9 Hz, 2H), 2.50 (m, 8H)
MS (ESI, m/z): 490.2 [M+H]⁺.

### Example 29: 6-((S)-5-{3-[(7-fluoro-2-methoxy-quinoxalin-6-ylmethyl)-amino]-propyl}-2-oxo-oxazolidin-3-yl)-4H-benzo[1,4]thiazin-3-one:

Starting from intermediate 19 (70 mg) and intermediate 17 (104 mg) and using procedure A, the title compound was isolated as a yellow solid (24 mg; 14% yield).
MS (ESI, m/z): 498.2 [M+H]⁺.

### Pharmacological properties of the invention compounds

### In vitro assays

### Experimental methods:

Minimal inhibitory concentrations (MICs; mg/L) were determined in cation-adjusted Mueller-Hinton Broth by a microdilution method following the description given in "Methods for Dilution Antimicrobial Susceptibility Tests for Bacteria that Grow Aerobically", Approved standard, 7th ed., Clinical and Laboratory Standards Institute (CLSI) Document M7-A7, Wayne, PA, USA, 2006.

### Results:

All Example compounds were tested against several Gram positive and Gram negative bacteria such as *S*. *aureus, E. faecalis, S. pneumoniae, M. catarrhalis, A. baumanii, E.coli* or *P. aeruginosa.*

Typical antibacterial test results are given in the table hereafter (MIC in mg/L).

| **Example No.** | **MIC for *M. catarrhalis* A894** | **Example No.** | **MIC for *M. catarrhalis* A894** |
|---|---|---|---|
| 1 | 0.5 | 2 | 0.031 |
| 3 | ≤ 0.031 | 4 | 0.063 |
| 5 | ≤ 0.031 | 6 | ≤ 0.031 |
| 7 | 0.063 | 8 | 0.063 |
| 9 | 0.125 | 10 | 0.25 |
| 11 | 4 | 12 | 1 |
| 13 | 0.5 | 14 | 1 |
| 15 | ≤ 0.031 | 16 | ≤ 0.031 |
| 17 | ≤ 0.031 | 18 | 0.5 |
| 19 | 0.25 | 20 | ≤ 0.031 |
| 21 | ≤ 0.031 | 22 | ≤ 0.031 |
| 23 | ≤ 0.031 | 24 | 0.031 |
| 25 | 0.125 | 26 | 0.25 |
| 27 | 1 | 28 | 0.063 |
| 29 | ≤ 0.031 | | |

## Claims

1. A compound of formula (I) 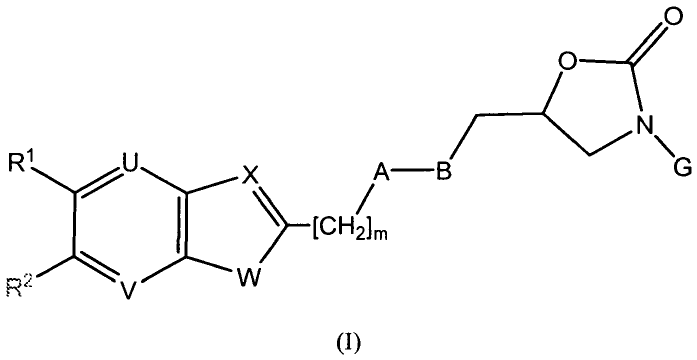 wherein
R¹ represents hydrogen, (C₁₋₄)alkoxy or halogen;
R² represents hydrogen or (C₁₋₄)alkoxy;
U represents N or CH;
V represents N or CR^{b}, wherein R^{b} is hydrogen or halogen;
W represents *-CH=CR^{a}-, *-N=CH- or S, wherein the asterisks indicate the bond which is linked to the carbon atom connecting V and W and wherein R^{a} represents hydrogen or halogen;
X represents N or CR^{c}, wherein R^{c} is hydrogen, (C₁₋₄)alkyl or halogen;
with the proviso that the group of formula (D) 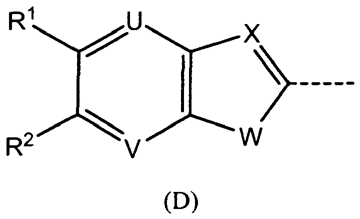 contains between none and three heteroatoms, wherein the heteroatoms are independently selected from nitrogen and, in case of W, sulfur;
m represents 1, A represents -NHCH₂-^{#}, -CH₂NH-^{#}, -NHCH₂CH₂-^{#}, -CH₂NHCH₂- or -CH₂CH₂NH-^{#}, -NHCH₂CH₂NH-, -CH₂NHCH₂CH₂-^{#} or piperazin-1,4-diyl, wherein the hash indicates the bond which is linked to B, and B represents a bond; or
m represents 0, A represents -NHCH₂CH₂NHCH₂-^{#} wherein the hash indicates the bond which is linked to B, and B represents a bond; or
m represents 0, A represents -OCH₂-^{#}, wherein the hash indicates the bond which is linked to B, and B represents a group of the formula (E) 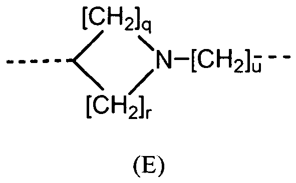 wherein q and r independently from each other represent 1 or 2 and u represents 0 or 1; or
m represents 0, A represents O and B represents a group of the formula (F) 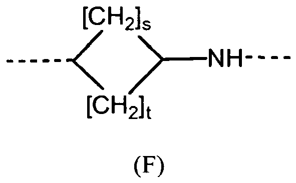 wherein s and t independently from each other represent 1 or 2;
G represents a group of the formula (G1) 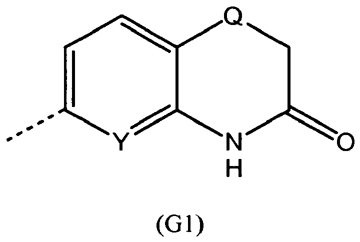 wherein Y represents CH or N, and Q represents O or S; or
G represents a group of the formula (G2) 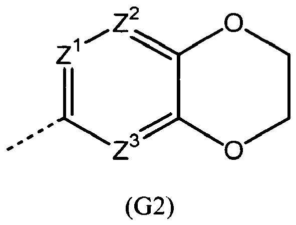 wherein
Z¹ represents CH, Z² represents CH, and Z³ represents N; or
Z¹ represents N, Z² represents CH or N, and Z³ represents CH; or
Z¹ represents CH, Z² represents CH or N, and Z³ represents CH;
or a salt of such a compound.

2. A compound of formula (I) according to claim 1, which is also a compound of formula (I_{P}) 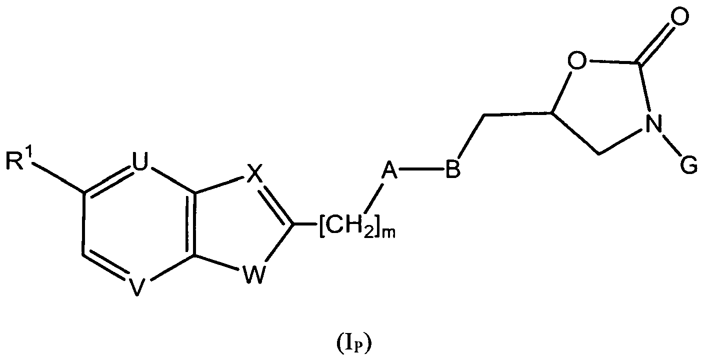 wherein
R¹ represents hydrogen, (C₁₋₄)alkoxy or halogen;
U represents N or CH;
V represents N or CR^{b}, wherein R^{b} is hydrogen or halogen;
W represents *-CH=CR^{a}-, *-N=CH- or S, wherein the asterisks indicate the bond which is linked to the carbon atom connecting V and W and wherein R^{a} represents hydrogen or halogen;
X represents N or CR^{c}, wherein R^{c} is hydrogen, (C₁₋₄)alkyl or halogen;
with the proviso that the group of formula (D) 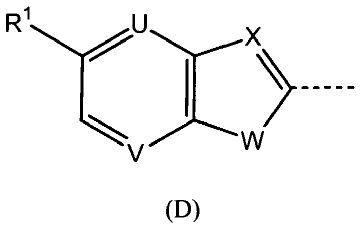 contains between none and three heteroatoms, wherein the heteroatoms are independently selected from nitrogen and, in case of W, sulfur;
m represents 1, A represents -NHCH₂-^{#}, -NHCH₂CH₂-^{#}, -CH₂NHCH₂- or -CH₂CH₂NH-^{#}, wherein the hash indicates the bond which is linked to B, and B represents a bond; or
m represents 0, A represents -OCH₂-^{#}, wherein the hash indicates the bond which is linked to B, and B represents a group of the formula (E) 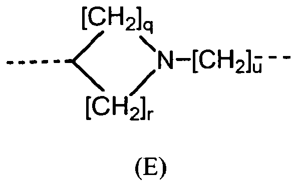 wherein q and r independently from each other represent 1 or 2 and u represents 0 or 1; or
m represents 0, A represents O and B represents a group of the formula (F) 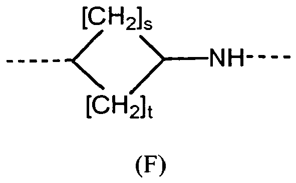 wherein s and t independently from each other represent 1 or 2;
G represents a group of the formula (G1) 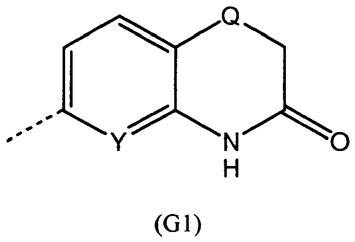 wherein Y represents CH or N, and Q represents O or S; or
G represents a group of the formula (G2) 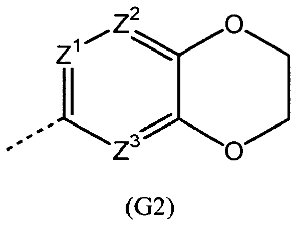 wherein
Z¹ represents CH, Z² represents CH, and Z³ represents N; or
Z¹ represents N, Z² represents CH or N, and Z³ represents CH; or
Z¹ represents CH, Z² represents CH or N, and Z³ represents CH;
or a salt of such a compound.

3. A compound of formula (I) according to claim 1, which is also a compound of formula (I_{CE}) 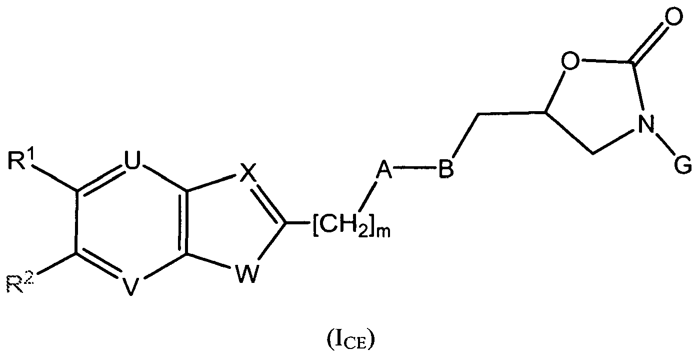 wherein
R¹ represents hydrogen, (C₁₋₄)alkoxy or halogen;
R² represents hydrogen or also, if R¹ represents hydrogen, (C₁₋₄)alkoxy;
U represents CH, V represents CR^{b}, wherein R^{b} is hydrogen or halogen, W represents - CH=CH- or *-N=CH-, wherein the asterisk indicates the bond which is linked to the carbon atom connecting V and W, and X represents N; or
U represents CH or N, V represents CH or N, W represents S and X represents CR^{c}, wherein R^{c} is hydrogen or (C₁₋₄)alkyl (notably methyl); or
U represents CH or N, V represents CH, W represents *-CH=CR^{a}- or *-N=CH-, wherein the asterisks indicate the bond which is linked to the carbon atom connecting V and W and wherein R^{a} represents hydrogen or halogen (notably fluorine), and X represents CH; or
U represents CH or N, V represents N, W represents -CH=CH-, and X represents CH;
m represents 1, A represents -NHCH₂-^{#}, -CH₂NH-^{#}, -NHCH₂CH₂-^{#}, -CH₂NHCH₂-, -CH₂CH₂NH-^{#}, -NHCH₂CH₂NH-^{#}, -CH₂NHCH₂CH₂-^{#} or piperazin-1,4-diyl, wherein the hash indicates the bond which is linked to B, and B represents a bond; or
m represents 0, A represents -NHCH₂CH₂NHCH₂-^{#} wherein the hash indicates the bond which is linked to B, and B represents a bond; or
m represents 0, A represents -OCH₂-^{#}, wherein the hash indicates the bond which is linked to B, and B represents a group of the formula (E) 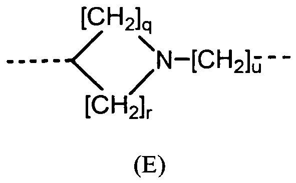 wherein q and r independently from each other represent 1 or 2 and u represents 0 or 1; or
m represents 0, A represents O and B represents a group of the formula (F) 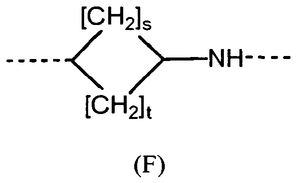 wherein s and t both represent 1;
G represents a group of the formula (G1-a) 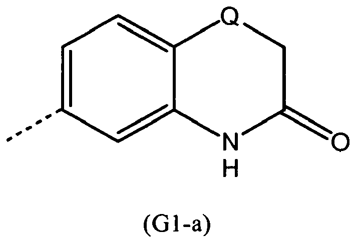 wherein Q represents O or S; or
G represents a 2,3-dihydro-benzo[1,4]dioxin-6-yl group;
or a salt of such a compound.

4. A compound of formula (I) according to any of claims 1 to 3, wherein R¹ represents hydrogen, methoxy or fluorine;
or a salt of such a compound.

5. A compound of formula (I) according to any one of claims 1 to 4, wherein U represents CH, V represents CR^{b}, wherein R^{b} is hydrogen or halogen, W represents -CH=CH- or *-N=CH-, wherein the asterisk indicates the bond which is linked to the carbon atom connecting V and W, and X represents N;
or a salt of such a compound.

6. A compound of formula (I) according to any one of claims 1 to 4, wherein U represents CH or N, V represents CH or N, W represents S, and X represents CR^{c}, wherein R^{c} is hydrogen or (C₁₋₄)alkyl;
or a salt of such a compound.

7. A compound of formula (I) according to any one of claims 1 to 4, wherein U represents CH or N, V represents CH, W represents *-CH=CR^{a}- or *-N=CH-, wherein the asterisks indicate the bond which is linked to the carbon atom connecting V and W and wherein R^{a} represents hydrogen or halogen, and X represents CH;
or a salt of such a compound.

8. A compound of formula (I) according to any one of claims 1 to 4, wherein U represents CH or N, V represents N, W represents -CH=CH-, and X represents CH;
or a salt of such a compound.

9. A compound of formula (I) according to any one of claims 1 to 8, wherein m represents 1, A represents -NHCH₂-^{#} or -NHCH₂CH₂-^{#}, wherein the hash indicates the bond which is linked to B, and B represents a bond;
or a salt of such a compound.

10. A compound of formula (I) according to any one of claims 1 to 8, wherein m represents 0, A represents -OCH₂-^{#}, wherein the hash indicates the bond which is linked to B, and B represents a group of the formula (E), wherein q and r independently from each other represent 1 or 2 and u represents 0;
or a salt of such a compound.

11. A compound of formula (I) according to any one of claims 1 to 8, wherein m represents 0, A represents O and B represents a group of the formula (F), wherein s and t both represent 1;
or a salt of such a compound.

12. A compound of formula (I) according to claim 1 or 2, which is selected from the following:
- 6-((*R*)-5-{3-[(7-fluoro-quinolin-2-ylmethyl)-amino]-propyl}-2-oxo-oxazolidin-3-yl)-4*H*-benzo[ 1,4]oxazin-3-one;
- 6-((*R*)-2-oxo-5-{3-[(quinolin-2-ylmethyl)-amino]-propyl}-oxazolidin-3-yl)-4*H*-benzo[1,4]oxazin-3-one;
- 6-{(*S*)-2-oxo-5-[(3-quinolin-2-yl-propylamino)-methyl]-oxazolidin-3-yl}-4*H*-benzo[1,4]thiazin-3-one;
- 6-((*R*)-2-oxo-5-{2-[(quinolin-2-ylmethyl)-amino]-ethyl}-oxazolidin-3-yl)-4*H*-benzo[ 1,4]thiazin-3-one;
- 6-{(*R*)-2-oxo-5-[2-(2-quinolin-2-yl-ethylamino)-ethyl]-oxazolidin-3-yl}-4*H*-benzo[1,4]thiazin-3-one;
- 6-{(*R*)-5-[(*R*)-3-(7-methoxy-quinolin-2-yloxymethyl)-pyrrolidin-1-ylmethyl]-2-oxo-oxazolidin-3-yl}-4*H*-benzo[1,4]thiazin-3-one;
- 6-{(*R*)-5-[4-(7-methoxy-quinolin-2-yloxymethyl)-piperidin-1-ylmethyl]-2-oxo-oxazolidin-3-yl}-4*H*-benzo[1,4]thiazin-3-one;
- (*R*)-3-(2,3-dihydro-benzo[1,4]dioxin-6-yl)-5-[3-(7-methoxy-quinoxalin-2-yloxymethyl)-azetidin-1-ylmethyl]-oxazolidin-2-one;
- 6-((*R*)-5-{2-[3-(7-methoxy-quinoxalin-2-yloxymethyl)-azetidin-1-yl]-ethyl}-2-oxo-oxazolidin-3-yl)-4*H*-benzo[1,4]thiazin-3-one;
- 6-{(*R*)-5-[3-(7-methoxy-quinoxalin-2-yloxymethyl)-azetidin-1-ylmethyl]-2-oxo-oxazolidin-3-yl}-4*H*-benzo[1,4]thiazin-3-one;
- 6-{(*R*)-5-[3-(7-methoxy-quinoxalin-2-yloxymethyl)-azetidin-1-ylmethyl]-2-oxo-oxazolidin-3-yl}-4*H*-benzo[1,4]oxazin-3-one;
- 6-((*R*)-5-{[3-(7-methoxy-quinolin-2-yloxy)-cyclobutylamino]-methyl}-2-oxo-oxazolidin-3-yl)-4*H*-benzo[1,4]thiazin-3-one;
- 6-(2-oxo-5-{3-[(quinolin-2-ylmethyl)-amino]-propyl} -oxazolidin-3-yl)-4*H*-benzo[ 1,4]thiazin-3-one;
- 6-(5-{3-[(7-fluoro-quinolin-2-ylmethyl)-amino]-propyl}-2-oxo-oxazolidin-3-yl)-4*H*-benzo[ 1,4]thiazin-3-one;
- 6-(5-{3-[(6-fluoro-quinolin-7-ylmethyl)-amino]-propyl}-2-oxo-oxazolidin-3-yl)-4*H*-benzo[1,4]thiazin-3-one;
- 6-(5-{3-[(5-fluoro-quinolin-2-ylmethyl)-amino]-propyl}-2-oxo-oxazolidin-3-yl)-4*H*-benzo[1,4]thiazin-3-one;
- 6-(5-{3-[(3-methyl-benzo[b]thiophen-2-ylmethyl)-amino]-propyl}-2-oxo-oxazolidin-3-yl)-4*H*-benzo[1,4]thiazin-3-one;
- 6-(2-oxo-5-{3-[(thieno[2,3-b]pyridin-2-ylmethyl)-amino]-propyl}-oxazolidin-3-yl)-4*H*-benzo[1,4]thiazin-3-one;
- 6-(2-oxo-5-{3-[(thieno[2,3-b]pyrazin-6-ylmethyl)-amino]-propyl}-oxazolidin-3-yl)-4*H*-benzo[1,4]thiazin-3-one;
- 6-(2-oxo-5-{3-[(quinolin-3-ylmethyl)-amino]-propyl}-oxazolidin-3-yl)-4*H*-benzo[1,4]thiazin-3-one;
- 6-(5-{3-[(naphthalen-2-ylmethyl)-amino]-propyl}-2-oxo-oxazolidin-3-yl)-4*H*-benzo[1,4]thiazin-3-one;
- 6-(2-oxo-5-{3-[(quinolin-7-ylmethyl)-amino]-propyl}-oxazolidin-3-yl)-4*H*-benzo[1,4]thiazin-3-one; and
- 6-(2-oxo-5-{3-[(quinolin-6-ylmethyl)-amino]-propyl}-oxazolidin-3-yl)-4*H*-benzo[1,4]thiazin-3-one;
- 6-{(*R*)-2-oxo-5-[(2-quinolin-2-yl-ethylamino)-methyl]-oxazolidin-3-yl}-4*H*-benzo[ 1,4]thiazin-3-one;
- 6-{(*R*)-2-oxo-5-[3-(2-quinolin-2-yl-ethylamino)-propyl]-oxazolidin-3-yl}-4*H*-benzo[ 1,4]thiazin-3-one;
- 6-((*R*)-2-oxo-5-{2-[2-(quinolin-2-ylamino)-ethylamino]-ethyl}-oxazolidin-3-yl)-4*H*-benzo[ 1,4]oxazin-3-one;
- 6-[(*R*)-2-oxo-5-({2-[(quinolin-2-ylmethyl)-amino]-ethylamino}-methyl)-oxazolidin-3-yl]-4*H*-benzo[1,4]thiazin-3-one;
- 6-[(*R*)-2-oxo-5-(4-quinolin-2-ylmethyl-piperazin-1-ylmethyl)-oxazolidin-3-yl]-4*H*-benzo[ 1,4]thiazin-3-one;
- 6-((*S*)-5-{3-[(7-fluoro-2-methoxy-quinoxalin-6-ylmethyl)-amino]-propyl}-2-oxo-oxazolidin-3-yl)-4*H*-benzo[ 1,4]thiazin-3-one;
or a salt of such a compound.

13. As a medicament, a compound of formula (I) as defined in any one of claims 1 to 12, or a pharmaceutically acceptable salt thereof.

14. A pharmaceutical composition containing, as active principle, a compound of formula (I) as defined in any one of claims 1 to 12, or a pharmaceutically acceptable salt thereof, and at least one therapeutically inert excipient.

15. Use of a compound of formula (I) as defined in any one of claims 1 to 12, or of a pharmaceutically acceptable salt thereof, for the manufacture of a medicament for the prevention or treatment of a bacterial infection.

16. A compound of formula (I) as defined in any one of claims 1 to 12, or a pharmaceutically acceptable salt thereof, for the prevention or treatment of a bacterial infection.

## Patentansprüche

1. Verbindung der Formel (I) 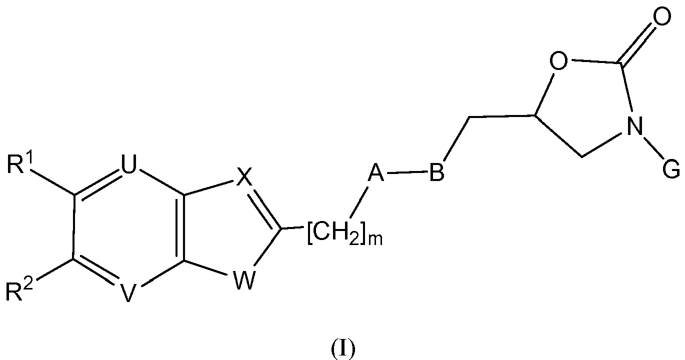 wobei
R¹ Wasserstoff, (C₁₋₄)Alkoxy oder Halogen repräsentiert;
R² Wasserstoff oder (C₁₋₄)Alkoxy repräsentiert;
U N oder CH repräsentiert;
V N oder CR^{b} repräsentiert, wobei R^{b} Wasserstoff oder Halogen ist;
W *-CH=CR^{a}-, *-N=CH- oder S repräsentiert, wobei die Sternchen die Bindung andeuten, die mit dem Kohlenstoffatom verknüpft ist, das V und W verbindet, und wobei R^{a} Wasserstoff oder Halogen repräsentiert;
X N oder CR^{c} repräsentiert, wobei R^{c} Wasserstoff, (C₁₋₄)Alkyl oder Halogen ist;
unter der Voraussetzung, dass die Gruppe der Formel (D) 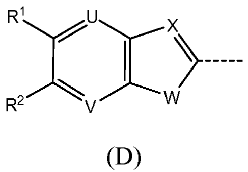 zwischen null und drei Heteroatome enthält, wobei die Heteroatome unabhängig ausgewählt sind aus Stickstoff und, im Falle von W, Schwefel;
m 1 repräsentiert, A -NHCH₂-^{#}, -CH₂NH-^{#}, -NHCH₂CH₂-^{#}, -CH₂NHCH₂- oder -CH₂CH₂NH-^{#}, -NHCH₂CH₂NH-, -CH₂NHCH₂CH₂-^{#} oder Piperazin-1,4-diyl repräsentiert, wobei die Raute die Bindung andeutet, die mit B verknüpft ist, und B eine Bindung repräsentiert; oder
m 0 repräsentiert, A -NHCH₂CH₂NHCH₂-^{#} repräsentiert, wobei die Raute die Bindung andeutet, die mit B verknüpft ist, und B eine Bindung repräsentiert; oder
m 0 repräsentiert, A -OCH₂-^{#} repräsentiert, wobei die Raute die Bindung andeutet, die mit B verknüpft ist, und B eine Gruppe der Formel (E) repräsentiert 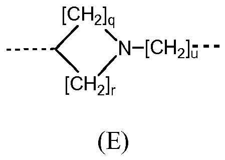 wobei q und r unabhängig voneinander 1 oder 2 repräsentieren und u 0 oder 1 repräsentiert; oder
m 0 repräsentiert, A O repräsentiert und B eine Gruppe der Formel (F) repräsentiert 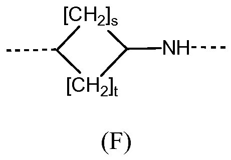 wobei s und t unabhängig voneinander 1 oder 2 repräsentieren;
G eine Gruppe der Formel (G1) repräsentiert 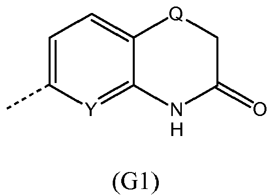 wobei Y CH oder N repräsentiert und Q O oder S repräsentiert; oder
G eine Gruppe der Formel (G2) repräsentiert 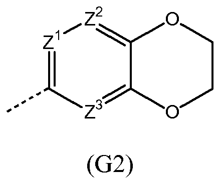 wobei
Z¹ CH repräsentiert, Z² CH repräsentiert und Z³ N repräsentiert; oder
Z¹ N repräsentiert, Z² CH oder N repräsentiert und Z³ CH repräsentiert; oder
Z¹ CH repräsentiert, Z² CH oder N repräsentiert und Z³ CH repräsentiert;
oder ein Salz einer solchen Verbindung.

2. Verbindung der Formel (I) nach Anspruch 1, die auch eine Verbindung der Formel (I_{P}) ist 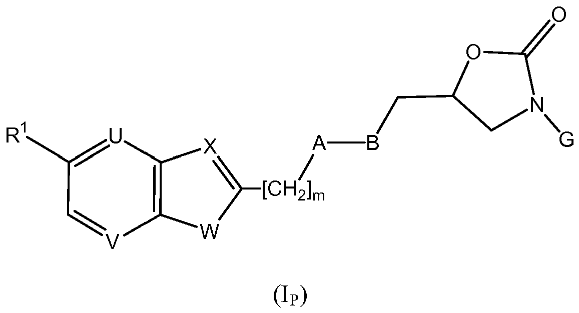 wobei
R¹ Wasserstoff, (C₁₋₄)Alkoxy oder Halogen repräsentiert;
U N oder CH repräsentiert;
V N oder CR^{b} repräsentiert, wobei R^{b} Wasserstoff oder Halogen ist;
W *-CH=CR^{a}-, *-N=CH- oder S repräsentiert, wobei die Sternchen die Bindung andeuten, die mit dem Kohlenstoffatom verknüpft ist, das V und W verbindet, und wobei R^{a} Wasserstoff oder Halogen repräsentiert;
X N oder CR^{c} repräsentiert, wobei R^{c} Wasserstoff, (C₁₋₄)Alkyl oder Halogen ist;
unter der Voraussetzung, dass die Gruppe der Formel (D) 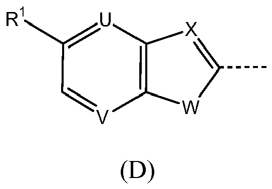 zwischen null und drei Heteroatome enthält, wobei die Heteroatome unabhängig ausgewählt sind aus Stickstoff und, im Falle von W, Schwefel;
m 1 repräsentiert, A -NHCH₂-^{#}, -NHCH₂CH₂-^{#}, -CH₂NHCH₂- oder -CH₂CH₂NH-^{#} repräsentiert, wobei die Raute die Bindung andeutet, die mit B verknüpft ist, und B eine Bindung repräsentiert; oder
m 0 repräsentiert, A -OCH₂-^{#} repräsentiert, wobei die Raute die Bindung andeutet, die mit
B verknüpft ist, und B eine Gruppe der Formel (E) repräsentiert 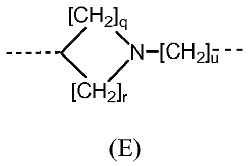 wobei q und r unabhängig voneinander 1 oder 2 repräsentieren und u 0 oder 1 repräsentiert; oder
m 0 repräsentiert, A O repräsentiert und B eine Gruppe der Formel (F) repräsentiert 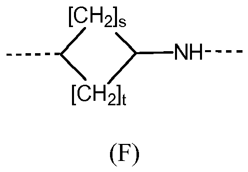 wobei s und t unabhängig voneinander 1 oder 2 repräsentieren;
G eine Gruppe der Formel (G1) repräsentiert 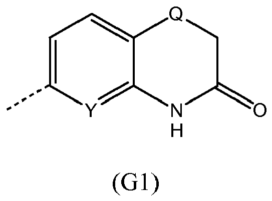 wobei Y CH oder N repräsentiert und Q O oder S repräsentiert; oder
G eine Gruppe der Formel (G2) repräsentiert 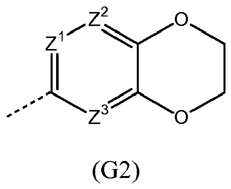 wobei Z¹ CH repräsentiert, Z² CH repräsentiert und Z³ N repräsentiert; oder
Z¹ N repräsentiert, Z² CH oder N repräsentiert und Z³ CH repräsentiert; oder
Z¹ CH repräsentiert, Z² CH oder N repräsentiert und Z³ CH repräsentiert;
oder ein Salz einer solchen Verbindung.

3. Verbindung der Formel (I) nach Anspruch 1, die auch eine Verbindung der Formel (I_{CE}) ist 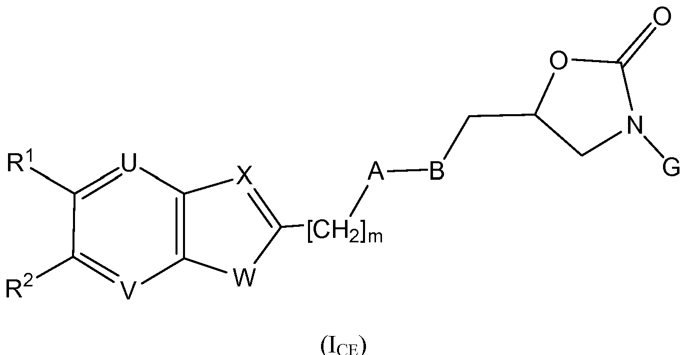 wobei
R¹ Wasserstoff, (C₁₋₄)Alkoxy oder Halogen repräsentiert;
R² Wasserstoff oder auch, wenn R¹ Wasserstoff repräsentiert, (C₁₋₄)Alkoxy repräsentiert; U CH repräsentiert, V CR^{b} repräsentiert, wobei R^{b} Wasserstoff oder Halogen ist, W -CH=CH- oder *-N=CH- repräsentiert, wobei das Sternchen die Bindung andeutet, die mit dem Kohlenstoffatom verknüpft ist, das V und W verbindet, und X N repräsentiert; oder U CH oder N repräsentiert, V CH oder N repräsentiert, W S repräsentiert und X CR^{c} repräsentiert, wobei R^{c} Wasserstoff oder (C₁₋₄)Alkyl (insbesondere Methyl) ist; oder
U CH oder N repräsentiert, V CH repräsentiert, W *-CH=CR^{a}- oder *-N=CH-repräsentiert, wobei die Sternchen die Bindung andeuten, die mit dem Kohlenstoffatom verknüpft ist, das V und W verbindet, und wobei R^{a} Wasserstoff oder Halogen (insbesondere Fluor) repräsentiert und X CH repräsentiert; oder
U CH oder N repräsentiert, V N repräsentiert, W -CH=CH- repräsentiert und X CH repräsentiert;
m 1 repräsentiert, A -NHCH₂-^{#}, -CH₂NH-^{#}, -NHCH₂CH₂-^{#}, -CH₂NHCH₂-, -CH₂CH₂NH-^{#}, -NHCH₂CH₂NH-^{#}, -CH₂NHCH₂CH₂-^{#} oder Piperazin-1,4-diyl repräsentiert, wobei die Raute die Bindung andeutet, die mit B verknüpft ist, und B eine Bindung repräsentiert; oder
m 0 repräsentiert, A -NHCH₂CH₂NHCH₂-^{#} repräsentiert, wobei die Raute die Bindung andeutet, die mit B verknüpft ist, und B eine Bindung repräsentiert; oder
m 0 repräsentiert, A -OCH₂-^{#} repräsentiert, wobei die Raute die Bindung andeutet, die mit B verknüpft ist, und B eine Gruppe der Formel (E) repräsentiert 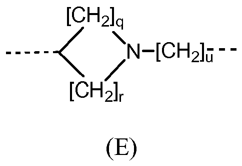 wobei q und r unabhängig voneinander 1 oder 2 repräsentieren und u 0 oder 1 repräsentiert; oder
m 0 repräsentiert, A O repräsentiert und B eine Gruppe der Formel (F) repräsentiert 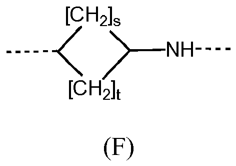 wobei sowohl s als auch t 1 repräsentieren;
G eine Gruppe der Formel (G1-a) repräsentiert 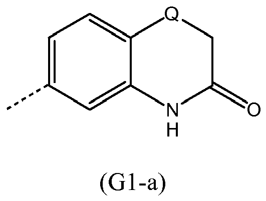 wobei Q O oder S repräsentiert; oder
G eine 2,3-Dihydro-benzo[1,4]dioxin-6-yl Gruppe repräsentiert;
oder ein Salz einer solchen Verbindung.

4. Verbindung der Formel (I) nach einem der Ansprüche 1 bis 3, wobei R¹ Wasserstoff, Methoxy oder Fluor repräsentiert;
oder ein Salz einer solchen Verbindung.

5. Verbindung der Formel (I) nach einem der Ansprüche 1 bis 4, wobei U CH repräsentiert, V CR^{b} repräsentiert, wobei R^{b} Wasserstoff oder Halogen ist, W -CH=CH- oder *-N=CH-repräsentiert, wobei das Sternchen die Bindung andeutet, die mit dem Kohlenstoffatom verknüpft ist, das V und W verbindet, und X N repräsentiert;
oder ein Salz einer solchen Verbindung.

6. Verbindung der Formel (I) nach einem der Ansprüche 1 bis 4, wobei U CH oder N repräsentiert, V CH oder N repräsentiert, W S repräsentiert und X CR^{c} repräsentiert, wobei R^{c} Wasserstoff oder (C₁₋₄)Alkyl ist;
oder ein Salz einer solchen Verbindung.

7. Verbindung der Formel (I) nach einem der Ansprüche 1 bis 4, wobei U CH oder N repräsentiert, V CH repräsentiert, W *-CH=CR^{a}- oder *-N=CH- repräsentiert, wobei die Sternchen die Bindung andeuten, die mit dem Kohlenstoffatom verknüpft ist, das V und W verbindet, und wobei R^{a} Wasserstoff oder Halogen repräsentiert und X CH repräsentiert;
oder ein Salz einer solchen Verbindung.

8. Verbindung der Formel (I) nach einem der Ansprüche 1 bis 4, wobei U CH oder N repräsentiert, V N repräsentiert, W -CH=CH- repräsentiert und X CH repräsentiert;
oder ein Salz einer solchen Verbindung.

9. Verbindung der Formel (I) nach einem der Ansprüche 1 bis 8, wobei m 1 repräsentiert, A -NHCH₂-^{#} oder -NHCH₂CH₂-^{#} repräsentiert, wobei die Raute die Bindung andeutet, die mit B verknüpft ist, und B eine Bindung repräsentiert;
oder ein Salz einer solchen Verbindung.

10. Verbindung der Formel (I) nach einem der Ansprüche 1 bis 8, wobei m 0 repräsentiert, A -OCH₂-^{#} repräsentiert, wobei die Raute die Bindung andeutet, die mit B verknüpft ist, und B eine Gruppe der Formel (E) repräsentiert, wobei q und r unabhängig voneinander 1 oder 2 repräsentieren und u 0 repräsentiert;
oder ein Salz einer solchen Verbindung.

11. Verbindung der Formel (I) nach einem der Ansprüche 1 bis 8, wobei m 0 repräsentiert, A O repräsentiert und B eine Gruppe der Formel (F) repräsentiert, wobei sowohl s als auch t 1 repräsentieren;
oder ein Salz einer solchen Verbindung.

12. Verbindung der Formel (I) nach Anspruch 1 oder 2, die ausgewählt ist aus Folgendem:
- 6-((*R*)-5-{3-[(7-Fluor-chinolin-2-ylmethyl)-amino]-propyl}-2-oxo-oxazolidin-3-yl)-4*H*-benzo[1,4]oxazin-3-on;
- 6-((*R*)-2-Oxo-5-{3-[(chinolin-2-ylmethyl)-amino]-propyl}-oxazolidin-3-yl)-4*H*-benzo[1,4]oxazin-3-on;
- 6-{(S-2-Oxo-5-[(3-chinolin-2-yl-propylamino)-methyl]-oxazolidin-3-yl}-4*H*-benzo[1,4]thiazin-3-on;
- 6-((*R*)-2-Oxo-5-{2-[(chinolin-2-ylmethyl)-amino]-ethyl}-oxazolidin-3-yl)-4*H*-benzo[1,4]thiazin-3-on;
- 6- {(*R*)-2-Oxo-5-[2-(2-chinolin-2-yl-ethylamino)-ethyl]-oxazolidin-3-yl}-4*H*-benzo[1,4]thiazin-3-on;
- 6-{(*R*)-5-[(*R*)-3-(7-Methoxy-chinolin-2-yloxymethyl)-pyrrolidin-1-ylmethyl]-2-oxo-oxazolidin-3-yl}-4*H*-benzo[1,4]thiazin-3-on;
- 6-{(*R*)-5-[4-(7-Methoxy-chinolin-2-yloxymethyl)-piperidin-1-ylmethyl]-2-oxo-oxazolidin-3-yl}-4*H*-benzo[1,4]thiazin-3-on;
- (*R*)-3-(2,3-Dihydro-benzo[1,4]dioxin-6-yl)-5-[3-(7-methoxy-chinoxalin-2-yloxymethyl)-azetidin-1-ylmethyl]-oxazolidin-2-on;
- 6-((*R*)-5- {2-[3-(7-Methoxy-chinoxalin-2-yloxymethyl)-azetidin-1-yl]-ethyl}-2-oxo-oxazolidin-3-yl)-4*H*-benzo[1,4]thiazin-3-on;
- 6- {(*R*)-5-[3-(7-Methoxy-chinoxalin-2-yloxymethyl)-azetidin-1-ylmethyl]-2-oxo-oxazolidin-3-yl}-4*H*-benzo[1,4]thiazin-3-on;
- 6-{(*R*)-5-[3-(7-Methoxy-chinoxalin-2-yloxymethyl)-azetidin-1-ylmethyl]-2-oxo-oxazolidin-3-yl}-4*H*-benzo[1,4]oxazin-3-on;
- 6-((*R*)-5-{[3-(7-Methoxy-chinolin-2-yloxy)-cyclobutylamino]-methyl}-2-oxo-oxazolidin-3-yl)-4*H*-benzo[1,4]thiazin-3-on;
- 6-(2-Oxo-5-{3-[(chinolin-2-ylmethyl)-amino]-propyl}-oxazolidin-3-yl)-4*H*-benzo[1,4]thiazin-3-on;
- 6-(5- {3-[(7-Fluor-chinolin-2-ylmethyl)-amino]-propyl} -2-oxo-oxazolidin-3-yl)-4*H*-benzo[1,4]thiazin-3-on;
- 6-(5-{3-[(6-Fluor-chinolin-7-ylmethyl)-amino]-propyl}-2-oxo-oxazolidin-3-yl)-4*H*-benzo[1,4]thiazin-3-on;
- 6-(5- {3-[(5-Fluor-chinolin-2-ylmethyl)-amino]-propyl}-2-oxo-oxazolidin-3-yl)-4*H*-benzo[1,4]thiazin-3-on;
- 6-(5-{3-[(3-Methyl-benzo[b]thiophen-2-ylmethyl)-amino]-propyl}-2-oxo-oxazolidin-3-yl)-4*H*-benzo[1,4]thiazin-3-on;
- 6-(2-Oxo-5-{3-[(thieno[2,3-b]pyridin-2-ylmethyl)-amino]-propyl}-oxazolidin-3-yl)-4*H*-benzo[1,4]thiazin-3-on;
- 6-(2-Oxo-5-{3-[(thieno[2,3-b]pyrazin-6-ylmethyl)-amino]-propyl}-oxazolidin-3-yl)-4*H*-benzo[1,4]thiazin-3-on;
- 6-(2-Oxo-5-{3-[(chinolin-3-ylmethyl)-amino]-propyl}-oxazolidin-3-yl)-4*H*-benzo[1,4]thiazin-3-on;
- 6-(5-{3-[(Naphthalen-2-ylmethyl)-amino]-propyl}-2-oxo-oxazolidin-3-yl)-4*H*-benzo[1,4]thiazin-3-on;
- 6-(2-Oxo-5-{3-[(chinolin-7-ylmethyl)-amino]-propyl}-oxazolidin-3-yl)-4*H*-benzo[1,4]thiazin-3-on; und
- 6-(2-Oxo-5-{3-[(chinolin-6-ylmethyl)-amino]-propyl}-oxazolidin-3-yl)-4*H*-benzo[1,4]thiazin-3-on;
- 6-{(*R*)-2-Oxo-5-[(2-chinolin-2-yl-ethylamino)-methyl]-oxazolidin-3-yl}-4*H*-benzo[1,4]thiazin-3-on;
- 6- {(*R*)-2-Oxo-5-[3-(2-chinolin-2-yl-ethylamino)-propyl]-oxazolidin-3-yl}-4*H*-benzo[1,4]thiazin-3-on;
- 6-((*R*)-2-Oxo-5-{2-[2-(chinolin-2-ylamino)-ethylamino]-ethyl}-oxazolidin-3-yl)-4*H*-benzo[1,4]oxazin-3-on;
- 6-[(*R*)-2-Oxo-5-({2-[(chinolin-2-ylmethyl)-amino]-ethylamino}-methyl)-oxazolidin-3-yl]-4*H*-benzo[1,4]thiazin-3-on;
- 6-[(*R*)-2-Oxo-5-(4-chinolin-2-ylmethyl-piperazin-1-ylmethyl)-oxazolidin-3-yl]-4*H*-benzo[1,4]thiazin-3-on;
- 6-((*S*)-5-{3-[(7-Fluor-2-methoxy-chinoxalin-6-ylmethyl)-amino]-propyl}-2-oxo-oxazolidin-3-yl)-4*H*-benzo[1,4]thiazin-3-on;
oder ein Salz einer solchen Verbindung.

13. Als Medikament eine Verbindung der Formel (I) nach einem der Ansprüche 1 bis 12 oder ein pharmazeutisch akzeptables Salz davon.

14. Pharmazeutische Zusammensetzung, die als Wirkstoff eine Verbindung der Formel (I) nach einem der Ansprüche 1 bis 12 oder ein pharmazeutisch akzeptables Salz davon und wenigstens einen therapeutisch inerten Exzipienten enthält.

15. Verwendung einer Verbindung der Formel (I) nach einem der Ansprüche 1 bis 12 oder eines pharmazeutisch akzeptablen Salzes davon zur Herstellung eines Medikaments zur Verhütung oder Behandlung einer bakteriellen Infektion.

16. Verbindung der Formel (I) nach einem der Ansprüche 1 bis 12 oder ein pharmazeutisch akzeptables Salz davon zur Verhütung oder Behandlung einer bakteriellen Infektion.

## Revendications

1. Composé de formule (I) 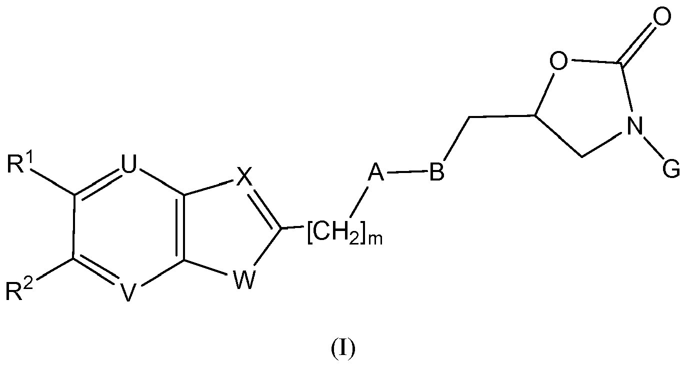 où
R¹ représente un hydrogène, un (C₁₋₄)alkoxy ou un halogène ;
R² représente un hydrogène ou un (C₁₋₄)alkoxy ;
U représente N ou CH ;
V représente N ou CR^{b}, où R^{b} est un hydrogène ou un halogène ;
W représente *-CH=CR^{a}-, *-N=CH- ou S, où les astérisques indiquent la liaison qui est rattachée à l'atome de carbone reliant V et W et où R^{a} représente un hydrogène ou un halogène ;
X représente N ou CR^{c}, où R^{c} est un hydrogène, un (C₁₋₄)alkyle ou un halogène ;
à condition que le groupement de formule (D) 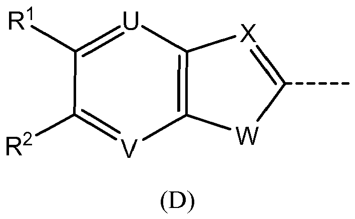 contienne entre zéro et trois hétéroatomes, où les hétéroatomes sont indépendamment sélectionnés parmi l'azote et, dans le cas de W, le soufre ;
m représente 1, A représente -NHCH₂-^{#}, -CH₂NH-^{#}, -NHCH₂CH₂-^{#}, -CH₂NHCH₂- ou -CH₂CH₂NH-^{#}, -NHCH₂CH₂NH-, -CH₂NHCH₂CH₂-^{#} ou un groupement pipérazin-1,4-diyle, où le dièse indique la liaison qui est rattachée à B et où B représente une liaison ;
ou
m représente 0, A représente -NHCH₂CH₂NHCH₂-^{#}, où le dièse indique la liaison qui est rattachée à B et où B représente une liaison ; ou
m représente 0, A représente -OCH₂-^{#}, où le dièse indique la liaison qui est rattachée à B et où B représente un groupement de formule (E) 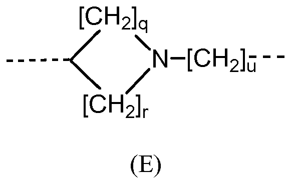 où q et r représentent indépendamment l'un de l'autre 1 ou 2 et u représente 0 ou 1 ; ou
m représente 0, A représente O et B représente un groupement de formule (F) 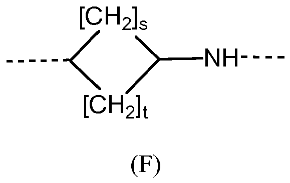 où s et t représentent indépendamment l'un de l'autre 1 ou 2 ;
G représente un groupement de formule (G1) 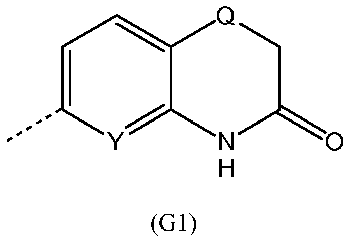 où Y représente CH ou N et Q représente O ou S ; ou G représente un groupement de formule (G2) 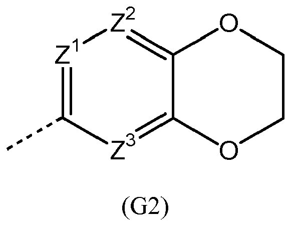 où
Z¹ représente CH, Z² représente CH et Z³ représente N ; ou
Z¹ représente N, Z² représente CH ou N et Z³ représente CH ; ou
Z¹ représente CH, Z² représente CH ou N et Z³ représente CH ;
ou sel d'un tel composé.

2. Composé de formule (I) selon la revendication 1, qui est également un composé de formule (I_{P}) 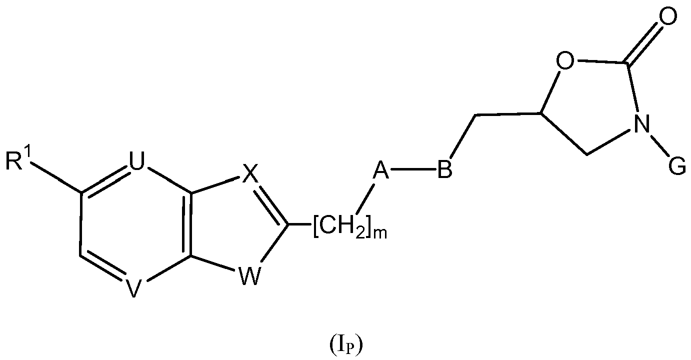 où
R¹ représente un hydrogène, un (C₁₋₄)alkoxy ou un halogène ;
U représente N ou CH ;
V représente N ou CR^{b}, où R^{b} est un hydrogène ou un halogène ;
W représente *-CH=CR^{a}-, *-N=CH- ou S, où les astérisques indiquent la liaison qui est rattachée à l'atome de carbone reliant V et W et où R^{a} représente un hydrogène ou un halogène ;
X représente N ou CR^{c}, où R^{c} est un hydrogène, un (C₁₋₄)alkyle ou un halogène ;
à condition que le groupement de formule (D) 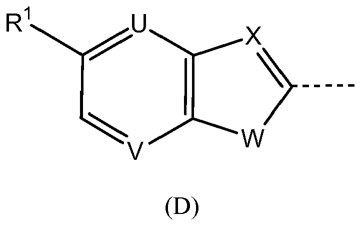 contienne entre zéro et trois hétéroatomes, où les hétéroatomes sont indépendamment sélectionnés parmi l'azote et, dans le cas de W, le soufre ;
m représente 1, A représente -NHCH₂-^{#}, -NHCH₂CH₂-^{#}, -CH₂NHCH₂- ou -CH₂CH₂NH-^{#}, où le dièse indique la liaison qui est rattachée à B et où B représente une liaison ; ou
m représente 0, A représente -OCH₂-^{#}, où le dièse indique la liaison qui est rattachée à B et où B représente un groupement de formule (E) 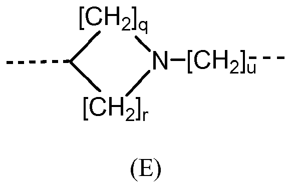 où q et r représentent indépendamment l'un de l'autre 1 ou 2 et u représente 0 ou 1 ; ou
m représente 0, A représente O et B représente un groupement de formule (F) 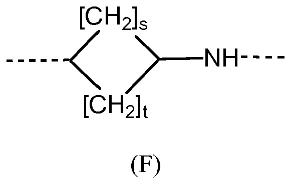 où s et t représentent indépendamment l'un de l'autre 1 ou 2 ;
G représente un groupement de formule (G1) 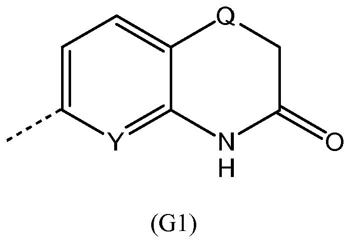 où Y représente CH ou N et Q représente O ou S ; ou G représente un groupement de formule (G2) 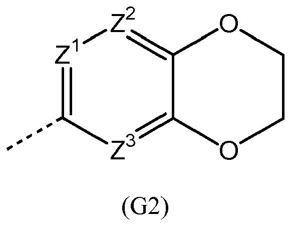 où
Z¹ représente CH, Z² représente CH et Z³ représente N ; ou
Z¹ représente N, Z² représente CH ou N et Z³ représente CH ; ou
Z¹ représente CH, Z² représente CH ou N et Z³ représente CH ;
ou sel d'un tel composé.

3. Composé de formule (I) selon la revendication 1, qui est également un composé de formule (I_{CE}) 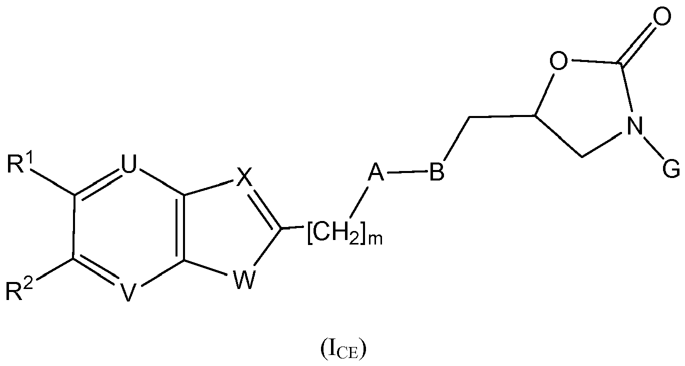 où
R¹ représente un hydrogène, un (C₁₋₄)alkoxy ou un halogène ;
R² représente un hydrogène ou également, si R¹ représente un hydrogène, un (C₁₋₄)alkoxy ; U représente CH, V représente CR^{b}, où R^{b} est un hydrogène ou un halogène, W représente -CH=CH- ou *-N=CH-, où l'astérisque indique la liaison qui est rattachée à l'atome de carbone reliant V et W, et où X représente N ; ou
U représente CH ou N, V représente CH ou N, W représente S et X représente CR^{c}, où R^{c} est un hydrogène ou un (C₁₋₄)alkyle (notamment un méthyle) ; ou
U représente CH ou N, V représente CH, W représente *-CH=CR^{a}- ou *-N=CH-, où les astérisques indiquent la liaison qui est rattachée à l'atome de carbone reliant V et W, et où R^{a} représente un hydrogène ou un halogène (notamment un fluor) et X représente CH ; ou U représente CH ou N, V représente N, W représente -CH=CH- et X représente CH ;
m représente 1, A représente -NHCH₂-^{#}, -CH₂NH-^{#}, -NHCH₂CH₂-^{#}, -CH₂NHCH₂-, -CH₂CH₂NH-^{#}, -NHCH₂CH₂NH-^{#}, -CH₂NHCH₂CH₂-^{#} ou un groupement pipérazin-1,4-diyle, où le dièse indique la liaison qui est rattachée à B et où B représente une liaison ; ou m représente 0, A représente -NHCH₂CH₂NHCH₂-^{#}, où le dièse indique la liaison qui est rattachée à B et où B représente une liaison ; ou
m représente 0, A représente -OCH₂-^{#}, où le dièse indique la liaison qui est rattachée à B et où B représente un groupement de formule (E) 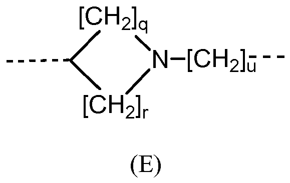 où q et r représentent indépendamment l'un de l'autre 1 ou 2 et u représente 0 ou 1 ; ou
m représente 0, A représente O et B représente un groupement de formule (F) 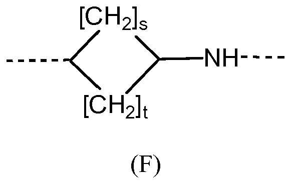 où s et t représentent tous les deux 1 ;
G représente un groupement de formule (G1-a) 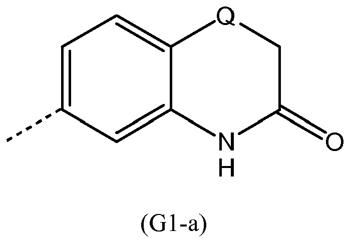 où Q représente O ou S ; ou
G représente un groupement 2,3-dihydro-benzo[1,4]dioxin-6-yle ;
ou sel d'un tel composé.

4. Composé de formule (I) selon l'une quelconque des revendications 1 à 3, où R¹ représente un hydrogène, un méthoxy ou un fluor ;
ou sel d'un tel composé.

5. Composé de formule (I) selon l'une quelconque des revendications 1 à 4, où U représente CH, V représente CR^{b}, où R^{b} est un hydrogène ou un halogène, W représente -CH=CH- ou *-N=CH-, où l'astérisque indique la liaison qui est rattachée à l'atome de carbone reliant V et W, et où X représente N ;
ou sel d'un tel composé.

6. Composé de formule (I) selon l'une quelconque des revendications 1 à 4, où U représente CH ou N, V représente CH ou N, W représente S et X représente CR^{c}, où R^{c} est un hydrogène ou un (C₁₋₄)alkyle ;
ou sel d'un tel composé.

7. Composé de formule (I) selon l'une quelconque des revendications 1 à 4, où U représente CH ou N, V représente CH, W représente *-CH=CR^{a}- ou *-N=CH-, où les astérisques indiquent la liaison qui est rattachée à l'atome de carbone reliant V et W, et où R^{a} représente un hydrogène ou un halogène et X représente CH ;
ou sel d'un tel composé.

8. Composé de formule (I) selon l'une quelconque des revendications 1 à 4, où U représente CH ou N, V représente N, W représente -CH=CH- et X représente CH ;
ou sel d'un tel composé.

9. Composé de formule (I) selon l'une quelconque des revendications 1 à 8, où m représente 1, A représente -NHCH₂-^{#} ou -NHCH₂CH₂-^{#}, où le dièse indique la liaison qui est rattachée à B, et où B représente une liaison ;
ou sel d'un tel composé.

10. Composé de formule (I) selon l'une quelconque des revendications 1 à 8, où m représente 0, A représente -OCH₂-^{#}, où le dièse indique la liaison qui est rattachée à B, et où B représente un groupement de formule (E), où q et r représentent indépendamment l'un de l'autre 1 ou 2 et u représente 0 ;
ou sel d'un tel composé.

11. Composé de formule (I) selon l'une quelconque des revendications 1 à 8, où m représente 0, A représente O et B représente un groupement de formule (F), où s et t représentent tous les deux 1 ;
ou sel d'un tel composé.

12. Composé de formule (I) selon la revendication 1 ou 2, qui est sélectionné parmi les suivants :
- 6-((*R*)-5-{3-[(7-fluoro-quinoléin-2-ylméthyl)-amino]-propyl}-2-oxo-oxazolidin-3-yl)-4*H*-benzo[1,4]oxazin-3-one,
- 6-((*R*)-2-oxo-5- {3-[(quinoléin-2-ylméthyl)-amino]-propyl}-oxazolidin-3-yl)-4*H*-benzo[1,4]oxazin-3-one,
- 6- {(*S*)-2-oxo-5-[(3-quinoléin-2-yl-propylamino)-méthyl]-oxazolidin-3-yl}-4*H*-benzo[1,4]thiazin-3-one ;
- 6-((*R*)-2-oxo-5- {2-[(quinoléin-2-ylméthyl)-amino]-éthyl}-oxazolidin-3-yl)-4*H*-benzo[1,4]thiazin-3-one ;
- 6- ((*R*)-2-oxo-5-[2-(2-quinoléin-2-yl-éthylamino)-éthyl]-oxazolidin-3-yl}-4*H*-benzo[1,4]thiazin-3-one ;
- 6- {(*R*)-5-[(*R*)-3-(7-méthoxy-quinoléin-2-yloxyméthyl)-pyrrolidin-1-ylméthyl]-2-oxo-oxazolidin-3-yl}-4*H*-benzo[1,4]thiazin-3-one ;
- 6-{(*R*)-5-[4-(7-méthoxy-quinoléin-2-yloxyméthyl)-pipéridin-1-ylméthyl]-2-oxo-oxazolidin-3-yl}-4*H*-benzo[1,4]thiazin-3-one ;
- (*R*)-3-(2,3-dihydro-benzo[1,4]dioxin-6-yl)-5-[3-(7-méthoxy-quinoxalin-2-yloxyméthyl)-azétidin-1-ylméthyl]-oxazolidin-2-one ;
- 6-((*R*)-5-{2-[3-(7-méthoxy-quinoxalin-2-yloxyméthyl)-azétidin-1-yl]-éthyl}-2-oxo-oxazolidin-3-yl)-4*H*-benzo[1,4]thiazin-3-one,
- 6- {(*R*)-5-[3-(7-méthoxy-quinoxalin-2-yloxyméthyl)-azétidin-1-ylméthyl]-2-oxo-oxazolidin-3-yl}-4*H*-benzo[1,4]thiazin-3-one ;
- 6-{(*R*)-5-[3-(7-méthoxy-quinoxalin-2-yloxyméthyl)-azétidin-1-ylméthyl]-2-oxo-oxazolidin-3-yl}-4*H*-benzo[1,4]oxazin-3-one ;
- 6-((*R*)-5-{[3-(7-méthoxy-quinoléin-2-yloxy)-cyclobutylamino]-méthyl}-2-oxo-oxazolidin-3-yl)-4*H*-benzo[1,4]thiazin-3-one ;
- 6-(2-oxo-5-{3-[(quinoléin-2-ylméthyl)-amino]-propyl}-oxazolidin-3-yl)-4*H*-benzo[1,4]thiazin-3-one ;
- 6-(5-{3-[(7-fluoro-quinoléin-2-ylméthyl)-amino]-propyl}-2-oxo-oxazolidin-3-yl)-4*H*-benzo[1,4]thiazin-3-one ;
- 6-(5-{3-[(6-fluoro-quinoléin-7-ylméthyl)-amino]-propyl}-2-oxo-oxazolidin-3-yl)-4*H*-benzo[1,4]thiazin-3-one ;
- 6-(5-{3-[(5-fluoro-quinoléin-2-ylméthyl)-amino]-propyl}-2-oxo-oxazolidin-3-yl)-4*H*-benzo[1,4]thiazin-3-one ;
- 6-(5-{3-[(3-méthyl-benzo[*b*]thiophén-2-ylméthyl)-amino]-propyl}-2-oxo-oxazolidin-3-yl)-4*H*-benzo[1,4]thiazin-3-one,
- 6-(2-oxo-5- {3-[(thiéno[2,3-*b*]pyridin-2-ylméthyl)-amino]-propyl}-oxazolidin-3-yl)-4*H*-benzo[1,4]thiazin-3-one ;
- 6-(2-oxo-5- {3-[(thiéno[2,3-*b*]pyrazin-6-ylméthyl)-amino]-propyl}-oxazolidin-3-yl)-4*H*-benzo[1,4]thiazin-3-one ;
- 6-(2-oxo-5-{3-[(quinoléin-3-ylméthyl)-amino]-propyl}-oxazolidin-3-yl)-4*H*-benzo[1,4]thiazin-3-one ;
- 6-(5-{3-[(naphtalén-2-ylméthyl)-amino]-propyl}-2-oxo-oxazolidin-3-yl)-4*H*-benzo[1,4]thiazin-3-one ;
- 6-(2-oxo-5-{3-[(quinoléin-7-ylméthyl)-amino]-propyl}-oxazolidin-3-yl)-4*H*-benzo[1,4]thiazin-3-one ; et
- 6-(2-oxo-5-{3-[(quinoléin-6-ylméthyl)-amino]-propyl}-oxazolidin-3-yl)-4*H*-benzo[1,4]thiazin-3-one ;
- 6- {(*R*)-2-oxo-5-[(2-quinoléin-2-yl-éthylamino)-méthyl]-oxazolidin-3-yl}-4*H*-benzo[1,4]thiazin-3-one ;
- 6- {(*R*)-2-oxo-5-[3-(2-quinoléin-2-yl-éthylamino)-propyl]-oxazolidin-3-yl}-4*H*-benzo[1,4]thiazin-3-one ;
- 6-((*R*)-2-oxo-5-{2-[2-(quinoléin-2-ylamino)-éthylamino]-éthyl}-oxazolidin-3-yl)-4*H*-benzo[1,4]oxazin-3-one,
- 6-[(*R*)-2-oxo-5-({2-[(quinoléin-2-ylméthyl)-amino]-éthylamino}-méthyl)-oxazolidin-3-yl]-4*H*-benzo[1,4]thiazin-3-one ;
- 6-[(*R*)-2-oxo-5-(4-quinoléin-2-ylméthyl-pipérazin-1-ylméthyl)-oxazolidin-3-yl]-4*H*-benzo[1,4]thiazin-3-one ;
- 6-((*S*)-5-{3-[(7-fluoro-2-méthoxy-quinoxalin-6-ylméthyl)-amino]-propyl}-2-oxo-oxazolidin-3-yl)-4*H*-benzo[1,4]thiazin-3-one,
ou sel d'un tel composé.

13. En tant que médicament, composé de formule (I) tel que défini à l'une quelconque des revendications 1 à 12 ou sel pharmaceutiquement acceptable de celui-ci.

14. Composition pharmaceutique contenant, comme principe actif, un composé de formule (I) tel que défini à l'une des revendications 1 à 12 ou un sel pharmaceutiquement acceptable de celui-ci et au moins un excipient thérapeutiquement inerte.

15. Utilisation d'un composé de formule (I) tel que défini à l'une des revendications 1 à 12 ou d'un sel pharmaceutiquement acceptable de celui-ci dans la fabrication d'un médicament pour la prévention ou le traitement d'une infection bactérienne.

16. Composé de formule (I) tel que défini à l'une des revendications 1 à 12 ou sel pharmaceutiquement acceptable de celui-ci, pour la prévention ou le traitement d'une infection bactérienne.
